# EUROPEAN PATENT APPLICATION

(11) **EP 4 585 586 A1**
(43) Date of publication of application: **16.07.2025**
(21) Application number: 23862519.8
(22) Date of filing: 08.09.2023
(51) Int. Cl.: C07C 219/06, C07C 229/06, A61K 9/127, A61P 35/00

(54) **CARBON NUCLEUS CATIONIC LIPID**

(30) Priority: 09.09.2022 CN 202211102740
(71) Applicant: Xiamen Sinopeg Biotech Co., Ltd., Xiamen, Fujian 361100 (CN)
(72) Inventor: WENG, Wengui, Xiamen, Fujian 361100 (CN); LIU, Chao, Xiamen, Fujian 361100 (CN); LIN, Sheng, Xiamen, Fujian 361100 (CN); WANG, Linlin, Xiamen, Fujian 361100 (CN); LIN, Minggui, Xiamen, Fujian 361100 (CN); WANG, Ailan, Xiamen, Fujian 361100 (CN); WEI, Guohua, Xiamen, Fujian 361100 (CN)
(74) Representative: Algemeen Octrooi- en Merkenbureau B.V.
(86) International application number: PCT/CN2023/117745
(87) International publication number: WO 2024/051825

(57) **Abstract**

The present invention discloses a novel cationic lipid and a method for preparing the same, a lipid composition containing said cationic lipid and an LNP-drug composition and a formulation thereof containing the lipid composition, in particular, the LNP-pharmaceutical composition and the formulation thereof containing the cationic lipid, which is characterized by high delivery efficiency and high biocompatibility, thereby improving the therapeutic and preventive effects of the drug. The structure of the cationic lipid is shown in the general formula (1), and the definition of each symbol in the formula is described in the text.

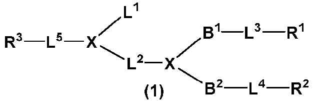

## Description

### TECHNICAL FIELD

The present application belongs to the field of drug delivery and specifically relates to a cationic lipid as a pharmaceutical carrier, in particular to a carbon-branched cationic lipid, as well as a lipid composition containing the cationic lipid, a lipid pharmaceutical composition, and formulations and applications thereof.

### BACKGROUND

Drug delivery strategies are generally categorized into viral and non-viral delivery vectors and have matured over the past few decades. Although viral delivery vectors have high cell transfection rates, they have limited scope for biomedical research and clinical development due to rapid clearance of existing antibodies, production of neutralizing antibodies against vectors, limited vector size, and possible side effects.

In recent years, lipid-based nano delivery systems have been the most widely used non-viral delivery vectors, among which LNPs (lipid nanoparticles) are the most popular lipid-based delivery vectors. Lipid nanoparticles typically include ionizable cationic lipid, cholesterol, phospholipid, and PEGylated lipid, where ionizable lipid plays a major role in protecting nucleic acids from nuclease degradation. In addition, auxiliary lipids, such as phospholipid and cholesterol, can improve formulation stability and promote membrane fusion. Unlike liposomes, LNPs have a micellar structure within the particle core, exhibiting better kinetic stability and a harder morphology. Large-scale commercial preparation methods, such as microfluidics, can obtain more homogeneous LNPs. LNPs can effectively deliver drugs (especially nucleic acid drugs). Ionizable cationic lipids have a near-neutral charge at physiological pH but are partially positively charged due to protonation at low pH, and can be combined with negatively charged phosphate groups on nucleic acids through electrostatic interaction, thereby effectively binding and transporting nucleic acid drugs.

In the field of drug delivery, cationic lipids have achieved some success, for example, ALC-0315 disclosed in CN108368028A, SM102 disclosed in US9868692B2, and MC3 disclosed in CN102625696A are all cationic lipids with excellent performance. Among them, MC3 contains two long hydrophobic aliphatic tail chains, and its polar tertiary amine group is connected to a non-ionizable carbon core. ALC-0315 contains four (type 2+2) long hydrophobic aliphatic tail chains, and it contains a nitrogen core that leads to the polar hydroxyl group; SM102 contains three (type 1+2) long hydrophobic aliphatic tail chains, and its nitrogen core leads to the polar hydroxyl group. Despite the successful applications of these cationic lipids, to enrich the variety of cationic lipids, it is necessary to continue to develop cationic lipids of novel types for substitution or even better performance.

### SUMMARY

To solve the above problems, the present application provides a novel cationic lipid and preparation methods thereof, comprising a lipid composition containing the cationic lipid, a lipid pharmaceutical composition containing the lipid composition and the formulation thereof, and a liposome or LNP containing the lipid composition. Especially, the LNP-nucleic acid drug composition containing the lipid composition and the formulations thereof have the advantages of high delivery efficiency, safety, low toxicity, and high biocompatibility, which can improve the therapeutic and/or preventive effects of the drugs.

The above-described purposes of this application can be realized via the embodiments below.

In one embodiment, provided herein is a cationic lipid:
A cationic lipid; wherein, the structure is represented by the general formula (1): 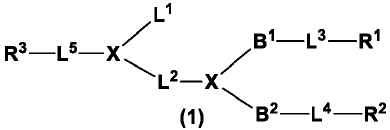
wherein, X is independently N or CR^{a} at each occurrence, and the R^{a} is H or a C₁₋₁₂ alkyl group;
L¹ is -L°R; L° is a linking bond or a divalent linking group, and R is a C₁₋₃₀ aliphatic hydrocarbon group; wherein, the aliphatic hydrocarbon group is an alkyl group, an alkenyl group or an alkynyl group;
L² is a linking bond or a divalent linking group;
L⁵ is a linking bond or a divalent linking group;
L³ and L⁴ are each independently a linking bond or a divalent linking group;
B¹ and B² are each independently a linking bond or a C₁₋₃₀ alkylene group;
R¹ and R² are each independently a C₁₋₃₀ aliphatic hydrocarbon group or 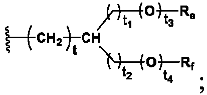 wherein, t is an integer from 0 to 12; t₁ and t₂ are each independently an integer from 0 to 5; t₃ and t₄ are each independently 0 or 1; t₁, t₂, t₃ and t₄ are not 0 simultaneously; Rₑ and R_{f} are each independently selected from the group consisting of a C₁₋₁₅ alkyl group, a C₂₋₁₅ alkenyl group and a C₂₋₁₅ alkynyl group;
R³ is selected from the group consisting of a hydrogen atom, -R^{d}, -OR^{d}, a C₃₋₆ carbocyclic group, a nitrogen-containing heterocyclic group, -NR^{d}R^{d}, -SR^{d}, -C(=O)R^{d}, -C(=O)OR^{d}, - OC(=O)R^{d}, -OC(=O)OR^{d} and a functional group R⁰¹ that can interact with bio-related substances; wherein, R^{d} is a C₁₋₁₂ alkyl group;
or a salt, tautomer, stereoisomer, or solvate thereof.

The present application also provides a lipid composition, embodied as follows:
A lipid composition containing a cationic lipid with the structure represented by the general formula (1).

The present application also provides a lipid pharmaceutical composition, embodied as follows:
Provided herein is a lipid pharmaceutical composition containing a lipid composition and a drug; wherein, the lipid composition contains the cationic lipid with the structure represented by the formula (1), and the drug is selected from the group consisting of a nucleic acid drug, a gene vaccine, an anti-tumor drug, a small molecule drug, a polypeptide drug and a protein drug.

The present application also provides a formulation of lipid pharmaceutical composition, embodied as follows:
Provided herein is a formulation of lipid pharmaceutical composition containing the aforementioned lipid pharmaceutical composition and a pharmaceutically acceptable diluent or excipient.

The present application also provides a liposome or LNP, embodied as follows:
Provided herein is a liposome or LNP containing a lipid composition, and the lipid composition contains a cationic lipid with the structure represented by the formula (1).

### Compared with the prior art, the present application brings the following beneficial effects:

The present application provides a novel cationic lipid compound, enriching the cationic lipid compound species, and providing more options for the delivery of nucleic acid drugs, gene vaccines, small molecule drugs, peptides, and protein drugs.

The novel cationic lipid of the present application contains four or five hydrophobic tail chains, and the arrangement of the four hydrophobic tail chains is the "1+2+1" type or "2+2+1" type; the arrangement is looser and leads to an enlarged cross-section of the tail region, which results in a conical structure that helps to increase the membrane instability and enhance the endosomal escape, therefore promoting the release of drugs into the cytoplasm to exert the corresponding efficacies.

The novel cationic lipid of the present application contains an ionizable tertiary amine structure, and the short polar head is connected to a carbon core. Compared with the carbon-core lipid (e.g., MC3) in the prior art, the combination of an ionizable tertiary amine structure and a short polar head is capable of enhancing the binding of the ionizable cationic lipid to a drug (e.g., negatively charged nucleic acid drug), thereby improving the efficiency of drug delivery.

For the novel cationic lipid of the present application, one of the aliphatic tail chains together with the polar head group connected to the carbon-core can be simultaneously obtained by the ring-opening of an epoxide, and the preparation process is simple and efficient.

The cationic liposome-nucleic acid drug composition prepared by the novel cationic lipid of the present application has higher stability in serum, stronger ability of gene complexation, higher biocompatibility, and stronger effect of gene transfection, which is helpful to improve the gene therapy effect of drugs, especially the gene therapy effect of nucleic acid drugs.

The terminal of the novel cationic lipid of the present application may also be coupled with a fluorescent group or a targeting group, and the lipid composition and lipid pharmaceutical composition prepared from the cationic lipid can have both fluorescent and targeting function, further improving the therapeutic and/or diagnostic effects.

### EMBODIMENTS OF THE APPLICATION

### Description of terms

In the present application, unless otherwise specified, each term has the following meanings.

In the present application and unless otherwise specified, a structure with isomers may refer to any form of the isomers. For example, when *cis-* and *trans-* isomers are present, it can refer to either a *cis*-structure or a *trans*-structure; when *E*/*Z* isomers are present, it can refer to either an (*E*)-structure or a (*Z*)-structure; and when optical activity is present, it can refer to either laevoisomer or dextroisomer.

In the present application, the numerical interval includes both the numerical interval marked by the dashed line (e.g., 1-6) and the numerical interval marked by the wavy line such as (1~6). In the present application and unless otherwise specified, an integer interval represents the group consisting of all integers within the range of the interval, and the range includes two endpoints as well. For example, the integer interval 1-6 represents the group consisting of 1, 2, 3, 4, 5, and 6. The numerical interval in the present application includes but is not limited to the integer intervals represented by integers, non-integers, percentages, and fractions; unless otherwise specified, all of the aforementioned numerical intervals include two endpoints.

In the present application, "about" or "approximately" followed by a numerical value generally suggests a ±10% numerical range; in some cases, the numerical range can be enlarged to ±15%, but no more than ±20%. For example, when the molar percentage of steroid lipid in the total lipids in a solution containing solvent is about 40%, it can be considered that, generally, the molar percentage of steroid lipid is 30%-50%.

In the present application, "pharmaceutically acceptable salt" means a salt in which the compounds of the present application is recognized for use in animals, and more precisely in humans, including compounds shown in formula (1) and salts formed by inorganic acids or organic acids. See, e.g., S. M. Berge et al., "Pharmaceutical Salts", J. Pharm. Sci. 1977, 66, 1-19. Wherein, exemplary inorganic acids include hydrochloric acid, hydrobromic acid, hydroiodic acid, sulfuric acid, phosphoric acid, nitric acid, etc.; exemplary organic acids include formic acid, acetic acid, acetoacetic acid, pyruvate, trifluoroacetic acid, propionic acid, butyric acid, caproic acid, heptanoic acid, undecanoic acid, lauric acid, benzoic acid, salicylic acid, 2-(4-hydroxybenzoyl)-benzoic acid, camphoric acid, cinnamic acid, cyclopentanepropionic acid, gluconic acid, 3-hydroxy-2-naphthoic acid, niacin, bamoic acid, pectin ester acid, 3-phenylpropionic acid, picric acid, pivaleric acid, 2-hydroxyethanesulfonic acid, itaconic acid, aminosulfonic acid, trifluoromethanesulfonic acid, lauryl sulfuric acid, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid, etc. For example, HCl (or hydrochloric acid), HBr (or hydrobromic acid solution), methanesulfonic acid, sulfuric acid, tartaric acid or fumaric acid may be used to form a pharmaceutically acceptable salt with the compound represented by the formula (1).

In the present application, the term "solvate" refers to a complex formed by a compound of formula (1) or a pharmaceutically acceptable salt thereof with a solvent (e.g., ethanol or water). It should be understood that any solvate of formula (1) used in the treatment of a disease or condition, although it may provide different properties (including pharmacokinetic properties), once absorbed into the subject, will result in the compound of formula (1), such that the use of a compound of formula (1) covers the use of any solvate of compound of formula (1), respectively. It should be further understood that the compound of formula (1) or a pharmaceutically acceptable salt thereof may be isolated in the form of a solvate, and therefore any said solvate is included within the scope of the present application. For example, a compound of formula (1) or a pharmaceutically acceptable salt thereof may exist in an unsolvated form or a solvated form formed with a pharmaceutically acceptable solvent (e.g., water, ethanol, etc.).

The terms "include", "contain", and similar expressions in the present application shall be interpreted, unless otherwise specified, as "including but not limited to", "include but are not limited to", or "includes but is not limited to", etc., in the description and claims with openness and inclusiveness.

In the present application, when two or more objects are "each independently preferably" selected from multiple levels of preferable options, the objects are not necessarily selected from preferable options of the same level. It is allowed that one is selected from a wider range of preferable options while another one is selected from a narrower range of preferable options. It is also allowed that one is selected from the maximum range of preferable options while another is selected from any allowable preferable options. It is also allowed that the objects are selected from preferable options of the same level.

In the present application and unless otherwise specified, divalent linking groups, e.g., a hydrocarbylene group, an alkylene group, an arylene group, an amide bond, and the like, either one of the two connection ends could be chosen to be connected to another group. For example, when an amide bond serves as a divalent linking group between C-CH₂CH₂- and -CH₂-D, both C-CH₂CH₂-C(=O)NH-CH₂-D and C-CH₂CH₂-NHC(=O)-CH₂-D are allowable.

In the present application, when distinguishing the terminus from the substituents of a linking group becomes questionable, " 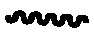 " is used to indicate the connection location between the linking group and the other group. For example, in structural formulas 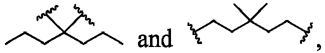 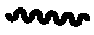 are used to indicate the connection locations between the divalent linking groups and the other groups; the two structural formulas mentioned above represent -CH(CH₂CH₂CH₃)₂- and -CH₂CH₂CH(CH₃)₂-CH₂CH₂-, respectively.

In the present application, a numerical interval written in the subscript of "C" can be used to indicate the number of carbon atoms in a group. For example, a C₁₋₁₂ group is a group having 1 to 12 carbon atoms; C₁₋₃₀ indicates "having 1 to 30 carbon atoms". A "substituted C₁₋₁₂ alkyl group" means a C₁₋₁₂ alkyl group with one or more hydrogen atoms being substituted. "C₁₋₁₂ substituted alkyl group" means an alkyl group with one or more hydrogen atoms being substituted has 1 to 12 carbon atoms remaining. As another example, when a group can be selected from C₁₋₁₂ alkylene groups, it can be any alkylene group with the number of carbon atoms in the range indicated by the subscript, that is, the group can be selected from the group consisting of C₁, C₂, C₃, C₄, C₅, C₆, C₇, C₈, C₉, C₁₀, C₁₁, and C₁₂ alkylene groups. In the present application and unless otherwise specified, a subscript being a numerical interval indicates that the subscript can be any integer within the interval which includes two endpoints.

In the present application, heteroatoms are not particularly limited, including but not limited to O, S, N, P, Si, F, Cl, Br, I, B, etc.

In the present application, the heteroatom used for substitution is referred to as "substituent atom", and the group used for substitution is referred to as "substituent group".

In the present application, a "substituted" group indicates that at least one hydrogen atom of the aforementioned group (e.g., aliphatic hydrocarbon groups, hydrocarbon groups, alkyl groups, or alkylene groups) is replaced by a bond connected to a non-hydrogen atom, the non-hydrogen atom including but not limited to halogen atom (F, Cl, Br, or I), an oxo group (=O), a hydroxyl group (-OH), a hydrocarbyloxy group (-OR^{d}; wherein, R^{d} is a C₁₋₁₂ alkyl group), a carboxyl group (-COOH), an amine group (-NR^{c}R^{c}; wherein, both R^{c} are each independently a hydrogen atom or a C₁₋₁₂ alkyl group), a C₁₋₁₂ alkyl group and a cycloalkyl group. In some embodiments, the substituent group is a C₁₋₁₂ alkyl group. In another embodiment, the substituent is a cycloalkyl group. In another embodiment, the substituent is a halo group. In another embodiment, the substituent is an oxo group. In another embodiment, the substituent is a hydroxyl group. In another embodiment, the substituent is an alkoxy group. In another embodiment, the substituent is a carboxyl group. In another embodiment, the substituent is an amine group.

In the present application, "optional" or "optionally" (e.g., optionally substituted) means that the event of the situation described thereafter may or may not occur, and the description includes instances in which the event or situation occurs as well as instances in which the event or situation does not occur. For example, "optionally substituted hydrocarbyl" means that the hydrocarbyl group may or may not be substituted, and the description includes both substituted and unsubstituted hydrocarbyl groups.

In the present application, "carbon chain linker" or "carbon chain linking group" refers to the linking group wherein main-chain atoms are all carbon atoms, allowing heteroatoms or groups containing heteroatoms in the side chains which substitute for hydrogen atoms connected to main-chain carbon atoms. When a "main-chain atom" is a heteroatom, it can also be called a "main-chain heteroatom". For example, A-S-CH₂-B, A-O-CH₂-B, and 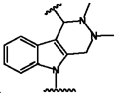 (wherein, the atomic spacing is 4) are considered to contain main-chain heteroatoms. Carbon chain linkers include hydrocarbylene groups and those wherein pendant groups contain heteroatoms; the carbon chain linker wherein pendant group contain heteroatoms include but are not limited to those substituted with an oxo group (=O), a thioxo group (=S), or an imino group (connected to the main-chain carbon through a carbon-nitrogen double bond), and an oxa-hydrocarbon group containing an ether bond, a thia-hydrocarbon group containing a thioether bond, and an aza-hydrocarbon group containing a tertiary amino group, etc. The main chain of the "carbon chain linker" is entirely composed of carbon atoms, and the pendant groups of the carbon chain are allowed to contain heteroatoms, that is, the main chain is constructed by connecting methylene groups or substituted methylene groups. The substituted methylene groups can have one monovalent substituent, two monovalent substituents, or one divalent substituent (e.g., divalent oxygen, one that forms a three-membered ring 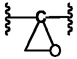 with a methylene group). The substituted methylene groups can have one hydrogen atom being substituted (e.g., -CH(CH₃)-), two hydrogen atoms being substituted respectively (e.g., - (CH₃)C(OCH₃)-), or two hydrogen atoms being simultaneously substituted (e.g., a carbonyl group, a thiocarbonyl group, -C(=NH)-, and -C(=N⁺H₂)-), or a cyclic pendant group (e.g., 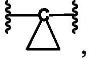 wherein the atomic spacing is 1).

In the present application, a compound or a group can be substituted and heterosubstituted simultaneously, e.g., a hydrogen atom can be replaced by a nitrophenyl group, and -CH₂-CH₂-CH₂- can be replaced by -CH₂-S-CH(CH₃)-. In the present application, "linking bond" without any atom is only for connection, that is, when a group is denoted as a linking bond, the group can be absent t.

In the present application, "each independently at each occurrence" not only means that different groups can be each independently selected from the definitions but also means that the same group at different positions can be independently selected from the definitions. For example, in -Z-L⁶-Z-, "Z is independently selected from the group consisting of -C(=O)-, - OC(=O)-, -C(=O)O-, -OC(=O)O-, -O-, -S-, -C(=O)S-, -SC(=O)-, -NRcC(=O)-, -C(=O)NR^{c}-, - NR^{c}C(=O)NR^{c}-, -OC(=O)NR^{c}-, -NR^{c}C(=O)O-, -SC(=O)NR^{c}- and -NR^{c}C(=O)S- at each occurrence; wherein, R^{c} is independently a hydrogen atom or a C₁₋₁₂ alkyl group at each occurrence"; two Z groups in "-Z-L⁶-Z-" can be the same or different, and two R^{c} groups in "-NR^{c}C(=O)NR^{c}-" can be the same or different, which are independently a hydrogen atom or a C₁₋₁₂ alkyl group.

In the present application, a "group" contains at least one atom, referring to the radical formed by a compound losing one or more atoms. With respect to a compound, the remaining group formed by the removal of other groups is also denoted as "residue". The valence of groups is not particularly limited, and examples include a monovalent group, a divalent group, a trivalent group, a tetravalent group, ..., a hectovalent group, etc. Wherein, groups with valence being equal to or greater than two are collectively defined as linking groups. A linking group can also contain only one atom, such as an oxo group and a thio group.

In the present application, "hydrocarbon" refers to a class of compounds that contain only carbon atoms and hydrogen atoms.

In the present application, hydrocarbons are classified into aliphatic hydrocarbons and aromatic hydrocarbons in terms of the type of hydrocarbon groups. Hydrocarbons containing neither phenyl rings nor hydrocarbyl-substituted phenyl rings are defined as aliphatic hydrocarbons. Hydrocarbons containing at least one phenyl ring or hydrocarbyl-substituted phenyl ring are defined as aromatic hydrocarbons. An aromatic hydrocarbon can contain one or more aliphatic hydrocarbyl groups, such as toluene, diphenylmethane, 2,3-dihydroindene, etc.

In the present application, "hydrocarbon" refers to a class of compounds that contain only carbon atoms and hydrogen atoms.

In the present application, hydrocarbons are classified into aliphatic hydrocarbons and aromatic hydrocarbons in terms of hydrocarbon groups. Hydrocarbons containing neither phenyl rings nor hydrocarbyl-substituted phenyl rings are defined as aliphatic hydrocarbons. Hydrocarbons containing at least one phenyl ring or hydrocarbyl-substituted phenyl ring are defined as aromatic hydrocarbons. An aromatic hydrocarbon can contain aliphatic hydrocarbyl groups, such as toluene, diphenylmethane, 2,3-dihydroindene, etc.

In the present application, hydrocarbons are classified into saturated hydrocarbons and unsaturated hydrocarbons in terms of the degree of saturation. All aromatic hydrocarbons are unsaturated hydrocarbons. Saturated aliphatic hydrocarbons are also termed alkanes. The degree of unsaturation of unsaturated aliphatic hydrocarbons is not particularly limited. For example, unsaturated aliphatic hydrocarbons include but are not limited to alkenes (containing carbon-carbon double bonds), alkynes (containing carbon-carbon triple bonds), dienes (containing two conjugated carbon-carbon double bonds), and the like. When the aliphatic moieties of aromatic hydrocarbons are saturated, the aromatic hydrocarbons are also termed aralkanes, such as toluene.

In the present application, the structures of hydrocarbons are not particularly limited, including linear structures without pendant groups, branched structures with pendant groups, ring-containing structures, dendritic structures, comb structures, hyperbranched structures, etc. Unless otherwise specified, preferable structures include linear structures without pendant groups, branched structures with pendant groups, and ring-containing structures, corresponding to linear hydrocarbons, branched hydrocarbons and cyclic hydrocarbons, respectively. Wherein, hydrocarbons that contain no rings are termed open-chain hydrocarbons, including but not limited to linear structures without pendant groups, and branched structures with pendant groups. Open-chain hydrocarbons belong to aliphatic hydrocarbons. Therefore, linear hydrocarbons are also referred to as linear aliphatic hydrocarbons and branched hydrocarbons are also referred to as branched aliphatic hydrocarbons.

In the present application, hydrocarbons with any carbon atom replaced by heteroatom are generally referred to as heterohydrocarbons.

In the present application, "hydrocarbon group" refers to the residue of a hydrocarbon molecule with at least one hydrogen atom being removed. According to the number of removed hydrogen atoms, hydrocarbon groups can be classified into monovalent hydrocarbon groups (with one hydrogen atom being removed), divalent hydrocarbon groups (with two hydrogen atoms being removed; also termed hydrocarbylene groups), trivalent hydrocarbon groups (with three hydrogen atoms being removed), and the like. Accordingly, when n hydrogen atoms are lost, the valence of the formed hydrocarbon group is n. Unless otherwise specified, hydrocarbon groups in the present application specifically refer to monovalent hydrocarbon groups. Unless otherwise expressly stated in this specification, a hydrocarbon group is optionally substituted.

In the present application, the source of hydrocarbon group is not particularly limited; for example, hydrocarbon group can be derived from aliphatic hydrocarbons or aromatic hydrocarbons, from saturated hydrocarbons or unsaturated hydrocarbons, from linear hydrocarbons, branched hydrocarbons or cyclic hydrocarbons, or from hydrocarbons or heterohydrocarbons, etc. According to the degree of saturation, hydrocarbon groups can be derived from alkanes, alkenes, alkynes, dienes, etc. Cyclic hydrocarbons can be derived, e.g., from alicyclic hydrocarbons, aromatic hydrocarbons, monocyclic hydrocarbons, or polycyclic hydrocarbons. Heterocyclic hydrocarbons can be derived, e.g., from aliphatic heterocyclic hydrocarbons or aromatic heterocyclic hydrocarbons.

In the present application, "aliphatic hydrocarbon group" refers to the residue of an aliphatic hydrocarbon molecule with at least one hydrogen atom being removed. Unless otherwise specified, aliphatic hydrocarbon groups in the present application specifically refer to monovalent hydrocarbon groups. The aliphatic group includes saturated aliphatic hydrocarbon groups and unsaturated aliphatic hydrocarbon groups. Unless otherwise expressly stated in this specification, aliphatic hydrocarbon groups are optionally substituted.

In the present application, "alkyl group" refers to a hydrocarbon group obtained from an alkane losing a hydrogen atom at any location, unless otherwise specified; the alkyl group can be linear or branched, substituted or unsubstituted. Specific examples include that a propyl group refers to either a 1-propyl group or an isopropyl group and that a propylene group refers to a 1,3-propylene group, a 1,2-propylene group, or an isopropylidene group. Unless otherwise expressly stated in this specification, alkyl groups are optionally substituted.

In the present application, "unsaturated hydrocarbon group" refers to the hydrocarbon group obtained from an unsaturated hydrocarbon losing hydrogen atoms. The hydrocarbon groups obtained from unsaturated hydrocarbons losing hydrogen atoms bonded to unsaturated carbon atoms, can include alkenyl groups, alkynyl groups, dienyl groups, and the like, and specifically, e.g., propenyl groups and propynyl groups. According to the type of unsaturated bond, the hydrocarbon groups formed by removing hydrogen atoms bonded to saturated carbon atoms of unsaturated hydrocarbons include alkenyl-hydrocarbyl groups, alkynyl-hydrocarbyl groups, dienyl-hydrocarbyl groups, and the like, and specifically, e.g., allyl groups and propargyl groups.

In the present application, "alkenyl group" refers to a substituted or unsubstituted alkenyl group with a linear structure or branched structure, containing two or more carbon atoms (e.g., 2, 3, 4, 5, 6, 7, 8, 9, 10, 11, 12, 13, 14, 15, 16, 17, 18, 19, 20 or more carbon atoms) and at least one carbon-carbon double bond. "C₂₋₁₅ alkenyl group" refers to a substituted or unsubstituted alkenyl group with a linear or branched structure containing 2 to 15 carbon atoms and at least one carbon-carbon double bond, that is, an alkenyl group can contain one, two, three, four, or more carbon-carbon double bonds. Unless otherwise specified, alkenyl groups include substituted and unsubstituted alkenyl groups in the present application. Unless otherwise expressly stated in this specification, alkenyl groups are optionally substituted.

In the present application, "alkynyl group" refers to an optionally substituted hydrocarbon with a linear or branched structure, containing two or more carbon atoms (such as 2, 3, 4, 5, 6, 7, 8, 9, 10, 11, 12, 13, 14, 15, 16, 17, 18, 19, 20, or more carbon atoms) and at least one carbon-carbon triple bond. "C₂₋₁₅ alkynyl group" refers to a substituted or unsubstituted alkynyl group with a linear or branched structure, containing 2 to 15 carbon atoms and at least one carbon-carbon triple bond, that is, an alkynyl group can contain one, two, three, four, or more carbon-carbon triple bonds. Unless otherwise specified, alkynyl groups include substituted and unsubstituted alkynyl groups in the present application. Unless otherwise expressly stated in this specification, alkynyl groups are optionally substituted.

In the present application, "hydrocarbylene group" or "hydrocarbylene chain" refers to a linear or branched divalent hydrocarbon chain that connects the remainder of a molecule to a free radical, consisting only carbon and hydrogen, being saturated or unsaturated. For example, a hydrocarbylene group with one to twenty-four carbon atoms (a C₁₋₂₄ hydrocarbylene group), a hydrocarbylene group with one to twelve carbon atoms (a C₁₋₁₂ hydrocarbylene group), and specifically, a methylene group, an ethylene group, a propylene group, n-butylene group, a vinylidene group, a propenylene group, an *n*-butenylene group, a propynylene group, an *n-*butynylene group, etc. Unless otherwise explicitly stated in this specification, hydrocarbylene groups are optionally substituted.

In the present application, "alkylene group" is also a divalent alkyl group, including an open-chain alkylene group and a divalent cycloalkyl group, and the open-chain alkylene group refers to a divalent alkyl group without any cyclic structure, and the divalent cycloalkyl group refers to a divalent alkyl group containing a cyclic structure. Unless otherwise expressly stated in this specification, alkylene groups are optionally substituted.

In the present application, aliphatic hydrocarbon derivatives are preferably ether-derived aliphatic hydrocarbons, preferably aliphatic hydrocarbon derivatives containing one or two ether bonds, and more preferably aliphatic hydrocarbon derivatives containing two ether bonds

In the present application, "molecular weight" refers to the mass of a compound. The measuring unit of "molecular weight" is Dalton (Da), unless otherwise specified.

In the present application, the term "about before a percentage refers to a range of ±0.5%.

In the present application, the terms "stable" and "degradable" are a pair of opposing concepts. For detailed examples of stable groups and degradable groups are given in paragraphs [0134]-[0145] in CN113402405A.

In the present application, "hydroxyl protecting group" includes all the groups that can be used as common hydroxyl protecting groups. A hydroxyl protecting group is preferably selected from the group consisting of an alkanoyl group (e.g., an acetyl group, a butyryl group), an aromatic alkanoyl group (e.g., a benzoyl group), a benzyl group, a triphenylmethyl group, a trimethylsilyl group, a *t*-butyldimethylsilyl group, an allyl group, an acetal group, or a ketal group. Removal of acetyl groups is generally carried out under basic conditions, most commonly by the ammonolysis with NH₃/MeOH or by the methanolysis catalyzed by methanol anions. The benzyl group can be easily removed via palladium-catalyzed hydrogenolysis in a neutral solution at room temperature, or via a reduction reaction by metallic sodium in ethanol or liquid ammonia. The triphenylmethyl group is generally removed via catalytic hydrogenolysis. The trimethylsilyl group is generally removed using reagents containing fluoride ions (e.g., tetrabutylammonium fluoride/anhydrous THF, etc.). The t-butyldimethylsilyl ether is relatively stable and can withstand ester hydrolysis conditions with alcoholic potassium hydroxide and mild reduction conditions (e.g., Zn/CH₃OH and the like), so that it can be removed by fluoride ions (e.g., Bu₄N⁺F⁻) in THF solution or by aqueous acetic acid at room temperature.

In the present application, "carboxyl protecting group" refers to the protecting group which can be transformed into a carboxyl group via the hydrolysisor the deprotection reaction of a carboxyl protecting group itself. A carboxyl protecting group is preferably selected from the group consisting of an alkyl group (e.g., a methyl group, an ethyl group, and a butyl group) and an aralkyl group (e.g., a benzyl group), and more preferably selected from the group consisting of a butyl group (tBu), a methyl group (Me) and an ethyl group (Et).

In the present application, "protected carboxyl group" refers to the group protected by an appropriate carboxyl protecting group, preferably selected from the group consisting of a methoxycarbonyl group, an ethoxycarbonyl group, a *t*-butoxycarbonyl group, and a benzyloxycarbonyl group. The carboxyl protecting groups can be removed through hydrolysis catalyzed by acids or alkalis, or through pyrolysis reactions occasionally; for example, the t-butyl groups can be removed under mild acidic conditions, and the benzyl group can be removed by hydrogenolysis. The reagent used for removal of carboxyl protecting groups is selected from the group consisting of TFA, H₂O, LiOH, NaOH, KOH, MeOH, EtOH and combinations thereof, preferably selected from the group consisting of the combination of TFA and H₂O, the combination of LiOH and MeOH, and the combination of LiOH and EtOH. A protected carboxyl group can undergo deprotection and then produce the corresponding free acid; the deprotection is conducted in the presence of an alkali, and the alkali forms a pharmaceutically acceptable salt with the free acid produced via the deprotection.

In the present application, "amino protecting group" includes all the groups which are used as amino protecting groups generally, such as an aryl C₁₋₆ alkyl group, a C₁₋₆ alkoxy C₁₋₆ alkyl group, a C₁₋₆ alkoxycarbonyl group, an aryloxycarbonyl group, a C₁₋₆ alkylsulfonyl group, an arylsulfonyl group, a methylsilyl group, etc. An amino protecting group is preferably selected from the group consisting of a *t*-butoxycarbonyl group (Boc), a *p-*methoxybenzyloxycarbonyl group (Moz), and a 9-fluorenylmethyloxycarbonyl (Fmoc). The reagent used for removal of amino protecting groups is selected from the group consisting of TFA, H₂O, LiOH, NaOH, KOH, MeOH, EtOH and combinations thereof, preferably selected from the group consisting of the combination of TFA and H₂O, the combination of LiOH and MeOH, and the combination of LiOH and EtOH. The reagent used for removal of the Boc protecting group can be TFA or HCl/EA, preferably TFA. The reagent used for the removal of the Fmoc protecting group can be the *N,N-*dimethylformamide (DMF) solution containing 20% piperidine.

In the present application, "cation" refers to the corresponding structure bearing a positive charge, either permanently, or non-permanently but in response to certain conditions such as pH. Therefore, the cations include permanent cations and those cationic compounds, groups, or atoms. Permanent cations refer to the corresponding compounds, groups, or atoms that bear positive charges under conditions of any pH value or hydrogen ion activity of their environment; typically, a positive charge is generated by the presence of quaternary nitrogen atom. When a compound carries multiple such positive charges, it can be termed a permanent cation. A cationic substance refers to a compound, group, or atom that is positively charged at a lower pH and uncharged at a higher pH of its environment. Moreover, in non-aqueous environments where pH cannot be determined, a cationic compound, group, or atom is positively charged at high hydrogen ion concentration and uncharged at low concentration or activity of hydrogen ions, which depends on the individual properties of the cationic or polycationic compound, in particular the pKa of the respective cationic group or atom which are charged or uncharged at the corresponding pH or hydrogen ion concentration. In the diluted aqueous environment, the Henderson-Hasselbalch equation can be used to estimate the fraction of positively charged cationic compounds, groups, or atoms, which is well-known to those skilled in the art. For example, in some embodiments, if a compound or moiety is cationic, pH of the compound or moiety is preferably about 1 to 9 , preferably 4 to 9, 5 to 8, or even 6 to 8, more preferably equal to or below 9, equal to or below 8, equal to or below 7, and most preferably at physiological pH values (e.g., about 7.3 to 7.4), i.e., positively charged under physiological conditions, especially under normal saline conditions in cells in vivo. In other embodiments, it is preferable that the cationic compound or moiety is predominantly neutral at physiological pH values, e.g., about 7.0 to 7.4, but becomes positively charged at lower pH values. In some embodiments, pKa for the cationic compound or moiety is preferably about 5 to about 7.

In the present application, "cationic lipid" refers to a lipid having a positive charge or being ionizable. In addition to the structural general formula (1) of the present application, cationic lipids include, but are not limited to, *N,N*-dioleyl-*N,N*-dimethylammonium chloride (DODAC), *N,N*-distearyl-*N,N*-dimethylammonium bromide (DDAB), *N*-(1-(2,3-dioleoyloxy)propyl)-*N,N,N*-trimethylammonium chloride (DOTAP), *N*-(1-(2,3-dioleyloxy)propyl)-*N,N,N*-trimethylammonium chloride (DOTMA), *N,N*-dimethyl-2, 3-Dioilyloxypropylamine (DODMA), 3-(didodecylamino)-*N1*,*N1*,4-tridodecyl-1-piperazineethanamine (KL10), *N-*[2(didodecylamino)ethyl]-*N1,N4,N4*-tridodecyl-1,4-piperazinediethanamine (KL22), 14,25-ditridecyl-15,18,21,24-tetraaza-octatriacontane (KL25), 1,2-Dileutheroxy-*N*, *N*-Dimethylaminopropane (DLin-DMA), 2,2-Dilinoleyl-4-dimethylaminomethyl-[1,3]-dioxolane (DLin-K-DMA), heptatriaconta-6,9,28,31-tetraen-19-yl 4-(dimethylamino)butanoate (DLin-MC3-DMA) and 2,2-dilinoleyl-4-(2dimethylaminoethyl)-[1,3]-dioxolane (DLin-KC2-DMA), ((4-hydroxybutyl)azanediyl)bis(hexane-6,1-diyl)bis(2-hexyldecanoate) (ALC-0315), heptadecan-9-yl 8-((2-hydroxyethyl) (6-oxo-6-(undecyloxy) hexyl) amino) octanoate) (SM102) and any mixture thereof.

In the present application, "PEGylated lipid" (i.e., PEG-lipid) refers to the molecules containing both lipid and PEG moieties.

In the present application, "neutral lipid" refers to any lipid substance that is uncharged or exists in the form of neutral zwitterion at the chosen pH, preferably phospholipid. The neutral lipid can be synthetic or natural.

In the present application, "steroid lipid" refers to a steroid or steroid analog.

In the present application, "variant form" refers to a structure that can be transformed into the target reactive group after any process of chemical change selected from the group consisting of oxidation, reduction, hydration, dehydration, electronic rearrangement, structural rearrangement, salt complexation and decomplexation, ionization, protonation, deprotonation, substitution, deprotection, leaving group transformation, etc.

In the present application, "variant form of reactive group" refers to a reactive form which still has reactivity (remains a reactive group) after the reactive group undergoes at least one process of chemical change selected from oxidation, reduction, hydration, dehydration, electronic rearrangement, structural rearrangement, salt complexation and decomplexation, ionization, protonation, deprotonation, substitution, deprotection, leaving group transformation, etc., or a non-reactive form of a protected reactive group.

In the present application, "micro-modification" refers to a process of chemical modification that can be completed through simple chemical reactions. Simple chemical reactions mainly include deprotection, salt complexation and decomplexation, ionization, protonation, deprotonation, leaving group transformation, etc. The "variant form with micro-modification" corresponds to the "micro-modification", referring to a structure that can be transformed into the target reactive group after simple chemical reactions selected from deprotection, salt complexation and decomplexation, ionization, protonation, deprotonation, leaving group transformation, etc., the leaving group transformation including the transformation from the form of ester to the form of acyl chloride.

In the present application, "N/P ratio" refers to a molar ratio of nitrogen atoms in cationic lipids to phosphates in nucleic acids.

In the present application, "nucleic acid" refers to DNA, RNA, or their modified form.

In the present application, "RNA" refers to a ribonucleic acid that may be naturally or non-naturally occurring. For example, an RNA may include modified and/or non-naturally occurring components such as one or more nucleobases, nucleosides, nucleotides, and linkers. An RNA may include a cap structure, a chain-terminating nucleoside, a stem-loop, a polyA sequence, and/or a polyadenylation signal. An RNA may have a nucleotide sequence encoding a polypeptide of interest. For example, an RNA may be a messenger RNA (mRNA). Translation of an mRNA encoding a particular polypeptide, for example, in vivo translation of an mRNA inside a mammalian cell, may produce the encoded polypeptide. RNAs may be selected from the non-limiting group consisting of small interfering RNA (siRNA), asymmetrical interfering RNA (aiRNA), microRNA (miRNA), Dicer-substrate RNA (dsRNA), small hairpin RNA (shRNA), mRNA (messenger RNA), single guide RNA (sgRNA), cas9 mRNA, and mixtures thereof.

In the present application, FLuc mRNA can express luciferase protein which emits bioluminescence in the presence of fluorescein substrate, so FLuc is commonly used in mammalian cell culture to measure gene expression and cell activity.

In the present application, suitable assays for determining the level of target gene expression include but are not limited to dot blot, northern blot, in situ hybridization, ELISA, immunoprecipitation, enzyme function, and phenotypic assays.

In the present application, "transfection" refers to the introduction of a species (e.g., an RNA) into a cell. Transfection may occur, for example, in vitro, ex vivo, or in vivo.

In the present application, "antigen" typically refers to a substance that can be recognized by the immune system, preferably recognized by the adaptive immune system, and trigger an antigen-specific immune response, for example, forming antibodies and/or antigen-specific T cells as a part of the adaptive immune response. Typically, the antigen may be or may contain a peptide or a protein that can be presented to T cells by MHC. In the present application, the antigen may be a translation product of the provided nucleic acid molecule (preferably mRNA as defined herein). In this context, fragments, variants, and derivatives of peptides and proteins containing at least one epitope are also defined as antigens.

In the present application, "delivery" refers to delivering an entity to the target, for example, delivering drugs and/or therapeutic agents and/or prophylactic agents to subjects, the subjects are tissues and/or cells of humans and/or other animals.

In the present application, "pharmaceutically acceptable carrier" refers to a diluent, adjuvant, excipient, or vehicle administered together with the therapeutic agent, which is, within the scope of reasonable medical judgment, suitable for contact with tissues of human and/or other animals without causing excessive toxicity, irritation, allergic reaction, or other problems or complications corresponding to reasonable benefit/risk ratio. Pharmaceutically acceptable carriers that can be used in the pharmaceutical composition in the present application include but are not limited to sterile liquids, such as water and oil, including those from petroleum, animal, vegetable, or synthesis, e.g., peanut oil, soybean oil, mineral oil, sesame oil, etc. When the pharmaceutical composition is administered intravenously, water is an exemplary carrier. Physiological saline, glucose, and aqueous glycerol solution can also be used as liquid carriers, especially as an injection. Suitable pharmaceutical excipients include starch, glucose, lactose, sucrose, gelatin, maltose, chalk, silica gel, sodium stearate, glyceryl monostearate, talc, sodium chloride, defatted milk powder, glycerol, propylene glycol, water, ethanol, etc. The composition can also contain a small amount of humectant, emulsifier, or pH buffer as needed. Oral preparations can contain standard carriers, such as pharmaceutical-grade mannitol, lactose, starch, magnesium stearate, sodium saccharin, cellulose, magnesium carbonate, etc. Specifically, excipients include but are not limited to anti-adherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspending or dispersing agents, sweeteners, and waters of hydration. More specifically, excipients include but are not limited to butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E (a-tocopherol), vitamin C, and xylitol.

In the present application, pharmaceutical compositions can act systematically and/or locally. For this purpose, they can be administered by appropriate routes such as injection (e.g., intravenous, intraarterial, subcutaneous, intraperitoneal, or intramuscular injection, including instillation) and transdermal delivery, and can also be administered by oral, buccal, transnasal, transmucosal, or topical routes, or in the form of ophthalmic preparation, or by inhalation. Regarding these routes of administration, the pharmaceutical compositions of the present application can be administered in suitable dosage forms. The dosage forms include but are not limited to, tablets, capsules, lozenges, hard sugar agents, powders, sprays, creams, ointments, suppositories, gels, pastes, lotions, ointments, aqueous suspensions, injectable solutions, elixirs, and syrups.

In the present application, vaccines are preventive or therapeutic materials that provide at least one antigen or antigenic function. Antigen or antigenic function can stimulate the body's adaptive immune system to provide an adaptive immune response.

In the present application, treatment refers to the management and care of patients to resist diseases, obstacles, or symptoms, which is intended to delay the development of diseases, obstacles, or symptoms, reduce or alleviate symptoms and complications, and/or cure or eliminate diseases, obstacles, or symptoms. The patients to be treated are preferably mammals, especially humans.

### DETAILED DESCRIPTION OF THE APPLICATION

**An embodiment of the present application:**

### 1.1. A cationic lipid; wherein, its structure is represented by the general formula (1):

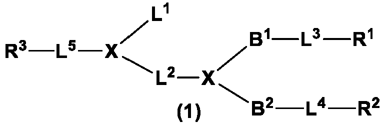

Wherein, X is independently N or CR^{a} at each occurrence, and the R^{a} is H or a C₁₋₁₂ alkyl group;
L¹ is -L°R; L^{c} is a linking bond or a divalent linking group, and R is a C₁₋₃₀ aliphatic hydrocarbon group; wherein, the aliphatic hydrocarbon group is an alkyl group, an alkenyl group or an alkynyl group;
L² is a linking bond or a divalent linking group;
L⁵ is a linking bond or a divalent linking group;
L³ and L⁴ are each independently a linking bond or a divalent linking group;
B¹ and B² are each independently a linking bond or a C₁₋₃₀ alkylene group;
R¹ and R² are each independently a C₁₋₃₀ aliphatic hydrocarbon group or represented by 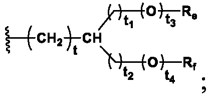 wherein, t is an integer from 0 to 12; t₁ and t₂ are each independently an integer from 0 to 5; t₃ and t₄ are each independently 0 or 1; t₁, t₂, t₃ and t₄ are not be 0 simultaneously; Rₑ and R_{f} are each independently selected from the group consisting of a C₁₋₁₅ alkyl group, a C₂₋₁₅ alkenyl group and a C₂₋₁₅ alkynyl group;
R³ is selected from the group consisting of a hydrogen atom, -R^{d}, -OR^{d}, a C₃₋₆ carbocyclic group, a nitrogen-containing heterocyclyic group, -NR^{d}R^{d}, -SR^{d}, -C(=O)R^{d}, -C(=O)OR^{d}, - OC(=O)R^{d}, -OC(=O)OR^{d} and a functional group R⁰¹ that can interact with bio-related substances; wherein, R^{d} is a C₁₋₁₂ alkyl group;
or a salt, tautomer, stereoisomer, or solvate thereof.

### 1.1.1. X

In the present application, X is independently N or CR^{a} at each occurrence; wherein, R^{a} is H or a C₁₋₁₂ alkyl group.

### 1.1.2. L¹, L², L³, L⁴, L⁵, L⁶, L⁷, Z

In the present application, the structures of L¹, L², L³, L⁴, L⁵, L⁶, L⁷, and Z are not particularly limited, and each independently includes but is not limited to a linear structure, a branched structure or a ring-containing structure.

In the present application, the number of non-hydrogen atoms in L¹, L², L³, L⁴, L⁵, L⁶, L⁷, or Z is not particularly limited, and are each independently 1 to 50, more preferably 1 to 20, more preferably 1 to 10. The non-hydrogen atom is a carbon atom or a heteroatom. The heteroatom includes, but is not limited to, O, S, N, P, SI, B, etc. When the number of non-hydrogen atoms is one, the non-hydrogen atom can be a carbon atom or a heteroatom. When the number of non-hydrogen atoms is greater than one, there is no special restriction on the types of non-hydrogen atoms, which can be one, two, or more types; when the number of non-hydrogen atoms is greater than one, it can be any combination of carbon atoms and carbon atoms, carbon atoms and heteroatoms, or heteroatoms and heteroatoms.

In the present application, two identical or different reactive groups may be reacted to form a divalent linking group. The reaction conditions, which are related to the type of divalent linking group obtained from the reaction, may be based on the prior art. For example: amino groups react with active esters, formic acid active ester, sulfonate, aldehydes, α,β-unsaturated bonds, carboxylic acid groups, epoxides, isocyanates, and isothiocyanates, respectively, to obtain divalent linking groups of amido groups, urethane group, amino groups, imine groups (which can be further reduced to secondary amino groups), amino groups, amido groups, amino alcohol, urea bonds, thiourea bonds, etc.; sulfhydryl groups react with active esters, formic acid active ester, sulfhydryl groups, maleimides, aldehydes, α,β-unsaturated bonds, carboxylic acid groups, iodoacetamides, and anhydrides, respectively, to obtain divalent linking groups of thioester groups, thiocarbonates, thioethers, disulfides, thioethers, thiohemiacetals, thioethers, thioesters, thioethers, imides, etc.; unsaturated bonds react with sulfhydryl groups to obtain thioether groups; carboxyl groups or acyl halides react with sulfhydryl groups and amino groups, respectively, to obtain thioester groups, amide groups, etc.; hydroxyl group reacts with carboxyl groups, isocyanates, epoxides, chloroformyloxy groups, respectively, to obtain divalent linking groups of ester groups, carbamate groups, ether bonds, carbonate groups, etc.; carbonyl groups or aldehyde groups react with amino groups, hydrazines, hydrazides, respectively, to obtain divalent linking groups of imine bonds, hydrazones, acylhydrazones, etc.; and, azides, alkynyl groups, alkenyl groups, sulfhydryl group, azides, dienes, maleimides, 1,2,4-triazoline-3,5-dione, dithioesters, hydroxylamines, hydrazides, acrylates, allyloxy groups, isocyanates, tetrazoles and other reactive groups can undergo click reactions to generate various divalent linking groups including but not limited to triazoles, isoxazoles, thioether bonds and other structures.

L¹, L², L³, L⁴, L⁵, L⁶, L⁷, and Z are not particularly limited with respect to the stability. Any divalent linking group thereof, or a divalent linking group composed of any divalent linking group thereof and adjacent heteroatom groups, is independently a stable linking group STAG or a degradable group DEGG.

### 1.1.2.1. L¹

In the present application, L¹ is -L°R; L° is a linking bond or a divalent linking group, and R is a C₁₋₃₀ aliphatic hydrocarbon group; wherein, the aliphatic hydrocarbon group is an alkyl group, an alkenyl group or an alkynyl group.

In a specific embodiment of the present application, L^{c} is preferably selected from the group consisting of a linking bond, -(CH₂)ₜₘ-, -(CH₂)ₜₘZ-, -Z(CH₂)ₜₘ-, -(CH₂)ₜₘZ(CH₂)ₜₘ- and -(CH₂)ₜₘZ(CH₂)ₜₘZ-; wherein, tm is independently an integer from 1 to 12 at each occurrence; the Z is independently selected from the group consisting of -C(=O)-, -OC(=O)-, -C(=O)O-, - OC(=O)O-, -O-, -S-, -C(=O)S-, -SC(=O)-, -NR^{c}C(=O)-, -C(=O)NR^{c}-, -NR^{c}C(=O)NR^{c-}, - OC(=O)NR^{c}-, -NR^{c}C(=O)O-, -SC(=O)NR^{c}- and -NR^{c}C(=O)S- at each occurrence; wherein, R^{c} is independently H or a C₁₋₁₂ alkyl group at each occurrence; the L° is preferably selected from the group consisting of a linking bond, -(CH₂)ₜₘ-, -(CH₂)ₜₘO-, -(CH₂)ₜₘC(=O)-, - (CH₂)ₜₘC(=O)O-, -(CH2)ₜₘOC(=O)-, -(CH₂)ₜₘC(=O)NH-, -(CH₂)ₜₘNHC(=O)-, - (CH₂)ₜₘOC(=O)O-, -(CH₂)ₜₘNHC(=O)O-, -(CH₂)ₜₘOC(=O)NH-, -(CH₂)ₜₘNHC(=O)NH-, - (CH₂)ₜₘO(CH₂)ₜₘ-, -(CH₂)ₜₘC(=O)(CH₂)ₜₘ-, -(CH₂)ₜₘC(=O)O(CH₂)ₜₘ-, - (CH₂)ₜₘOC(=O)(CH₂)ₜₘ-, -(CH₂)ₜₘC(=O)NH(CH2)ₜₘ-, -(CH₂)ₜₘNHC(=O)(CH₂)ₜₘ-, - (CH₂)ₜₘOC(=O)O(CH₂)ₜₘ-, -(CH₂)ₜₘNHC(=O)O(CH₂)ₜₘ-, -(CH₂)ₜₘOC(=O)NH(CH₂)ₜₘ-, and - (CH₂)ₜₘNHC(=O)NH(CH₂)ₜₘ-, and more preferably selected from the group consisting of a linking bond, -(CH₂)ₜₘO(CH₂)ₜₘ-, -(CH₂)ₜₘC(=O)O(CH₂)ₜₘ- and -(CH₂)ₜₘOC(=O)(CH₂)ₜₘ-.

In a specific embodiment of the present application, R is preferably selected from the group consisting of a linear alkyl group, a branched alkyl group, a linear alkenyl group, a branched alkenyl group, a linear alkynyl group, and a branched alkynyl group; R is preferably a linear alkyl group or a linear alkenyl group, and more preferably a C₁₋₂₅ linear alkyl group, and more preferably a C₁₋₁₇ linear alkyl group; specifically, R is selected from the group consisting of a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group and a heptadecyl group.

### 1.1.2.2. L²

In the present application, L² is a linking bond or a divalent linking group.

In a specific embodiment of the present application, L² is selected from the group consisting of a linking bond, -(CH₂)ₘ-, -(CH₂)ₘC(=O)-, -C(=O)(CH₂)ₘ- and - C(=O)(CH₂)ₘC(=O)-; wherein, m is an integer from 1 to 4.

### 1.1.2.3. L³, L⁴

In the present application, L³ and L⁴ are each independently a linking bond or a divalent linking group.

In a specific embodiment of the present application, L³ and L⁴ are preferably selected from the group consisting of a linking bond, -OC(=O)-, -C(=O)O-, -OC(=O)O- -C(=O)-, -O-, - O(CR^{c}R^{c})ₛO-, -S-, -C(=O)S-, -SC(=O)-, -NR^{c}C(=O)-, -C(=O)NR^{c}-, -NR^{c}C(=O)NR^{c}-, - OC(=O)NR^{c}-, -NR^{c}C(=O)O-, -SC(=O)NR^{c}- and -NR^{c}C(=O)S-; wherein, R^{c} is independently a hydrogen atom or C₁₋₁₂ alkyl group at each occurrence, and s is two, three, or four.

In a specific embodiment of the present application, L³ and L⁴ are more preferably selected from any one of the following cases:
Case (1): one of L³ and L⁴ is a linking bond, and the other is selected from the group consisting of -OC(=O)-, -C(=O)O-, -OC(=O)O-, -C(=O)-, -O-, -O(CR^{c}R^{c})ₛO-, -S-, -C(=O)S-, -SC(=O)-, -NR^{c}C(=O)-, -C(=O)NR^{c}-, -N^{Rc}C(=O)NR^{c}-, -OC(=O)NR^{c}-, -NR^{c}C(=O)O-, - SC(=O)NR°-, and -NR^{c}C(=O)S-;
Case (2): both L³ and L⁴ are linking bonds;
Case (3): L³ and L⁴ are each independently selected from the group consisting of - OC(=O)-, -C(=O)O-, -OC(=O)O-, -C(=O)-, -O-, -O(CH₂)ₛO-, -S-, -C(=O)S-, -SC(=O)-, - NHC(=O)-, -C(=O)NH-, -NHC(=O)NH-, -OC(=O)NH-, -NHC(=O)O-, -SC(=O)NH- and - NHC(=O)S-.

In a specific embodiment of the present application, it is more preferably that L³ and L⁴ are each independently selected from the group consisting of -OC(=O)-, -C(=O)O- and - OC(=O)O-.

In a specific embodiment of the present application, it is more preferably that one of L³ and L⁴ is -OC(=O)O- and the other is -OC(=O)- or -C(=O)O-.

In a specific embodiment of the present application, it is more preferably that both L³ and L⁴ are -OC(=O)-, -C(=O)O-, or -OC(=O)O-, simultaneously.

### 1.1.2.4. L⁵

In the present application, L⁵ is a linking bond or a divalent linking group.

In a specific embodiment of the present application, L⁵ is preferably a divalent linking group, which is selected from the group consisting of L⁶, L⁷, Z, and combinations of any two or more thereof; more preferably, L⁵ is selected from the group consisting of -L⁶-, -L⁶-Z-, -Z-L⁶- -Z-L⁶-Z-, -L⁶-Z-L⁷- -Z-L⁶-Z-L⁷-, -L⁶-Z-L⁷-Z-, -Z-L⁶-Z-L⁷-Z-, and -L⁶-Z-L⁷-Z-L⁶-Z-; wherein, L⁶ and L⁷ are carbon-chain linking groups, and are each independently represented by -(CR^{a}R^{b})ₜ-(CR^{a}R_{b})ₒ-(CR^{a}R^{b})ₚ-, and t, o, p are each independently an integer from 0 to 12, and t, o, p are not 0 simultaneously; R^{a} and R^{d} are each independently a hydrogen atom or a C₁₋₁₂ alkyl group at each occurrence; the Z is independently selected from the group consisting of -C(=O)-, -OC(=O)-, -C(=O)O-, -OC(=O)O-, -O-, -S-, -C(=O)S-, -SC(=O)-, -NR^{c}C(=O)-, - C(=O)NR^{c}- -NR^{c}C(=O)NR^{c}-, -OC(=O)NR^{c}-, -NR^{c}C(=O)O-, -SC(=O)NR^{c}- and -NR^{c}C(=O)S-; wherein, R^{c} is independently H or a C₁₋₁₂ alkyl group at each occurrence.

In a specific embodiment of the present application, L⁵ is more preferably selected from the group consisting of a linking bond, -(CH₂)ₜₙZ-, -Z(CH₂)ₜₙ-, -Z(CH₂)ₜₙZ-, -(CH₂)ₜₙZ(CH₂)ₜₙZ- and -Z(CH₂)ₜₙZ(CH₂)ₜₙZ-; wherein, tn is independently an integer from 1 to 12 at each occurrence; L⁵ is more preferably selected from the group consisting of a linking bond, - (CH₂)ₜₙO-, -(CH₂)ₜₙC(=O)O-, -(CH₂)ₜₙOC(=O)-, -(CH₂)ₜₙNHC(=O)-, -(CH₂)ₜₙOC(=O)O-, - (CH₂)ₜₙNHC(=O)O-, -O(CH₂)ₜₙO-, -C(=O)O(CH₂)ₜₙC(=O)O-, -OC(=O)(CH₂)ₜₙOC(=O)-, - C(=O)O(CH₂)ₜₙOC(=O)-, -OC(=O)(CH₂)ₜₙC(=O)O-, -OC(=O)O(CH₂)ₜₙOC(=O)O-, - NHC(=O)O(CH₂)ₜₙNHC(=O)O-, -OC(=O)NH(CH₂)ₜₙNHC(=O)O-, -C(=O)(CH₂)ₜₙO-, - C(=O)(CH₂)ₜₙC(=O)O-, -C(=O)(CH₂)ₜₙOC(=O)-, -C(=O)(CH₂)ₜₙOC(=O)O-, - C(=O)(CH₂)ₜₙNHC(=O)O- and -C(=O)(CH₂)ₜₙC(=O)(CH₂)ₜₙNHC(=O)O-, and more preferably selected from the group consisting of a linking bond, -C(=O)O-, -(CH₂)ₜₙO-, -(CH₂)ₜₙC(=O)O-, -(CH₂)ₜₙO-, -C(=O)(CH₂)ₜₙO-, -C(=O)(CH₂)ₜₙC(=O)O-, -C(=O)(CH₂)ₜₙNHC(=O)O- and - C(=O)(CH₂)ₜₙC(=O)(CH₂)ₜₙNHC(=O)O-.

### 1.1.3. B¹, B²

In the present application, B¹ and B² are each independently a linking bond or a C₁₋₃₀ alkylene group.

In a specific embodiment of the present application, B¹ and B² are preferably each independently a linking bond or a C₁₋₂₀ alkylene group; more preferably, B¹ and B² are selected from any one of the following cases:
Case (1): B¹ and B² are each independently a C₁₋₂₀ alkylene group; specifically, B¹ and B² are each independently selected from the group consisting of a methylene group, an ethylene group, a propylene group, a butylene group, a pentylene group, a hexylene group, a heptylene group, an octylene group, a nonylene group, a decylene group, a undecylene group, a dodecylene group, a tridecylene group, a tetradecylene group, a pentadecylene group, a hexadecylene group, a heptadecylene group, an octadecylene group, a nonadecylene group and an eicosylene group; more preferably, B¹ and B² are each independently a C₂₋₁₀ alkylene group;
Case (2): one of B¹ and B² is a linking bond, and the other is a C₁₋₂₀ alkylene group.
Case (3): both B¹ and B² are linking bonds.

### 1.1.4. R¹, R², R, R^{c}

### 1.1.4.1. R¹, R²

In the present application, R¹ and R² are each independently a C₁₋₃₀ aliphatic hydrocarbon group or represented by

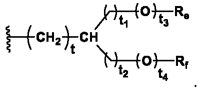

In a specific embodiment of the present application, R¹ and R² are each independently a C₁₋₃₀ linear aliphatic hydrocarbon group, a C₁₋₃₀ branched aliphatic hydrocarbon group or represented by

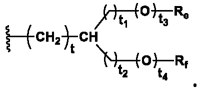

In a specific embodiment of the present application, the linear aliphatic hydrocarbon group is a linear alkyl group, a linear alkenyl group or a linear alkynyl group, more preferably a C₁₋₂₅ linear aliphatic hydrocarbon group, and more preferably a C₅₋₁₇ linear aliphatic hydrocarbon group.

In a specific embodiment of the present application, the branched aliphatic hydrocarbon group is a branched alkyl group, a branched alkenyl group or a branched alkynyl group, and is represented by

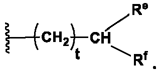

In one specific embodiment of the present application, R¹ and R² are each independently represented by 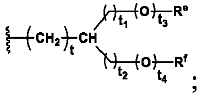 wherein, t is an integer from 0 to 12, t₁ and t₂ are each independently an integer from 0 to 5, t₃ and t₄ are each independently 0 or 1, and t₁, t₂, t₃, and t₄ are not 0 simultaneously; R^{e} and R^{f} are each independently a C₁₋₁₅ alkyl group, a C₂₋₁₅ alkenyl group or a C₂₋₁₅ alkynyl group; more preferably, R^{e} and R^{f} are each independently selected from the group consisting of a methyl group, an ethyl group, a propyl group, a butyl group, a pentyl group, a hexyl group, a heptyl group, an octyl group, a nonyl group, a decyl group, a undecyl group, a dodecyl group, a tridecyl group, a tetradecyl group, a vinyl group, a propenyl group, an allyl group, a butenyl group, an allyl carbinyl group, a pentenyl group, a neopentyl group, a hexenyl group, a neohexenyl group, a heptenyl group, a neoheptenyl group, an octenyl group, a neooctenyl group, a nonenyl group, a neononenyl group, a decenoyl group, a neodecenoyl group, an ethynyl group, a propynyl group, a propargyl group, a butynyl group, a butynediyl group, a pentynyl group, a neopentyl group, a hexynyl group, a neohexyl group, a heptynyl group, a neoheptyl group, an octynyl group, a neooctyl group, a nonynyl group, a neononyl group, a decynylgroup, and a neodecyl group; more preferably, R^{e} and R^{f} are each independently selected from the group consisting of a methyl group, an ethyl group, a propyl group, a butyl group, a pentyl group, a hexyl group, a heptyl group, an octyl group, a nonyl group and a decyl group.

In a specific embodiment of the present application, R¹ and R² are preferably selected from any one of the following cases:
Case (1): one of R¹ and R² is a C₁₋₃₀ linear aliphatic hydrocarbon group, and the other is a C₁₋₃₀ branched aliphatic hydrocarbon group;
Case (2): R¹ and R² are each independently a C₁₋₃₀ branched aliphatic hydrocarbon group represented by 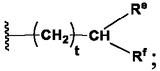
Case (3): R¹ and R² are each independently represented by 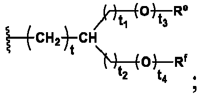
Case (4): one of R¹ and R² is a C₁₋₃₀ linear aliphatic hydrocarbon group or a C₁₋₃₀ branched aliphatic hydrocarbon group represented by 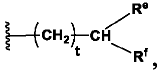 and the other is represented by

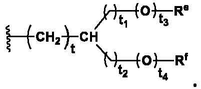

In a more specific embodiment of the present application, the aforementioned R¹ and R² are further preferably each independently selected from the group consisting of the following structures:

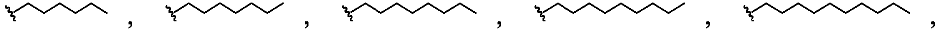

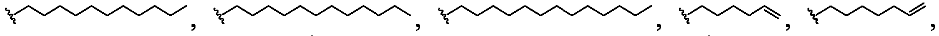

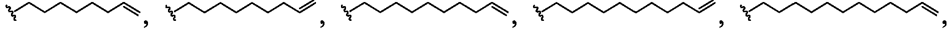

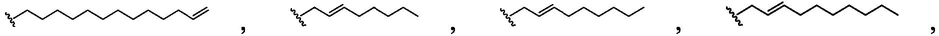

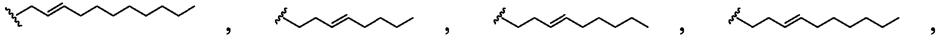

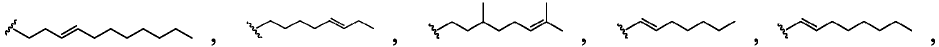

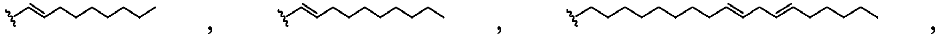

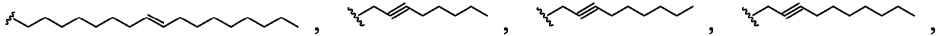

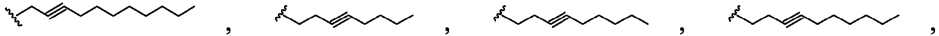

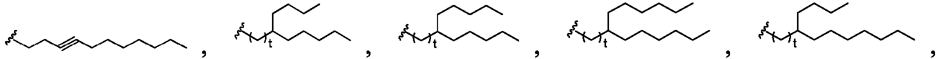

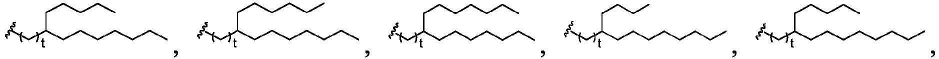

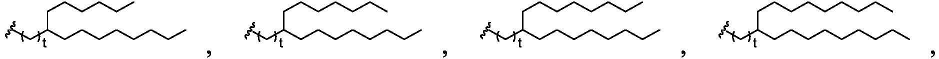

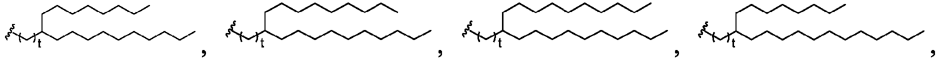

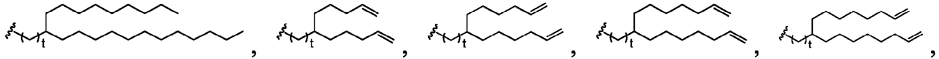

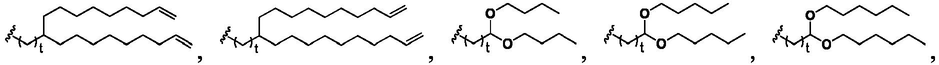

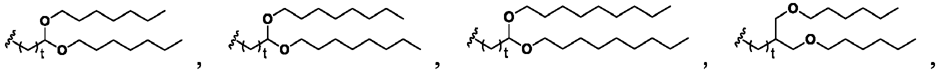

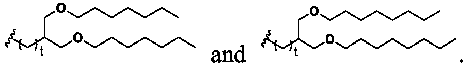

### 1.1.4.2. R

In the present application, R is a C₁₋₃₀ aliphatic hydrocarbon group.

In a specific embodiment of the present application, R is a linear alkyl group, a branched alkyl group, a linear alkenyl group, a branched alkenyl group, a linear alkynyl group or a branched alkynyl group, preferably a linear alkyl group or a linear alkenyl group, more preferably a C₁₋₂₅ linear alkyl group, more preferably a C₁₋₁₇ linear alkyl group; specifically, R is selected from the group consisting of a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group and a heptadecyl group.

### 1.1.4.3. R^{c}

In the present application, R^{c} is independently H or a C₁₋₁₂ alkyl group at each occurrence.

In one specific embodiment of the present application, R^{c} is preferably a hydrogen atom or a C₁₋₈ alkyl group, more preferably a hydrogen atom or a methyl group.

### 1.1.5. R³

In the present application, R³ is independently a hydrogen atom, -R^{d}, -OR^{d}, a C₃₋₆ carbocyclic group, a nitrogen-containing heterocyclic group, -NR^{d}R^{d}, -SR^{d}, -C(=O)R^{d}, - C(=O)OR^{d}, -OC(=O)R^{d}, -OC(=O)OR^{d} or a functional group R⁰¹ that can react with bio-related substances at each occurrence; wherein, R^{d} is independently a C₁₋₁₂ alkyl group at each occurrence.

In a specific embodiment of the present application, R₃ is preferably independently selected from the group consisting of a hydrogen atom, an alkyl group, an alkoxy group, - C(=O)OR^{d}, -OC(=O)R^{d}, -OC(=O)OR^{d}, an epoxy group, an alcoholic hydroxyl group, a protected alcoholic hydroxyl group, a sulfhydryl group, a protected sulfhydryl group, a carboxyl group, a protected carboxyl group, an amino group, a protected amino group, an aldehyde group, a protected aldehyde group, an active ester group, a carbonate group, a urethane group, an isocyanate group, an isothiocyanate group, a succinimidyl group, a maleimide group, a protected maleimide group, a dimethylamino group, an alkenyl group, an enoate group, an azido group, a cyano group, a dithiopyridinyl group, an α-haloacetyl alkynyl group, an alkynyl group, a folate group, a rhodamine group, a biotin group, a monosaccharide group, a polysaccharide group, 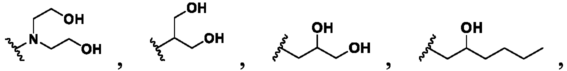 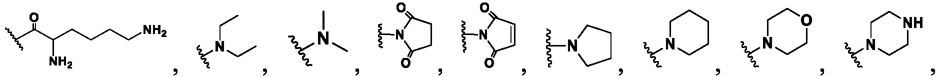 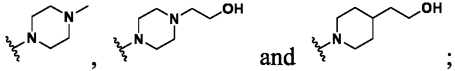 wherein, R^{d} is independently a C₁₋₁₂ alkyl group at each occurrence.

In a specific embodiment of the present application, R³ is, at each occurrence, independently a functional group R⁰¹ that can interact with a bio-related substance; the R⁰¹ is a functional group with a therapeutic targeting property; R⁰¹ is preferably a residue of folic acid, N-acetylgalactosamine or any functional derivative thereof, and further preferably selected from the group consisting of the following structures:

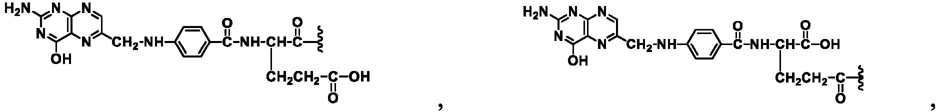

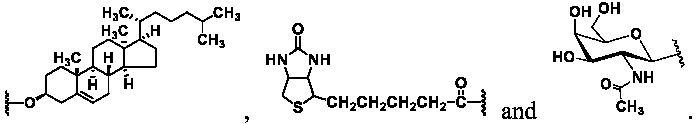

### 1.1.6. -L³-R³ fragment

In a specific embodiment of the present application, the -L⁵-R³, which is composed of the L⁵ described in section 1.1.2.4. and the R³ described in section 1.1.5, is independently selected from the group consisting of the following structures at each occurrence:

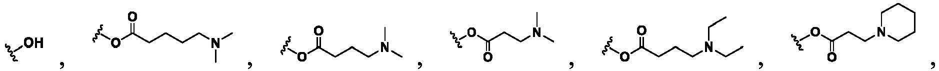

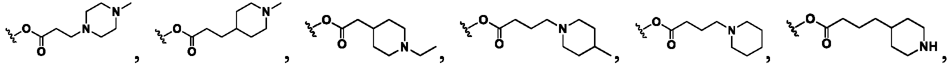

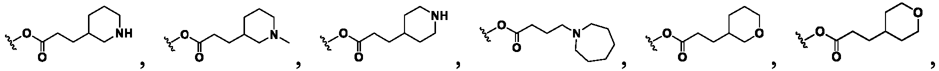

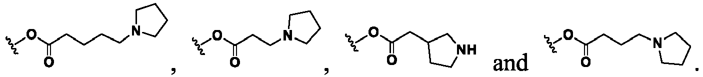

### 1.1.7. Examples of general formulas of structures

In a specific embodiment of the present application, according to the X described in 1.1.1, the structure of the cationic lipid of the present application is represented by the following general formula (2): 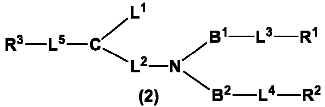 wherein, the definitions of L¹, L², L³, L⁴, L⁵, B¹, B², R¹, R², and R³ are consistent with those described in general formula (1) and will not be repeated here.

More preferably, the structure of the cationic lipid is selected from the group consisting of the following general formulas: 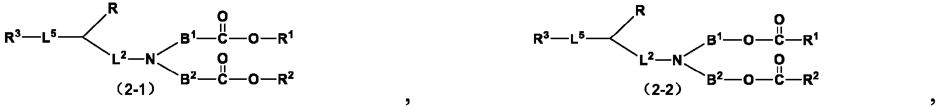 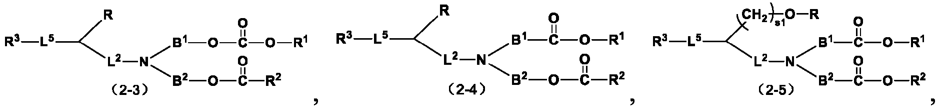 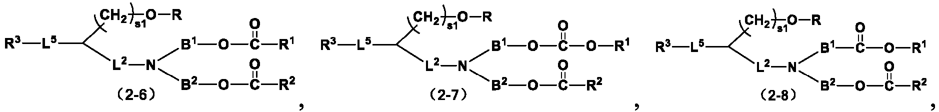 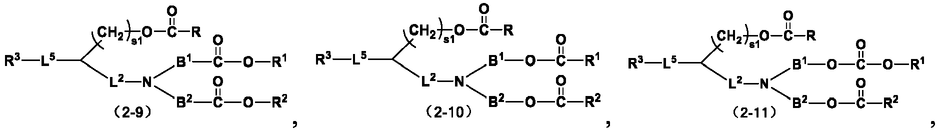 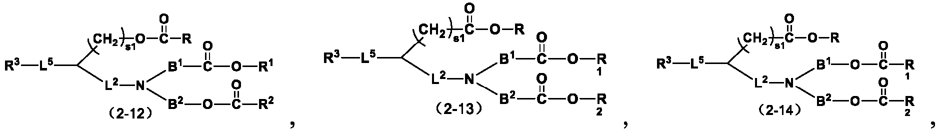 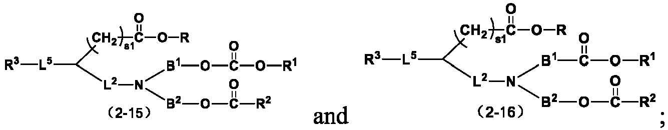 wherein, s1 is 0, 1, 2, 3, or 4; the definitions of L², L⁵, B¹, B², R, R¹, R² and R³ are consistent with those described in general formula (1) and will not be repeated here.

### 1.1.8. Examples of specific structures

In one embodiment of the present application, the structure of the cationic lipid is preferably selected from the group consisting of the following structures:

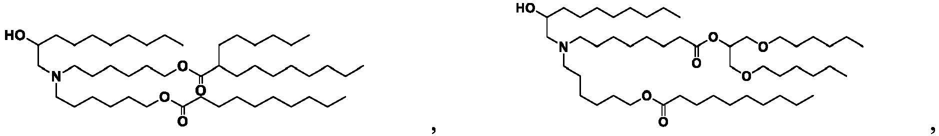

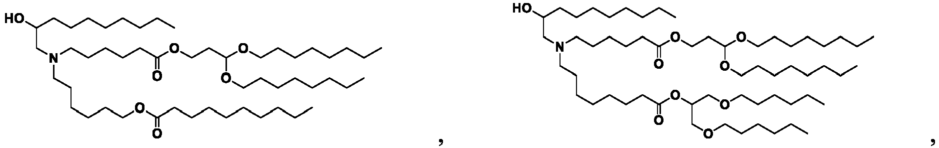

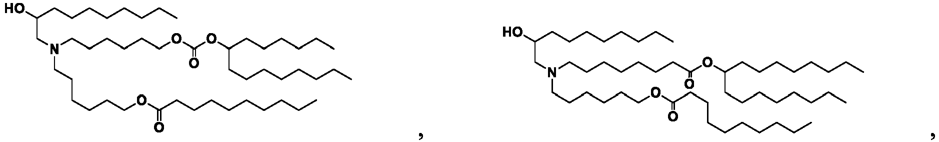

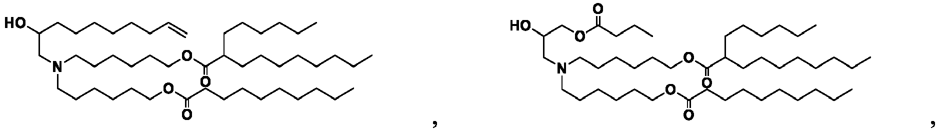

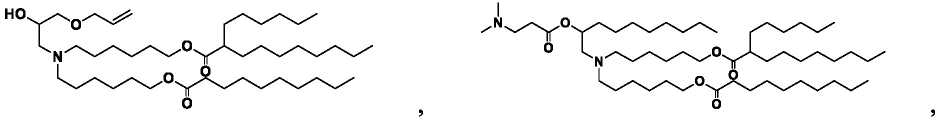

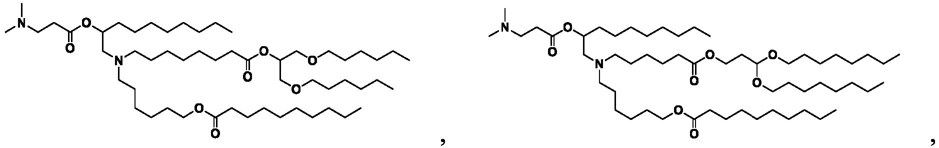

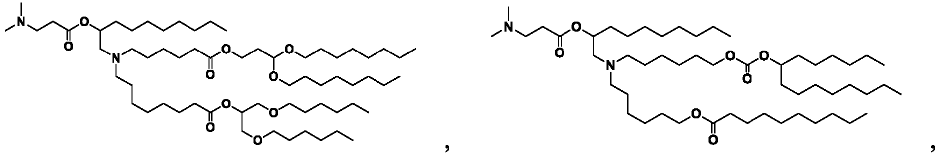

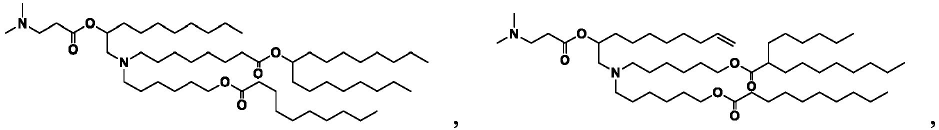

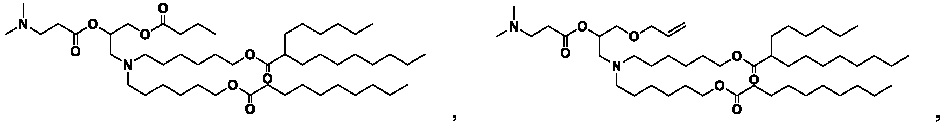

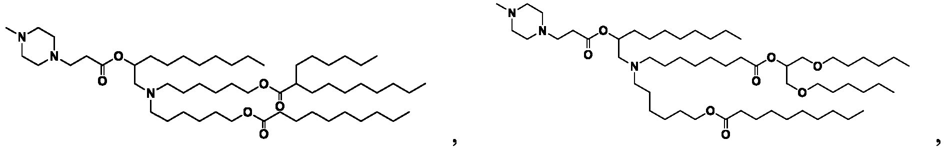

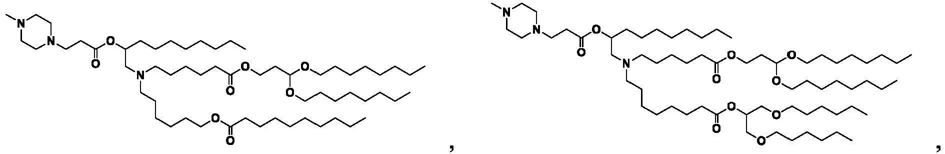

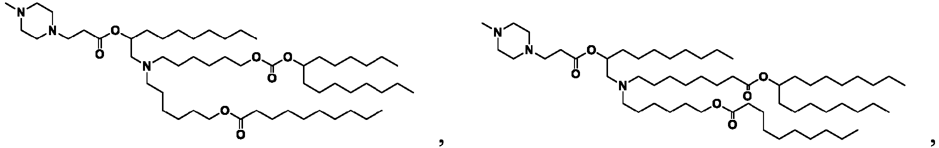

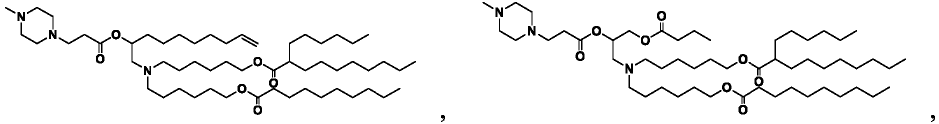

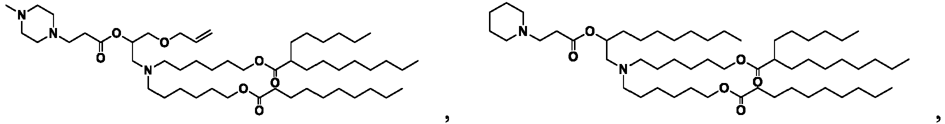

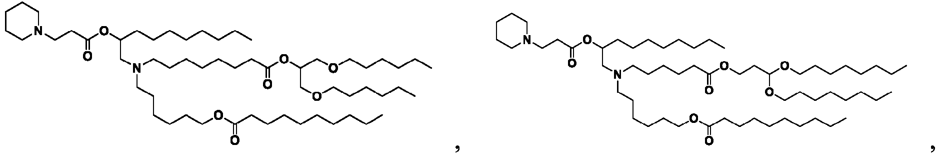

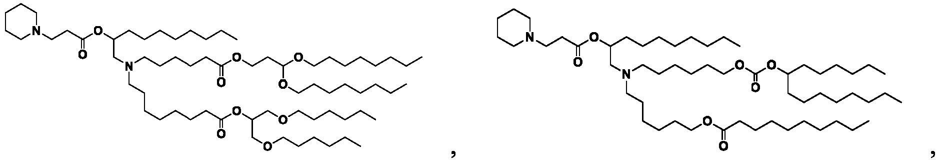

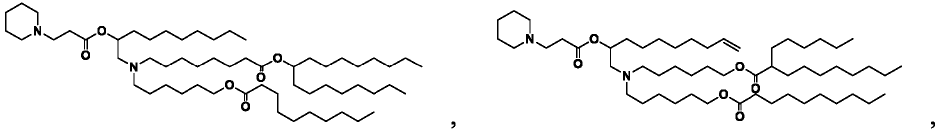

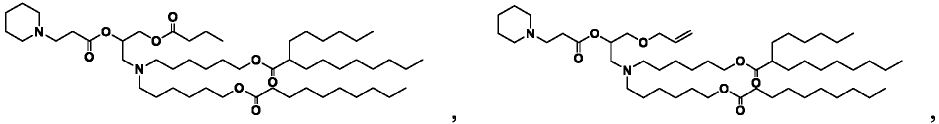

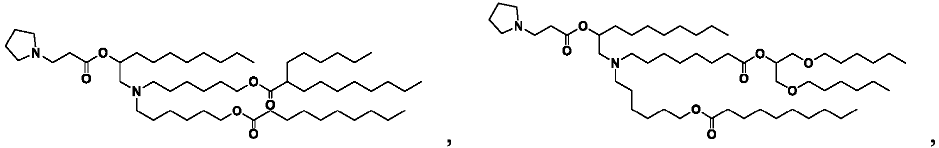

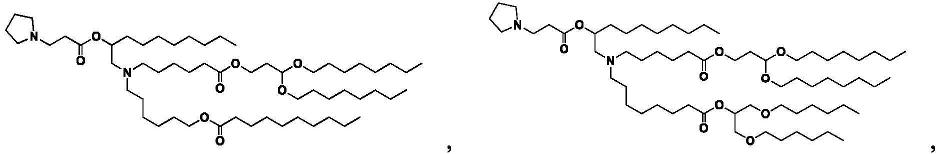

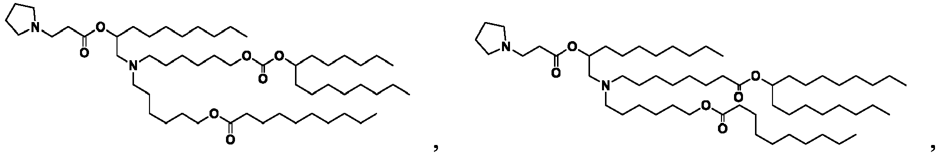

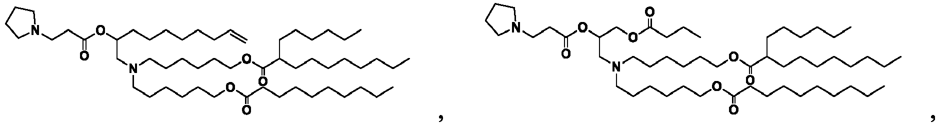

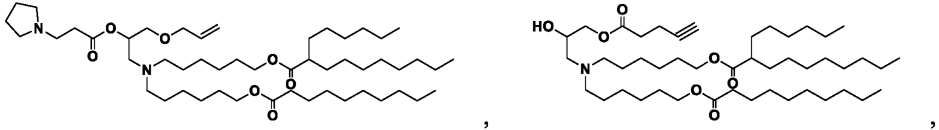

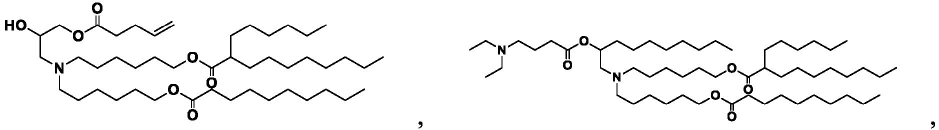

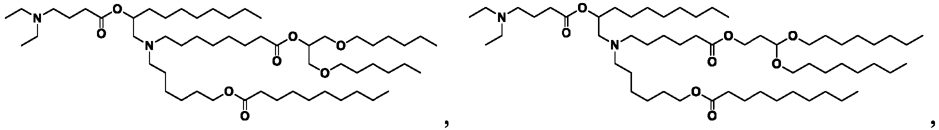

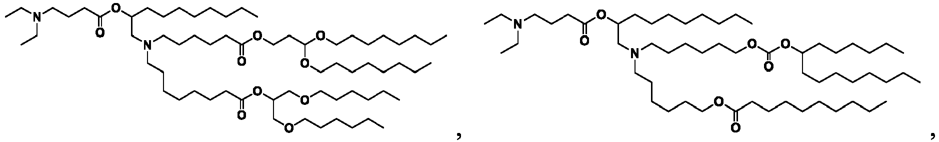

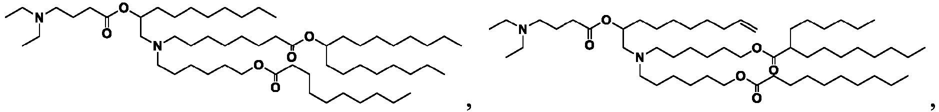

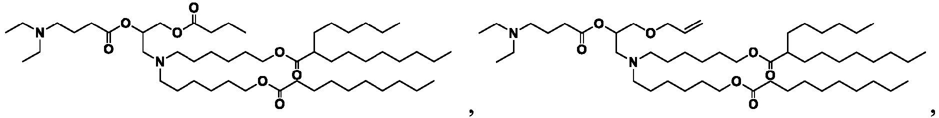

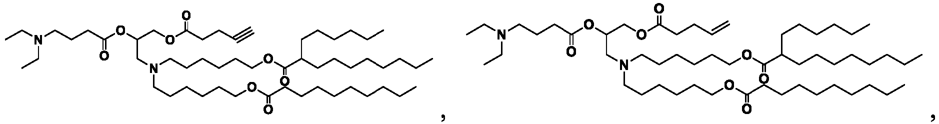

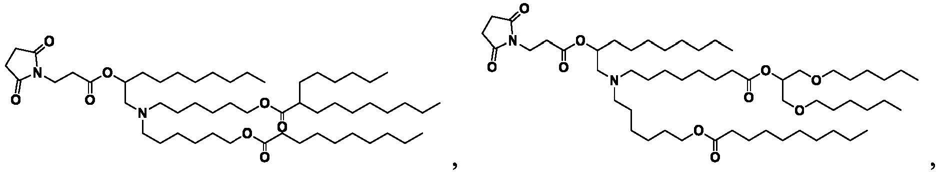

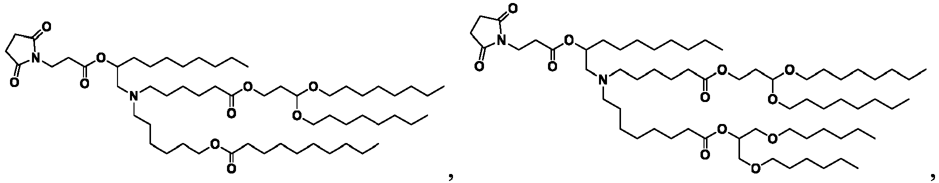

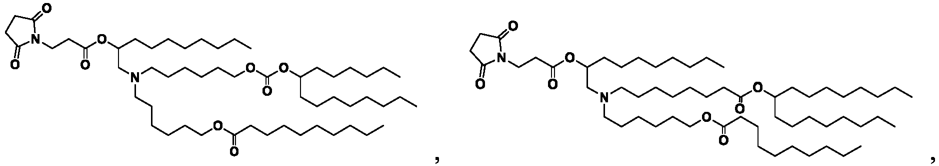

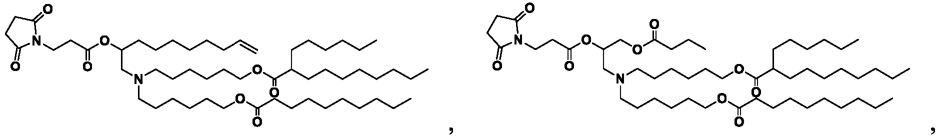

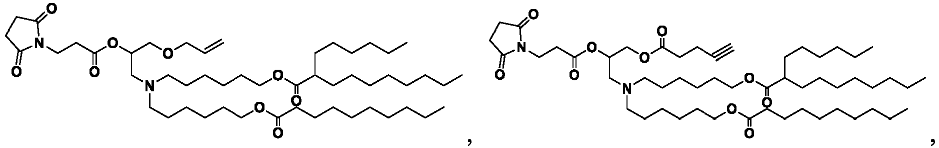

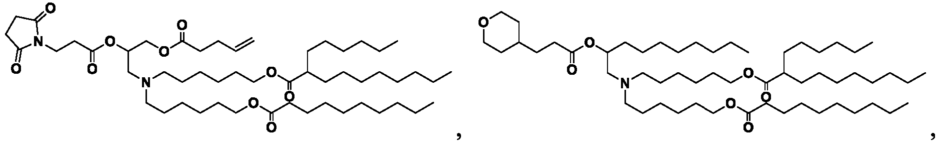

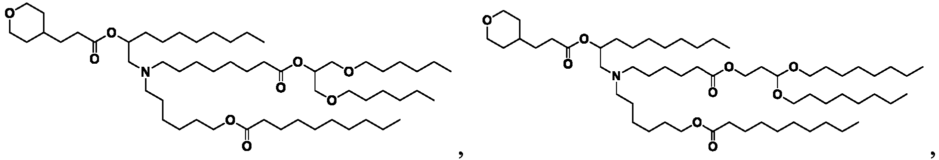

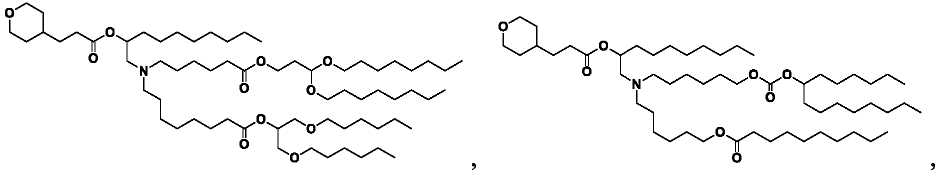

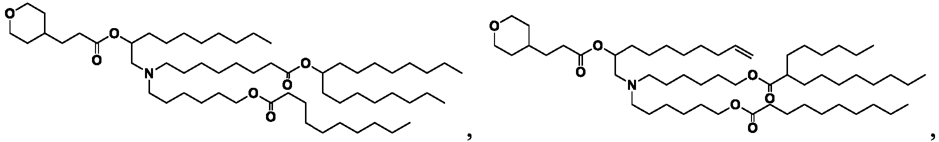

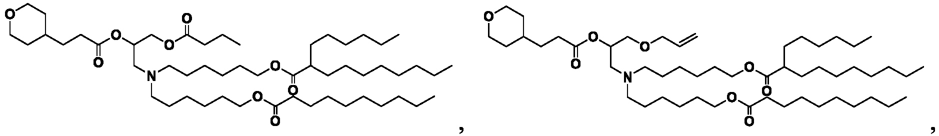

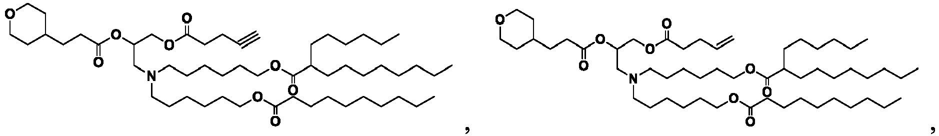

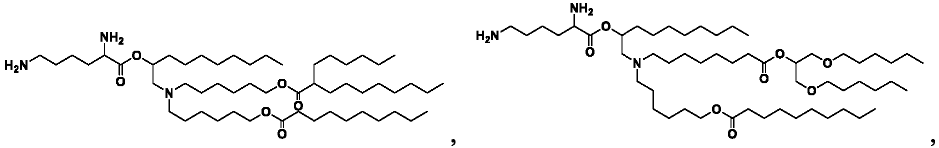

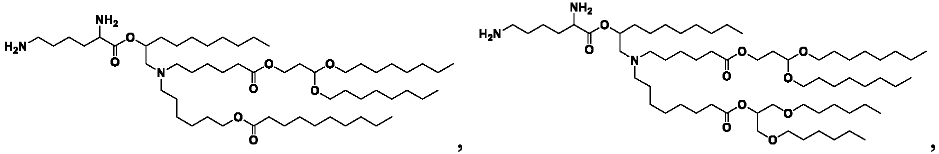

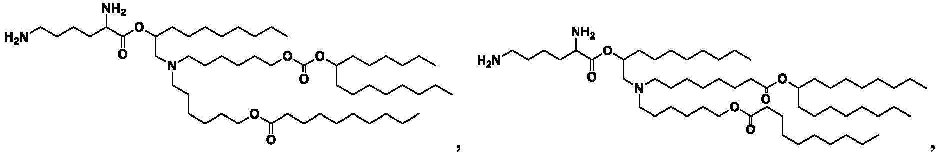

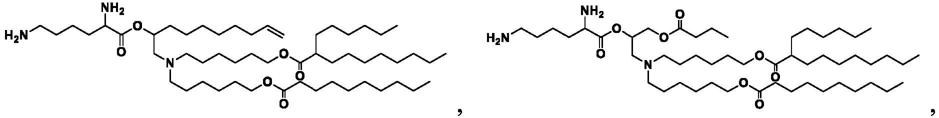

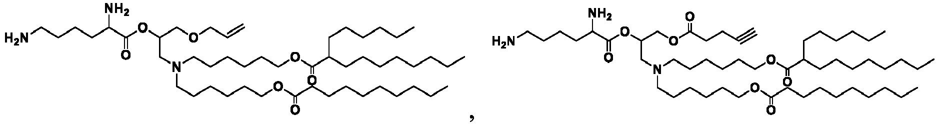

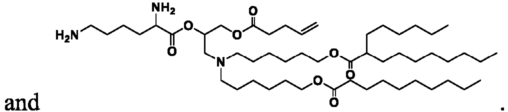

### 2. Preparation of cationic lipids

### 2.1. Preparation methods

In a specific embodiment of the present application, the cationic lipid represented by formula (2) is prepared by the following method.
Step 1: small-molecule starting materials including the primary amine derivative 1-3 and the 1-6 that can react with an amino group or a secondary amine group can be obtained by purchase or by synthesis. The synthesis may utilize the following route; wherein, F¹ and F² are reactive groups that can react to obtain the divalent linking group L³, and F³ and F⁴ are reactive groups that can react to obtain the divalent linking group L⁴; it is worth mentioning that the amino group in 1-1 can also be a protected amino group which can be transformed into the free amino group in 1-3 through a subsequent deprotection; F^{N} in 1-4 is a reactive group capable of reacting with an amino group, a secondary amine group, or protected form thereof, and F^{N} is preferably -OMs, -OTs, -CHO, -F, -Cl or -Br;
Step 2: the secondary amine derivative 1-8 is obtain from the following method: the secondary amine derivative 1-8 containing a polar hydroxyl group and L¹ is obtained from the ring-opening reaction between a primary amine derivative and the epoxide 1-7; wherein, tp is one, two, or three, and R^{a} is independently H or a C₁₋₁₂ alkyl group at each occurrence, that is, when tp is two or three, two or three R^{a} can be the same or different; the epoxy group 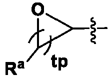 in the epoxide 1-7 is preferably selected from the group consisting of an ethylene oxide group, a propylene oxide group, a butylene oxide group, a substituted ethylene oxide group, a substituted propylene oxide and a substituted butylene oxide group;
Step 3: the compound represented by the formula (2') is obtained from the following method: the secondary amine derivative 1-8 is alkylated with the small molecule 1-6 to obtain the compound represented by the formula (2'); wherein, 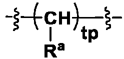 corresponds to L²;
Step 4: the end product represented by the formula (2) is obtained from the following method: when the -L⁵-R³ in formula (2) is a hydroxyl group, the compound represented by the formula (2') is also a compound of formula (2); when the -L⁵-R³ in formula (2) is not a hydroxyl group, the polar hydroxyl head group in formula (2') needs to be modified by terminal linear functionalization to obtain the compound of formula (2);
wherein, B¹, B², L¹, L², L³, L⁴, L⁵, R¹, R², and R³ are consistent with those described in the formula (2), and will not be repeated here.

### Step 1

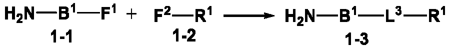

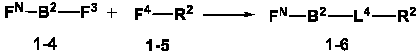

### Step 2

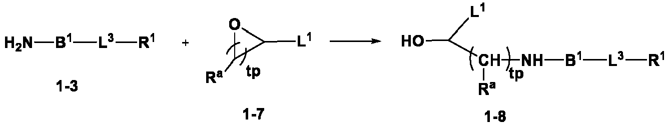

### Step 3

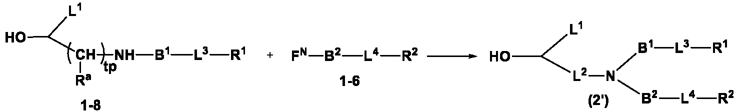

### Step 4

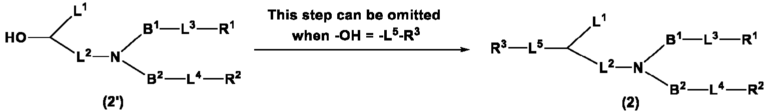

In a specific embodiment of the present application, the specific operating steps of the aforementioned preparation method are as follows:
Step 1: the small molecule A-1 ( 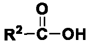 ) is reacted with the small molecule A-2 ( 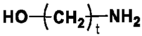 ) to obtain a primary amine derivative A-3 containing an ester bond, an amino group at one end, and R² at the other end; the small molecule A-1' (**R¹**-**OH**) is reacted with the small molecule A-2' ( 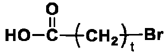 ) to obtain a small molecule intermediate A-3' ( 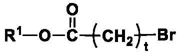 ) containing an ester bond, a bromo group at one end, and R¹ at the other end; wherein, t is an integer from 1 to 12;
Step 2: the primary amine derivative A-3 undergoes a ring-opening reaction with the epoxide A-4 ( 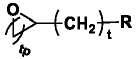 ) to obtain the secondary amine derivative A-5 ( 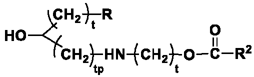 ) containing a divalent linking group and a free hydroxyl group, wherein tp is two, three, or four;
Step 3: one molecule of the small molecule intermediate A-3' undergoes an alkylation reaction with the secondary amine derivative A-5 to obtain the compound A-6 ( 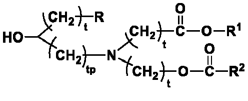 );
Step 4: the compound A-6 is coupled with the small molecule A-7 ( 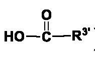 ) containing a reactive group to obtain the cationic lipid derivative A-8 or A-8'; wherein, the carboxyl group of A-7 can react with the hydroxyl group of the compound A-6 to obtain a carbon branching center and a divalent ester linking group;
When R³' is equal to R³, the resulting structure A-8' corresponds to the structure represented by the general formula (2);
When R³' is not equal to R³, A-8' is micromodified at the terminal group to obtain A-8, which corresponds to the structure represented by general formula (2); the terminal micro-modification is selected from the following chemical reactions: deprotection, salt complexation and decomplexation, ionization, protonation, deprotonation, and leaving group transformation;
When the -L⁵-R³ in formula (2) is a hydroxyl group, the compound A-6 obtained in Step 3 corresponds to the structure represented by the general formula (2);
wherein, the definitions of R¹, R², and R³ are consistent with those described for the general formula (2), and will not be repeated here.

### Step 1:

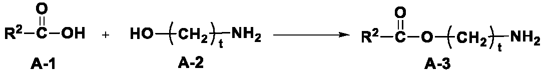

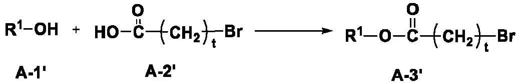

### Step 2:

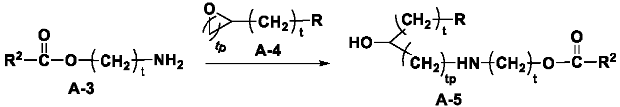

### Step 3:

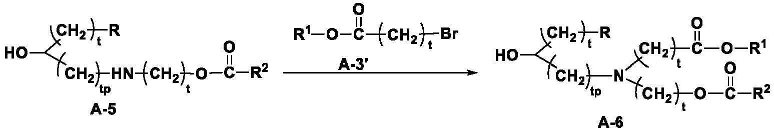

### Step 4:

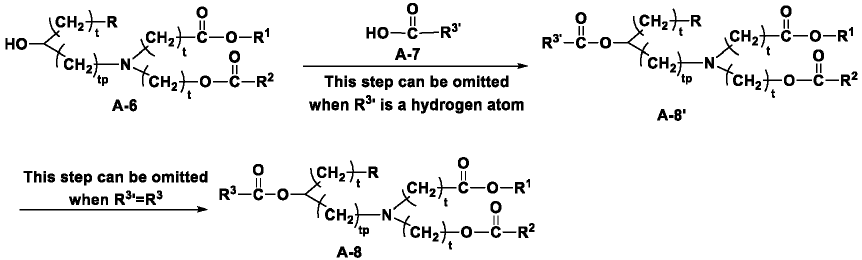

The aforementioned small molecule starting materials A-1, A-2, A-3, A-1', A-2', A-3', A-4, A-7, etc., can be obtained by purchase or synthesis. For example, the small molecule A-3' represented by 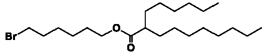 in Example 1, which can be obtained by the synthesis using 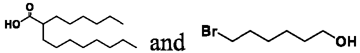 as starting materials.

The R² and R¹ in the starting materials A-3 and A-3' in the aforementioned preparation method can be each independently an etherified aliphatic hydrocarbon derivative residue represented by 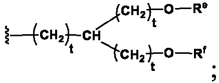 wherein, t is independently an integer from 0 to 12 at each occurrence; R^{e} and R^{f} are each independently a C₁₋₁₅ alkyl group, a C₂₋₁₅ alkenyl group or a C₂₋₁₅ alkynyl group

. More specifically, A-3 and A-3' can be represented by 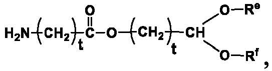 which can be obtained by purchase or synthesis, and the synthesis may utilize aldehyde alcohols, e.g., one molecule represented by 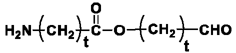 undergoes an addition reaction with two molecules of Re-OH to obtain a compound represented by 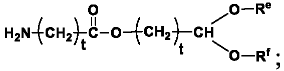 wherein, R^{e} and R^{f} are the same; A-3 and A-3' may also be represented by 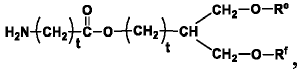 which can be obtained by purchase or synthesis, and in the case of synthesis can be obtained by reacting 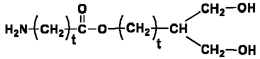 with the relevant alkylating reagent, which is preferably a halide, e.g., S2-4 ( 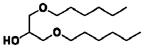 ) in Example 4 may be obtained by the reaction between one molecule of glycerol with a TBS protecting hydroxyl group and two molecules of bromohexane, followed by deprotection.

### 2.2. Description of relevant starting materials and/or steps in the preparation process

### 2.2.1. "Protection" and "deprotection" of relevant groups involved in the reaction process

In the present application, the reaction process also involves the "protection" and "deprotection" processes of relevant groups. To prevent a functional group from affecting the reaction, the functional group is usually protected. In addition, when there are two or more functional groups and only the target functional group needs to react, the other functional groups should therefore be protected. The protecting group not only protects the functional group stably but also needs to be removed easily as needed. Therefore, in organic synthesis, it is important to remove only the protecting group bonded to the specified functional group under appropriate conditions.

In the present application, the definitions of "carboxyl protection group" and "amino protection group" are consistent with those in the "Terminology" section, and will not be repeated herein.

In the present application, the hydroxyl groups protected by hydroxyl protecting groups are not particularly limited, e.g., alcoholic hydroxyl groups, phenolic hydroxyl groups, and the like. The amino groups protected by amino protecting groups are not particularly limited, such as those from primary amines, secondary amines, hydrazines, and amides. The amino groups in the present application are not particularly limited, including but not limited to primary amino groups, secondary amino groups, tertiary amino groups, and quaternary ammonium ions.

In the present application, the deprotection of protected hydroxyl groups is related to the types of hydroxyl protecting groups. The types of hydroxyl protecting groups are not particularly limited; for example, benzyl groups, silyl ethers, and tert-butyl groups can be used to protect terminal hydroxyl groups, and the corresponding deprotection methods include the following:

### A: deprotection of benzyl protecting groups

The deprotection of benzyl protecting groups can be achieved via hydrogenation using a hydrogenative reducing agent and a hydrogen donor. As used herein, the water content should be less than 1% to facilitate the reaction.

The hydrogenative reducing catalyst is not particularly limited, preferably palladium or nickel. The agent carrier is not particularly limited, preferably alumina or carbon, and more preferably carbon. The amount of palladium used is 1 to 100 wt% of that of compounds containing protected hydroxyl groups, preferably 1 to 20 wt%.

The reaction solvent is not particularly limited, as long as it allows the starting materials and the products to be dissolved. Preferable solvents include methanol, ethanol, ethyl acetate, tetrahydrofuran, and acetic acid, wherein methanol is more preferable. The hydrogen donor is not particularly limited, preferably hydrogen gas, cyclohexene, 2-propanol, ammonium formate, or the like. The reaction temperature is preferably 25 to 40 °C. The reaction time is not particularly limited, which is negatively correlated with the amount of catalyst used and preferably 1 to 5 h.

### B: deprotection of acetal and ketal protecting groups

The acetal or ketal compounds used to protect hydroxyl groups are preferably ethyl vinyl ether, tetrah ydropyran, acetone, 2,2-dimethoxypropane, benzaldehyde or the like. The deprotection of such acetal and ketone protecting group is achieved under acidic conditions, and the pH of the solution is preferably 0 to 4. There are no special restrictions on the acid, but acetic acid, phosphoric acid, sulfuric acid, hydrochloric acid and nitric acid are preferred, and hydrochloric acid is more preferred. There are no special restrictions on the reaction solvent, as long as the reactants and products can be dissolved, preferably by water. The reaction temperature is preferably 0 to 30 °C.

### C: Deprotection of silyl ether protecting group

Compounds used for this type of hydroxyl protection include trimethylsilyl ether, triethylsilyl ether, tert-butyldimethylsilyl ether, tert-butyldiphenylsilyl ether, and the like. The deprotection of such silyl ethers uses compounds containing fluoride ions, wherein the compound is preferably tetrabutylammonium fluoride, tetraethylammonium fluoride, hydrofluoric acid, or potassium fluoride, and more preferably tetrabutylammonium fluoride or potassium fluoride. The amount of the fluorine-containing compound is 5 to 20 folds of that of the protected hydroxyl group and preferably 8 to 15 folds of that of the initiator. When the amount of the fluorine-containing compound used is less than 5 molar equivalents per molar equivalent of protected hydroxyl groups, the deprotonation might not be complete. When the amount of deprotection reagent used exceeds 20 molar equivalents per molar equivalent of the initiator, the excess reagent or compound tends to cause difficulty in the purification process and may blend into the subsequent steps to result in side reactions. The reaction solvent is not particularly limited as long as it can dissolve the reagents and the products, preferably an aprotic solvent, and more preferably tetrahydrofuran or dichloromethane. The reaction temperature is preferably 0 to 30 °C; when it is lower than 0 °C, the reaction rate is relatively slow, and the protective group cannot be completely removed.

### D: Deprotection of t-butyl protecting groups

The deprotection of tert-butyl groups is carried out under an acidic condition, and the pH of the solution is preferably 0 to 4. The acid is not particularly limited but is preferably acetic acid, phosphoric acid, sulfuric acid, hydrochloric acid, or nitric acid, and more preferably hydrochloric acid. The reaction solvent is not particularly limited as long as it can dissolve the reagents and the products, preferably water. The reaction temperature is preferably 0 to 30 °C.

### 2.2.2. Alkylation reaction

In the present application, the alkylation reactions are preferably those based on hydroxyl groups, mercapto groups, or amino groups, corresponding to the formation of ether bonds, thioether bonds, and secondary or tertiary amino groups, respectively. Specific examples are as follows:

### 2.2.2.1. Alkylation reaction of substrate alcohols with sulfonates or halides

The ether intermediate can be obtained via the nucleophilic substitution of the substrate alcohol with a sulfonate derivative or a halide under a basic condition. Wherein, the amount of the sulfonate or halide is 1 to 50 and preferably 1 to 5 molar equivalents per molar equivalent of the substrate alcohol. When the amount of the sulfonate or halide is less than one molar equivalent per molar equivalent of the substrate alcohol, the substitution may not be complete, causing difficulty in the purification process. When the amount of the sulfonate or halide exceeds 50 molar equivalents per molar equivalent of the substrate alcohol, the excess sulfonate or halide tends to cause difficulty in the purification process and result in side reactions in the subsequent steps and therefore further increase the difficulty of purification.

The resulting product is a mixture of ether intermediate and excess sulfonate or halide and can be purified by purification methods such as anion exchange resin, permeation, ultrafiltration, and the like. Wherein, the anion exchange resin is not particularly limited as long as the target product can undergo ion exchange and adsorb on the resin, preferably the ion exchange resin of a tertiary amine or quaternary ammonia salt based on dextran, agarose, polyacrylate, polystyrene, poly(diphenylethylene), or the like. The solvents used for permeation and ultrafiltration are not limited, generally water or an organic solvent. The organic solvent is not particularly limited as long as the product can be dissolved therein, preferably dichloromethane, chloroform, or the like.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base can be an organic base such as triethylamine, pyridine, 4-dimethylaminopyridine, imidazole, and diisopropylethylamine, or an inorganic base such as sodium carbonate, sodium hydroxide, sodium bicarbonate, sodium acetate, potassium carbonate, and potassium hydroxide, preferably an organic base, and more preferably triethylamine or pyridine. The amount of the base is 1 to 50, preferably 1 to 10, and more preferably 3 to 5 molar equivalents per molar equivalent of the sulfonate or halide.

### 2.2.2.2. Alkylation reaction of substrate amines with sulfonates or halides

The amine intermediate can be obtained via the nucleophilic substitution of the substrate amine with a sulfonate derivative or halide under a basic condition. Wherein, the amount of the sulfonate or halide is 1 to 50 and preferably 1 to 5 molar equivalents per molar equivalent of the substrate amine. When the amount of the sulfonate or halide is less than one molar equivalent per molar equivalent of the substrate amine, the substitution may not be complete, causing difficulty in the purification process. When the amount of the sulfonate or halide exceeds 50 molar equivalents per molar equivalent of the substrate amine, the excess sulfonate or halide tends to cause difficulty in the purification process and result in side reactions in the subsequent steps and therefore further increase the difficulty of purification.

The resulting product is a mixture of amine intermediate and excess sulfonate or halide and can be purified by purification methods such as anion exchange resin, permeation, ultrafiltration, and the like. Wherein, the anion exchange resin is not particularly limited as long as the target product can undergo ion exchange and adsorb on the resin, preferably the ion exchange resin of a tertiary amine or quaternary ammonia salt based on dextran, agarose, polyacrylate, polystyrene, poly(diphenylethylene), or the like. The solvents used for permeation and ultrafiltration are not limited, generally water or an organic solvent. The organic solvent is not particularly limited as long as the product can be dissolved therein, preferably dichloromethane, chloroform, or the like.

The selections of "reaction solvent" and "base" are consistent with those in the section of "Alkylation reaction of substrate alcohols with sulfonates or halides", and will not be repeated here.

### 2.2.2.3. Alkylation reaction of substrate amines with aldehyde derivatives

The substrate amine reacts with an aldehyde derivative to obtain an imine intermediate, which is followed by obtaining an intermediate by reducing reagents. Wherein, the amount of the aldehyde derivative is 1 to 20, preferably 1 to 2, and more preferably 1 to 1.5 molar equivalents per molar equivalent of the substrate amine. When the amount of aldehyde exceeds 20 molar equivalents per molar equivalent of the substrate amine, the excess reagent tends to cause difficulty in the purification process and result in side reactions in the subsequent steps to increase the difficulty of purification. When the amount of aldehyde is less than one molar equivalent per molar equivalent of the substrate amine, the substitution may not be complete, causing the purification process to be difficult. Wherein, the resulting product can be obtained after purification by means such as cation exchange resin, permeation, ultrafiltration, and the like. The cation exchange resin is not particularly limited as long as it can undergo ion exchange with quaternary ammonium cations and realize the isolation. The solvents used for permeation and ultrafiltration are not limited, generally water or an organic solvent. The organic solvent is not particularly limited as long as the product can be dissolved therein, preferably dichloromethane, chloroform, or the like.

The reaction solvent is not limited, preferably an organic solvent such as methanol, ethanol, water, toluene, benzene, xylene, acetonitrile, ethyl acetate, tetrahydrofuran, chloroform, dichloromethane, dimethylsulfoxide, dimethylformamide, dimethylacetamide, and the like, and more preferably water or methanol.

The reducing reagent is not particularly limited as long as the imine can be reduced to an amine, preferably sodium borohydride, lithium aluminum hydride, sodium cyanoborohydride, Zn/AcOH, or the like, and more preferably sodium cyanoborohydride. The molar amount of the reduction reagent used is generally 0.5 to 50 folds and preferably 1 to 10 folds of that of aldehyde derivatives.

### 2.2.3. The linear end-functionalization

The method for linear end-functionalization is not particularly limited but related to the type of the final functional group or the protected form thereof. The method for linear functionalization of the terminal hydroxyl group is described herein, which is by functionally converting the terminal hydroxyl group of the compound represented by formula (2') or the compound A-6 into another functional group or the protected form thereof, i.e., -L⁵-R³. The specific preparation method is described in paragraphs [0960] to [1205] in the literature CN104530417A.

In the present application, the starting materials in every preparation method can be synthesized or purchased.

The intermediates and end-products prepared in the present application can be purified by the purification method including but not limited to extraction, recrystallization, adsorption treatment, precipitation, reverse precipitation, membrane dialysis, supercritical extraction, and the like. The characterization of the structure and the molecular weight of end-products can use methods including but not limited to NMR, electrophoresis, UV-visible spectrophotometer, FTIR, AFM, GPC, HPLC, MALDI-TOF, circular dichroism spectroscopy, mass spectrometry, and the like.

### 3.1. Lipid compositions

In the present application, provided herein is a lipid composition containing any cationic lipid wherein structure is represented by the general formula (1).

One specific embodiment of the present application, the lipid composition preferably contains, in addition to a cationic lipid with a structure represented by the general formula (1), one or more types of lipids selected from the group consisting of phospholipid, steroid lipid, and PEGylated lipid, and selected from any one of the following cases:
Case (1): also contains a phospholipid;
Case (2): also contains a steroid lipid;
Case (3): also contains a PEGylated lipid;
Case (4): also contains a phospholipid and a steroid lipid;
Case (5): also contains a phospholipid and a PEGylated lipid;
Case (6): also contains a steroid lipid and a PEGylated lipid;
Case (7): also contains a phospholipid, a steroid lipid, and a PEGylated lipid;
more preferably, the lipid composition also contains a neutral lipid, a steroid lipid, and a PEGylated lipid, simultaneously.

In a specific embodiment of the present application, the phospholipid in the lipid composition is preferably selected from the group consisting of 1,2-dilinoleoyl-sn-glycero-3-phosphocholine (DLPC), 1,2-dimyristoleoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphatidylcholine (DSPC), 1,2-diundecanoyl-sn-glycero-3-phosphatidylcholine (DUPC), 1-plamitoyl-2-oleoyl-sn-glycero-3-phosphocholine (POPC), 1,2-di-O-octadecenyl-sn-glycero-3-phosphatidylcholine (18:0 Diether PC), 1-oleoyl-2-cholesterylhemisuccinyl-sn-glycero-3-phosphocholine (OChemsPC), 1-O-hexadecyl-sn-glycero-3-phosphatidylcholine (C16 Lyso PC), 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholine, 1,2-dioleoyl-sn-glycero-3-phosphoethanolamine (DOPE), 1,2-diphytanyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt (DOPG), dioleoyl phosphatidylserine (DOPS), dipalmitoyl phosphatidylglycerol (DPPG), palmitoyloleoyl phosphatidylethanolamine (POPE), distearoyl phosphatidylethanolamine (DSPE), dipalmitoyl phosphatidylethanolamine (DPPE), dimyristoleoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine (LPE), and combinations thereof.

In a specific embodiment of the present application, the steroid lipid in the lipid composition is preferably selected from the group consisting of cholesterol, fecal sterol, sitosterol, ergosterol, campesterol, stigmasterol, rapeseed sterol, lycopene, ursolic acid, α-tocopherol, and combinations thereof.

In a specific embodiment of the present application, the PEGylated lipid in the lipid composition is preferably selected from the group consisting of 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-cholesterol, PEG-diacylglycamide (PEG-DAG), PEG-dialkyloxypropyl (PEG-DAA), PEG500-dipalmitoylphosphatidylcholine, PEG2000-dipalmitoylphosphatidylcholine, PEG500-stearylphosphatidylethanolamine PEG2000-distearylphosphatidylethanolamine, PEG500-1,2-oleoylphosphatidylethanolamine, PEG2000-1,2-oleoylphosphatidylethanolamine, PEG2000-2,3-distearoylglycerol (PEG-DMG), and combinations thereof.

In a specific embodiment of the present application, the PEGylated lipid in the lipid composition is preferably selected from the group consisting of the following structures and combinations thereof: 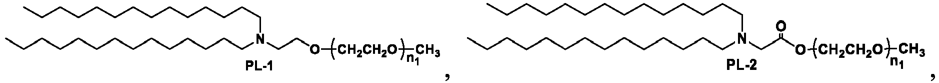 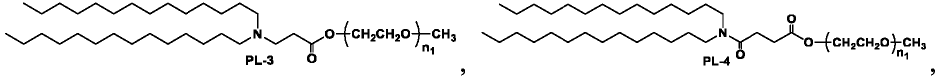 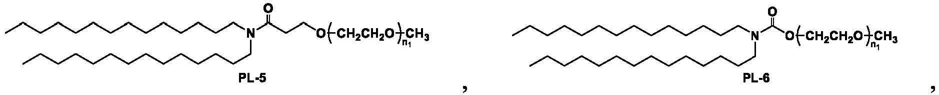 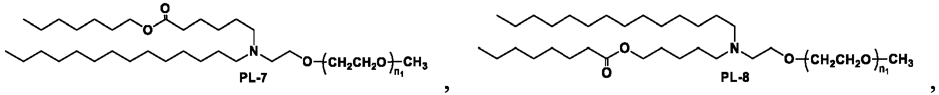 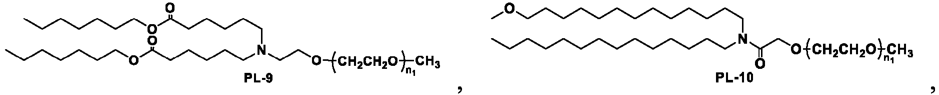 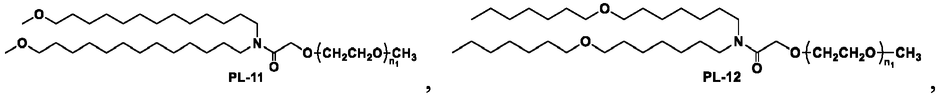 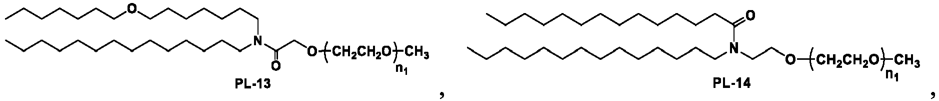 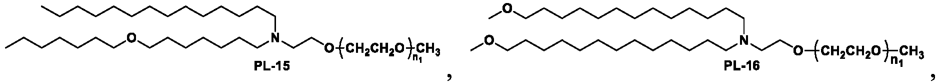 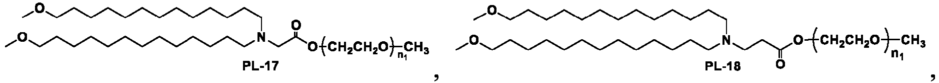 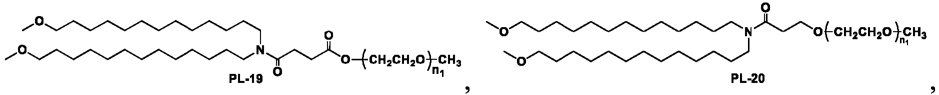 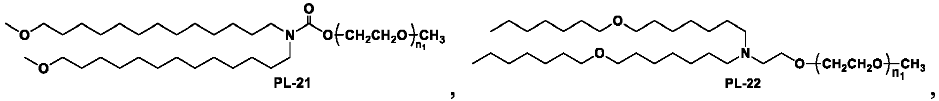 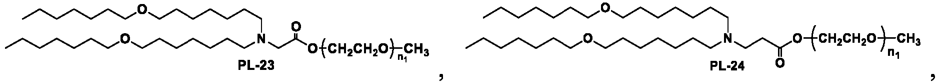 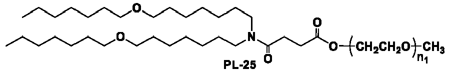 and 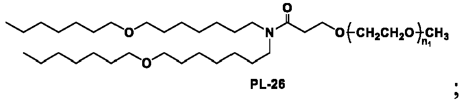 wherein, n₁ is an integer from 25 to 300 and more preferably n₁ is selected from the group consisting of 44, 45, 46, 47 and 48.

In one specific embodiment of the present application, any of the aforementioned lipid composition preferably contains 20-80% cationic lipid represented by the formula (1), 5-15% phospholipid, 25-55% steroid lipid, and 0.5-10% PEGylated lipid, and the percentage is the molar percentages of each lipid in the total lipids in the solution containing solvent.

In a specific embodiment of the present application, in any of the aforementioned lipid composition, the molar percentage of cationic lipid in the total lipids in the solution containing solvent is preferably 30-65%, more preferably about 35%, 40%, 45%, 46%, 47%, 48%, 49%, 50%, or 55%.

In a specific embodiment of the present application, in any of the aforementioned lipid composition, the molar percentage of phospholipid in the total lipids in the solution containing solvent is about 7.5-13%, more preferably about 8%, 9%, 10%, 11%, or 12%.

In a specific embodiment of the present application, in any of the aforementioned lipid composition, the molar percentage of steroid lipid in the total lipids in a solution with a solvent is 35-50%, more preferably about 40%, 41%, 42%, 43%, 44%, 45%, 46%, 47%, 48%, 49%, or 50%.

In a specific embodiment of the present application, in any of the aforementioned lipid composition, the molar percentage of PEGylated lipid in the total lipid in the solution containing solvent is 0.5-5%, preferably 1-3%, and more preferably about 1.5%, 1.6%, 1.7%, 1.8%, or 1.9%.

### 3.2. Preparation of lipid compositions

In the present application, the lipid composition can be prepared by the following methods, including but not limited to ethanol injection, microfluidic method, T-tube mixing method, periplasmic extrusion method, preferably ethanol injection and microfluidic method.

### 4. Lipid pharmaceutical compositions and formulations thereof

### 4.1. Lipid pharmaceutical compositions

In one embodiment of the present application, a lipid pharmaceutical composition contains any lipid composition and drug described in section 3.1 above; wherein, the lipid composition contains any of the aforementioned cationic lipid with the structure represented by formula (1), and the drug is selected from the group consisting of a nucleic acid drug, a gene vaccine, an antitumor drug, a small molecule drug, a peptide drug and a protein drug. In a specific embodiment of the present application, in a lipid pharmaceutical composition, the nucleic acid drug is selected from the group consisting of RNA, DNA, antisense nucleic acid, plasmid, interfering nucleic acid, aptamer, antagomir and ribozyme, and the RNA is selected from the group consisting of mRNA, saRNA, circRNA, miRNA and siRNA; preferably, the nucleic acid drug is selected from the group consisting of DNA, mRNA, miRNA and siRNA.

In a specific embodiment of the present application, the lipid pharmaceutical composition is preferably used as a drug, and is selected from the group consisting of the following drugs: an antineoplastic agent, an antiviral agent, an antifungal agent, and a vaccine.

In a specific embodiment of the present application, the N/P ratio of the lipid composition to the nucleic acid is preferably (0.1~100): 1, more preferably (0.2~30): 1, and most preferably (0.5~20): 1.

### 4.2. Formulations of Lipid pharmaceutical compositions

In a specific embodiment of the present application, the drug in the lipid pharmaceutical composition is the nucleic acid drug, and the working solution of the formulation is deionized water, ultrapure water, phosphate buffer or normal saline, and more preferably phosphate buffer or normal saline, and most preferably normal saline; the ratio of lipid composition: working solution is preferably (0.05~20) g: 100 mL, more preferably (0.1~10) g: 100 mL, most preferably (0.2~5) g: 100 mL.

In a specific embodiment of the application, a formulation of lipid pharmaceutical composition contains the aforementioned lipid pharmaceutical composition and a pharmaceutically acceptable diluent or excipient; the diluent or excipient is preferably deionized water, ultrapure water, phosphate buffer, or normal saline, more preferably phosphate buffer or normal saline, most preferably normal saline.

In the present application, the preparation of the formulation of lipid pharmaceutical composition includes the following steps:
(1) equilibrate the lipid composition in the diluent or excipient;
(2) add the nucleic acid drug to the equilibrated mixture of the lipid composition and the diluent or excipient, for complexing;
wherein, the equilibrium time is preferably 0.1~12 h, preferably 0.2~6 h, and more preferably 0.5~3 h; the time for complexing is preferably 0.1~12 h, preferably 0.2~5 h, and more preferably 0.5~2 h.

### 5. Liposomes or lipid nanoparticles and preparation thereof

### 5.1. Liposomes or lipid nanoparticles

In a specific embodiment of the present application, a liposome or lipid nanoparticle contains any lipid pharmaceutical composition described above.

In a specific embodiment of the present application, the aforementioned lipid nanoparticle is preferably an LNP pharmaceutical composition, an LPP pharmaceutical composition or a PNP pharmaceutical composition, preferably an LNP pharmaceutical composition, more preferably an LNP-nucleic acid pharmaceutical composition, more preferably an LNP-mRNA pharmaceutical composition.

### 5.2. Preparation of liposomes or lipid nanoparticles

In a specific embodiment of the present application, liposomes can be prepared by the following methods, including but not limited to thin-film dispersion method, ultrasonic dispersion method, reverse-phase evaporation method, freeze-drying method, freeze-thaw method, double emulsion method and injection method, preferably by thin-film dispersion method, ultrasonic dispersion method and/or reverse-phase evaporation method.

In a specific embodiment of the present application, lipid nanoparticles can be prepared by the following methods, including but not limited to microemulsion method, double emulsion method, high-shear ultrasonic homogenization method, thin film hydration extrusion method, microfluidic method.

In a specific embodiment of the present application, liposomes are prepared by the thin-film dispersion method that includes the following steps:
Step (1): weigh cationic lipid, steroid lipid, phospholipid, and PEGylated lipid, fully dissolve them in an organic solvent, shake well, remove the organic solvent by reducedpressure rotary evaporation to obtain an oil film, and further dry with a vacuum pump to remove the organic solvent;
Step (2): add phosphate buffer with dissolved cryoprotectant, and use water-bath ultrasonication to obtain a translucent emulsion;
Step (3): add the emulsion to a high-pressure homogenizer for overpressure, and then add the over pressurized homogenized emulsion to the liposome extruder for film-passing to obtain the liposome;
Step (4): optionally, dry the liposome in a freeze dryer to obtain a liposome powder;
wherein, the organic solvent is preferably dichloromethane, chloroform, and/or methanol, more preferably chloroform or methanol; wherein, the rotational speed of vacuum rotary evaporation is preferably 30 to 300 rpm, more preferably 50 to 200 rpm, and most preferably 100 to 170 rpm; and wherein, the temperature of vacuum rotary evaporation is preferably 10 to 200 °C, more preferably 20 to 200 °C, and most preferably 40 to 80 °C;
the time of the drying with a vacuum pump is preferably 1 to 72 h, more preferably 5 to 48 h, and most preferably 15 to 36 h;
the mass concentration of the cryoprotectant dissolved in phosphate buffer is 0.1 to 80%, preferably 1 to 50%, and more preferably 5 to 20%;
the frequency of the water-bath ultrasonication is preferably 10 to 300 kHz, more preferably 30 to 200 kHz, and most preferably 60 to 150 kHz;
the time of the water-bath ultrasonication is preferably 0.1 to 5 h, more preferably 0.2 to 2 h, and most preferably 0.25 to 1 h;
the pressure of the high-pressure homogenizer is preferably 50 to 240 MPa, more preferably 80 to 200 MPa, and most preferably 100 to 150 MPa;
the times of the overpressure of the high-pressure homogenizer is preferably any integer from 1 to 50, more preferably any integer from 3 to 20, and most preferably any integer from 5 to 10;
the pressure of the liposome extruder is preferably 50 to 300 MPa, more preferably 80 to 250 MPa, and most preferably 120 to 200 MPa;
the times of the film-passing of liposome extruder is preferably any integer from 1 to 50, more preferably any integer from 3 to 30, and most preferably any integer from 5 to 20;
the time of the drying in a freeze dryer is preferably 1 to 120 h, more preferably 5 to 72 h, and most preferably 10 to 36 h.

In the preparation methods for cationic liposomes in the present application, the ratio of cationic liposome to phosphate buffer with dissolved cryoprotectant could be 1 mg : (0.1~100) mL, preferably 1 mg : (0.3~50) mL, and more preferably 1 mg : (0.5~5) mL.

In a specific embodiment of the present application, lipid nanoparticles are preferably prepared by the microfluidic method, and the steps are as follows:
Step (1): dissolve every lipid component in the organic solvent to obtain a lipid composition dissolved in the organic phase; the organic phase is preferably ethanol;
Step (2): add the nucleic acid drug to the buffer to obtain an aqueous solution; the aqueous phase is preferably a citrate buffer or sodium acetate buffer;
Step (3): the organic phase solution and aqueous phase solution are mixed by a microfluidic device to form the lipid nanoparticle composition, followed by purification such as ultrafiltration and the like to remove the organic solvent and free nucleic acid molecules.

The following specific examples are further descriptions of the preparation methods of cationic lipids, lipid compositions, and formulations of lipid pharmaceutical compositions, and the biological activity assays for lipid pharmaceutical compositions; the specific examples are disclosed to further illustrate the application, but should not be regarded as a limitation of the scope of the present application. Wherein, in the embodiments of preparing cationic lipids, the structures of end products are characterized by NMR, and the molecular weight is confirmed by mass spectrometry.

### Example 1: Cationic lipid (E1-1)

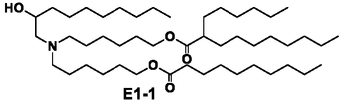

The preparation process is as follows:
Step a: under nitrogen protection, dicyclohexylcarbodiimide (**DCC**, 3.6 g, 17.6 mmol) was added to a round-bottom flask containing 6-amino-1-hexanol with a Boc protected amino group (**S1-1,** 2.08 g, 9.6 mmol), caprylic acid (**S1-2,** 1.15 g, 8.0 mmol) and 4-(dimethylamino)pyridine (**DMAP,** 0.24 g, 2.0 mmol) dissolved in dichloromethane (30 mL) and reacted for 16 h at room temperature. After the end of the reaction, the precipitate was removed by filtration, the filtrate was concentrated, and the crude product obtained was purified by column chromatography to obtain the ester **S1-3** (2.55 g) with a Boc protected amino group.
Step b: removing the Boc protecting group. A solution of trifluoroacetic acid/dichloromethane (1:2, v/v) was prepared in a dry and clean round-bottom flask, and the dichloromethane solution of **S1-3** (1.86 g, 5.0 mmol) was slowly added dropwise under ice bath conditions and reacted for 2 h at room temperature. After the end of the reaction, water was added to the reaction solution and stirred evenly, and then extracted. The organic phase was dried with anhydrous magnesium sulfate, filtered, and then the filtrate was concentrated and recrystallized to obtain the ester **S1-4** (1.24 g, 91.4%) containing a free amino group.
Step c: the above compound **S1-4** (0.81 g, 3.0 mmol) and 1,2-epoxydecane (**S1-5,** 0.43 g, 3.0 mmol) were dissolved in 15 mL of acetonitrile, followed by adding calcium trifluoromethanesulfonate (**Ca(OTf)₂,** 0.51 g, 1.5 mmol). The reaction mixture was stirred at room temperature until TLC showed that the reaction was complete. Then, the acetonitrile was removed by rotary evaporation. Afterward, water (5 mL) was added, mixed thoroughly, and the mixture was extracted with dichloromethane (5 mL*3). The organic layers were combined, dried with anhydrous Na₂SO₄, filtered, and concentrated. The crude product was purified by column chromatography to obtain the ring-opening product **S1-6** (1.02 g) containing a hydroxyl group.
Step d: under the protection of nitrogen, the above compound **S1-6** (0.86 g, 2.0 mmol) was dissolved in acetonitrile (15 mL), and **S1-7** (1.05 g, 2.5 mmol, **S1-7** was obtained by the reaction between 2-hexyldecanoic acid represented by 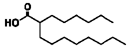 and 6-bromohexsanol represented by 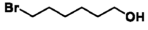 ) and N,N-diisopropylethanamine (**DIPEA,** 0.18 g, 2.0 mmol) were added to the solution sequentially under slow stirring. The reaction was conducted for about 20 h at room temperature. After the end of the reaction, the reaction solution was concentrated and dissolved with dichloromethane, then extracted with 0.6 M hydrochloric acid/10% sodium chloride solution and saturated sodium bicarbonate solution, successively.

The organic phases were combined, and dried with anhydrous magnesium sulfate, filtered, concentrated and then purified by column chromatography to obtain the cationic lipid **E1-1** (1.29 g). ¹H NMR (400 MHz, CDCl₃) δ: 4.03 (t, 4H), 3.57-3.54 (m, 1H), 3.10-2.83 (m, 6H), 2.31-2.24 (m, 3H), 1.86-1.75 (m, 4H), 1.65-1.20 (m, 64H), 0.88 (t, 12H). MS (ESI): m/z=766.72 ([M+H]⁺).

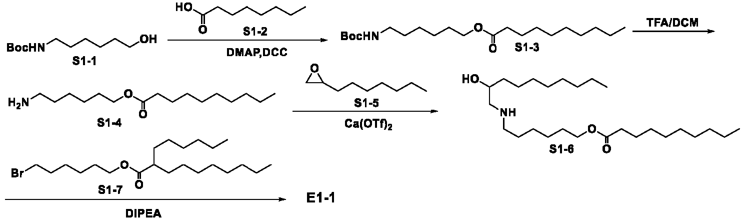

### Example 2: Cationic lipid (E2-1)

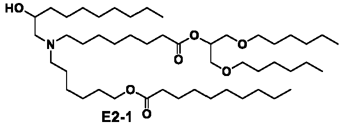

The preparation process is as follows:
Step a: under nitrogen protection, the glycerol with a TBS protected hydroxyl group (S2-1, 2.06 g, 10.0 mmol), potassium carbonate (**K₂CO₃,** 4.14 g, 30.0 mmol), and bromohexane (**S2-2,** 1.80 g, 11.0 mmol) were dissolved in 30 mL of DMF. The mixture was stirred at 110 °C for 16 h until TLC showed that the reaction was complete, then the reaction solution was added into water (30 mL) for precipitation, and the solution was filtered, concentrated, and then purified by column chromatography to obtain the glycerol ether **S2-3** (3.00 g, 88.7%) with a TBS protected hydroxyl group.
Step b: the above product **S2-3** (1.88 g, 8.0 mmol) was dissolved in THF (20 mL), then **TBAF** (20 mL, 1 M) was added to the solution under nitrogen protection and reacted overnight to remove TBS protection. After the completion of the reaction, the reaction solution was concentrated to obtain the crude product of compound **S2-4.** The crude product was purified by column chromatography, concentrated, and dried with an oil pump to obtain glycerol ether **S2-4** (1.84 g, 88.4%) containing a hydroxyl group.
Step c: under nitrogen protection, **DCC** (1.36 g, 6.6 mmol) was added to a round-bottom flask containing **S2-4** (0.94 g, 3.6 mmol), 8-bromooctanoic acid (**S2-5,** 0.67 g, 3.0 mmol) and **DMAP** (91.50 mg, 0.8 mmol) dissolved in dichloromethane (20 mL), and the solution was reacted for 16 h at room temperature. After the end of the reaction, the precipitate was removed by filtration, the filtrate was concentrated, and the crude product obtained was purified by column chromatography to obtain the bromoester **S2-6** (1.15 g).
Step d: under nitrogen protection, compound **S1-6** (0.64 g, 1.5 mmol) was dissolved in acetonitrile (15 mL), and then **S2-6** (0.87 g, 1.9 mmol) and **DIPEA** (0.14 g, 1.5 mmol) were added to the solution sequentially under slow stirring and reacted for about 20 h at room temperature. After the end of the reaction, the reaction solution was concentrated and then dissolved with dichloromethane, extracted with 0.6 M hydrochloric acid/10% sodium chloride solution and saturated sodium bicarbonate solution, successively. The organic phases were combined, dried with anhydrous magnesium sulfate, filtered, and the filtrate was concentrated, and purified by column chromatography to obtain cationic lipid **E2-1** (1.03 g). ¹H NMR (400 MHz, CDCl₃) δ: 5.13-5.06 (m, 1H), 4.05-3.99 (t, 2H), 3.57-3.49 (m, 5H), 3.46-3.36 (m, 4H), 2.53-2.43 (m, 2H), 2.38-2.17 (m, 8H), 1.60-1.20 (m, 62H), 0.88 (t, 12H). MS (ESI): m/z=812.73 ([M+H]⁺).

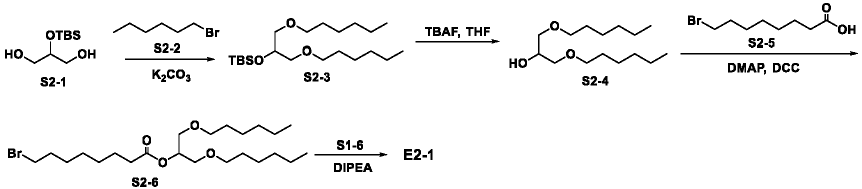

### Example-3: Cationic lipid (E3-1)

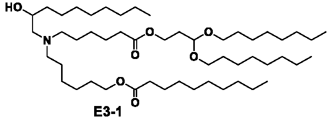

The preparation process is as follows:
Step a: 1,3-propanediol (**S3-1,** 9.50 g, 50 mmol) with a TBS protected hydroxyl group was dissolved in 200 mL of dichloromethane solution, followed by the addition of pyridinium chlorochromate (**PCC,** 16.13 g, 75.0 mmol). After stirring at 15 °C for at least 2 h, the mixture was filtered, and filtrate was concentrated under reduced pressure, and then purified by column chromatography to obtain 3-hydroxypropionic acid **S3-2** (6.14 g) with a TBS protected hydroxyl group.
Step b: the above compound **S3-2** (5.64 g, 30.0 mmol) and 1-octanol (**S3-3,** 9.75 g, 75.0 mmol) were dissolved in 150 mL of dichloromethane solution, and then p-toluenesulfonic acid monohydrate (**TsOH·H₂O,** 1.14 g, 6.0 mmol) and anhydrous sodium sulfate (10.65 g, 75.0 mmol) were added to the solution. After stirring at 15 °C for at least 24 h, the mixed solution was filtered, and filtrate was concentrated under reduced pressure, and the crude product was purified by column chromatography to obtain the acetal **S3-4** (3.00 g) with a TBS protected hydroxyl group.
Step c: the above product **S3-4** (2.16 g, 5.0 mmol) was dissolved in THF (30 mL), and **TBAF** (30 mL, 1 M) was added to the solution under nitrogen protection, and the reaction was conducted overnight to remove TBS protection. After the reaction was completed, the reaction solution was concentrated to obtain a crude product of compound **S3-5.** The crude product was purified by column chromatography, concentrated, and dried with an oil pump to obtain the acetal **S3-5** (1.38 g, 87.1%) with a free hydroxyl group.
Step d: under nitrogen protection, **DCC** (0.91 g, 4.4 mmol) was added to a round-bottomed flask containing **S3-5** (0.76 g, 2.4 mmol), **S3-6** (0.39 g, 2.0 mmol) and **DMAP** (61.00 mg, 0.5 mmol) dissolved in dichloromethane (15 mL), and the reaction was conducted at room temperature for 16 h. After the end of reaction, the precipitate was removed by filtration, the filtrate was concentrated, and the crude product was purified by silica gel column chromatography to obtain the bromoester **S3-7** (0.81 g).
Step e: under nitrogen protection, compound **S1-6** (0.43 g, 1.0 mmol) was dissolved in acetonitrile (10 mL), and **S3-7** (0.62 g, 1.3 mmol) and **DIPEA** (0.09 g, 1.0 mmol)) were added to the solution in sequence under slow stirring. The reaction was stirred at room temperature for about 20 h. After the reaction was completed, the reaction solution was concentrated, dissolved in dichloromethane, and extracted with 0.6 M hydrochloric acid/10% sodium chloride solution and saturated sodium bicarbonate solution in sequence. Then, the organic phases were combined, dried with anhydrous magnesium sulfate, filtered, and the filtrate was concentrated, and then purified by column chromatography to obtain the cationic lipid **E3-1** (0.71 g). ¹H NMR (400 MHz, CDCl₃) δ: 4.65 (t, 1H), 4.07 (t, 4H), 3.57-3.54 (m, 1H), 3.52-3.36 (m, 4H), 2.53-2.43 (m, 2H), 2.38-2.19 (m, 8H), 1.71-1.22 (m, 68H), 0.87 (t, 12H). MS (ESI): m/z=840.76 ([M+H]⁺).

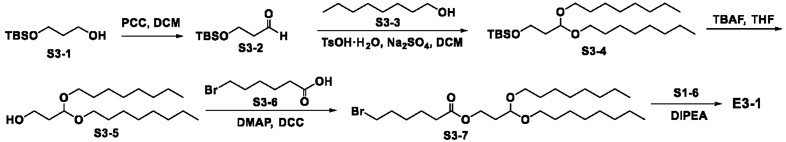

### Example-4: Cationic lipid (E4-1)

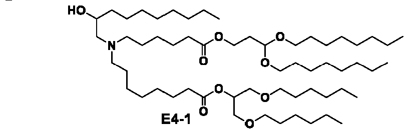

The preparation process is as follows:
Step a: under nitrogen protection, **DCC** (3.63 g, 17.6 mmol) was added to a round-bottomed flask containing **S2-4** (2.50 g, 9.6 mmol), 8-aminooctanoic acid with a Boc protected amino group (**S4-1,** 2.07 g, 8.0 mmol) and **DMAP** (0.24 g, 2.0 mmol) dissolved in dichloromethane (30 mL), and the reaction was conducted at room temperature for 16 h. After the end of the reaction, the precipitate was removed by filtration, the filtrate was concentrated, and the crude product was purified by column chromatography to obtain the ester **S4-2** (3.45 g) with a Boc protected amino group.
Step b: removing the Boc protecting group. In a dry and clean round-bottomed flask, a solution of trifluoroacetic acid/dichloromethane (1:2, v/v) was prepared, and the dichloromethane solution of **S4-2** (2.51 g, 5.0 mmol)was slowly added dropwise under an ice bath. The reaction was conducted at room temperature for 2 h. At the end of the reaction, water was added to the reaction solution and stirred well, and then the mixture was extracted. The organic phase was dried with anhydrous magnesium sulfate, filtered, and the filtrate was concentrated and recrystallized to obtain the ester **S4-3** (1.84 g, 91.6%) with a free amino group.
Step c: the above compounds **S4-3** (1.21 g, 3.0 mmol) and **S1-5** (0.43 g, 3.0 mmol) were dissolved in 20 mL acetonitrile, then **Ca(OTf)₂** (0.51 g, 1.5 mmol) was added to the solution, and the reaction mixture was stirred at room temperature until TLC showed that the reaction was complete. Then, acetonitrile was removed by rotary evaporation. Water (10 mL) was added and mixed evenly, and then the mixture was extracted with dichloromethane (10 mL*3). The organic phases were combined, dried with anhydrous Na₂SO4, filtered and the filtrate was concentrated. The crude product was purified by column chromatography to obtain the ring-opened product **S4-4** (1.33 g) with a hydroxyl group.
Step d: the above compound **S4-4** (1.12 g, 2.0 mmol) was dissolved in acetonitrile (20 mL) under nitrogen protection, and **S3-7** (1.24 g, 2.5 mmol) and **DIPEA** (0.18 g, 2.0 mmol)) were added successively under slow stirring. The reaction was stirred at room temperature for about 20 h. After the reaction was completed, the reaction solution was concentrated, dissolved in dichloromethane, and then extracted with 0.6 M hydrochloric acid/10% sodium chloride solution and saturated sodium bicarbonate solution in sequence. The organic phases were combined, dried with anhydrous magnesium sulfate, filtered, and the filtrate was concentrated, and purified by column chromatography to obtain the cationic lipid **E4-1** (1.63 g). ¹H NMR (400 MHz, CDCl³) δ: 5.13-5.06 (m, 1H), 4.64 (t, 1H), 4.07 (t, 2H), 3.57-3.36 (m, 13H), 2.52-2.43 (m, 2H), 2.39-2.17 (m, 8H), 1.71-1.22 (m, 72H), 0.89 (t, 15H). MS (ESI): m/z=970.86 ([M+H]+).

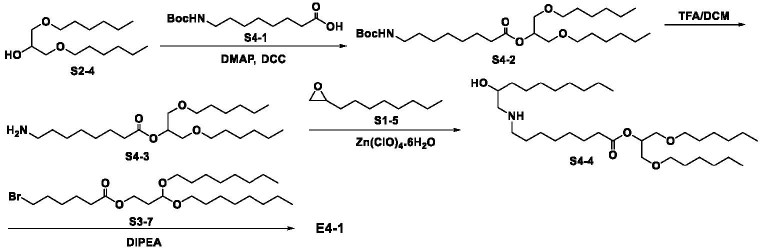

### Example-5: Cationic lipid (E5-1)

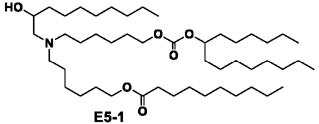

The preparation process is as follows:
Step a: under the protection of nitrogen, 6-bromohexyl-4-nitrophenyl carbonate (**S5-1,** 3.46 g, 10.0 mmol, **S5-1** was obtained by the reaction between p-nitrophenyl chloroformate and 6-bromo-n-hexanol) was dissolved in dichloromethane (100 mL), and pentadecane-7-ol (**S5-2,** 9.12 g, 40.0 mmol) was added dropwise with stirring at room temperature, then **pyridine** (1.00 mL, 12.5 mmol) was slowly added dropwise over 10 min, and then **DMAP** (0.24 g, 2.0 mmol) was added in one portion. The reaction was stirred at room temperature for 16 h and then extracted twice with dichloromethane. The organic phases were combined and washed with brine, then dried with anhydrous magnesium sulfate, filtered and the filtrate was concentrated to obtain a crude product. The crude product was separated and purified by column chromatography to obtain the bromocarbonate (**S5-3,** 1.13 g).
Step b: the above compound **S1-6** (0.64 g, 1.5 mmol) was dissolved in acetonitrile (10 mL) under nitrogen protection, and **S5-3** (0.82 g, 1.9 mmol) and **DIPEA** (0.14 g, 1.5 mmol) were added successively under slow stirring. The reaction was stirred at room temperature for about 20 h. After the reaction was completed, the reaction solution was concentrated, then dissolved in dichloromethane, and extracted with 0.6 M hydrochloric acid/10% sodium chloride solution and saturated sodium bicarbonate solution in sequence. The organic phases were combined, dried with anhydrous magnesium sulfate, filtered, and the filtrate was concentrated, and purified by column chromatography to obtain the cationic lipid **E5-1** (0.99 g). ¹H NMR (400 MHz, CDCl₃) δ: 4.71-4.63 (m, 1H), 4.11 (t, 2H), 4.05 (t, 2H), 3.57-3.54 (m, 1H), 2.53-2.43 (m, 2H), 2.38-2.18 (m, 6H), 1.71-1.22 (m, 68H), 0.89 (t, 12H). MS (ESI): m/z=782.72 ([M+H]⁺).

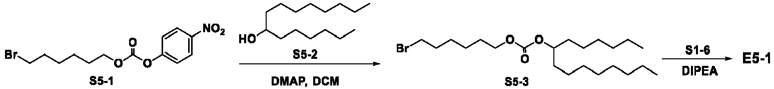

### Example-6: Cationic lipid (E6-1)

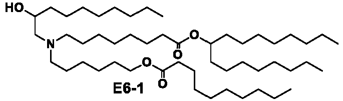

The preparation process is as follows:
The above compound **S1-6** (0.64 g, 1.5 mmol) was dissolved in acetonitrile (10 mL) under nitrogen protection, and then **S6-1** (0.87 g, 1.9 mmol, **S6-1** was obtained by the reaction between 8-bromooctanoic acid represented by 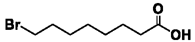 and 9-heptadecanol represented by 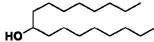 ) and **DIPEA** (0.14 g, 1.5 mmol) were added successively under slow stirring. The reaction was stirred at room temperature for about 20 h. After the reaction was completed, the reaction solution was concentrated, then dissolved in dichloromethane, and then extracted with 0.6 M hydrochloric acid/10% sodium chloride solution and saturated sodium bicarbonate solution in sequence. The organic phases were combined, dried over anhydrous magnesium sulfate, filtered and the filtrate was concentrated, and purified by column chromatography to obtain the cationic lipid **E6-1** (1.02 g). ¹H NMR (400 MHz, CDCl₃) δ: 4.91-4.83 (m, 1H), 4.06 (t, 2H), 3.57-3.54 (m, 1H), 2.53-2.43 (m, 2H), 2.38-2.17 (m, 8H), 1.71-1.22 (m, 74H), 0.88 (t, 12H). MS (ESI): m/z=808.77 ([M+H]⁺).

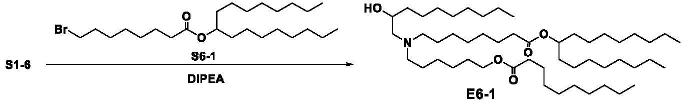

### Example-7: Cationic lipid (E7-1)

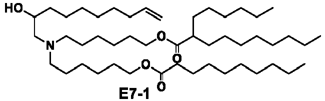

The preparation process is as follows:
Step a: compound **S1-4** (0.81 g, 3.0 mmol) and 1,2-epoxy-9-decene (**S7-1,** 0.46 g, 3.0 mmol) were dissolved in 15 mL acetonitrile, and then **Ca(OTf)₂** (0.51 g, 1.5 mmol) was added to the solution. The mixture was stirred at room temperature until TLC showed that the reaction was complete. Then, acetonitrile was removed by rotary evaporation, water (5 mL) was added and mixed evenly, and the mixture was extracted with dichloromethane (5 mL*3). The organic layers were combined, then dried with anhydrous Na₂SO₄, filtered and the filtrate was concentrated, and then purified by column chromatography to obtain the ring-opened product **S7-2** (1.02 g) with a hydroxyl group.
Step b: the above compound **S7-2** (0.85 g, 2.0 mmol) was dissolved in acetonitrile (15 mL) under nitrogen protection, and **S1-7** (1.05 g, 2.5 mmol) and **DIPEA** (0.18 g, 2.0 mmol) were added to the solution in sequence under slow stirring. The reaction was stirred at room temperature for about 20 h. After the end of the reaction, the reaction solution was concentrated, then dissolved in dichloromethane, and extracted with 0.6 M hydrochloric acid/10% sodium chloride solution and saturated sodium bicarbonate solution in sequence. The organic phases were combined, dried over anhydrous magnesium sulfate, filtered and the filtrate was concentrated, and purified by column chromatography to obtain the cationic lipid **E7-1** (1.28 g). ¹H NMR (400 MHz, CDCl₃) δ: 5.87-5.75 (m, 1H), 5.05-4.82 (d, 2H), 4.07 (t, 4H), 3.57-3.54 (m, 1H), 2.53-2.43 (m, 2H), 2.38-2.17 (m, 7H), 2.11-1.94 (m, 2H), 1.71-1.22 (m, 64H), 0.88 (t, 9H). MS (ESI): m/z=764.71 ([M+H]⁺).

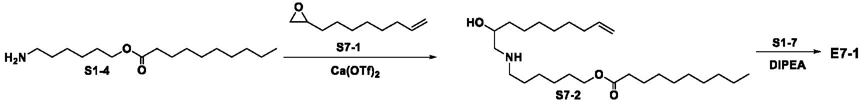

### Example-8: Cationic lipid (E8-1)

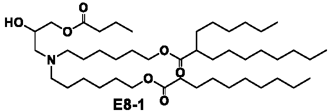

The starting material **S7-1** in Example 7 was replaced with glycidyl butyrate (**S8-1** represented by 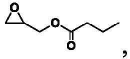 0.43 g, 3.0 mmol), and the same reaction steps were followed to obtain **E8-1** (1.26 g). ¹H NMR (400 MHz, CDCl₃) δ: 4.12-4.00 (m, 6H), 3.86-3.78 (m, 1H), 2.53-2.43 (m, 2H), 2.32-2.19 (m, 9H), 1.72-1.22 (m, 56H), 0.90 (t, 12H). MS (ESI): m/z=754.65 ([M+H]⁺).

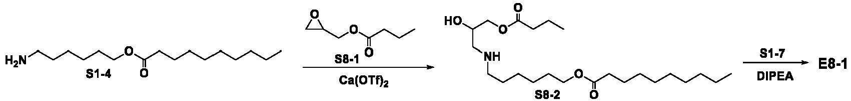

### Example-9: Cationic lipid (E9-1)

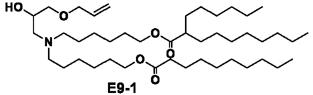

The starting material **S7-1** in Example 7 was replaced with allyl glycidyl ether (**S9-1** represented by 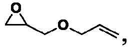 0.34 g, 3.0 mmol), and the same reaction steps were followed to obtain **E9-1** (1.21 g). ¹H NMR (400 MHz, CDCl₃) δ: 5.85-5.76 (m, 1H), 5.16-5.07 (d, 2H), 4.07 (t, 4H), 3.96-3.92 (d, 2H), 3.76-3.69 (m, 1H), 3.36-3.32 (m, 2H), 2.53-2.43 (m, 2H), 2.38-2.17 (m, 7H), 1.71-1.22 (m, 54H), 0.88 (t, 9H). MS (ESI): m/z=724.64 ([M+H]⁺).

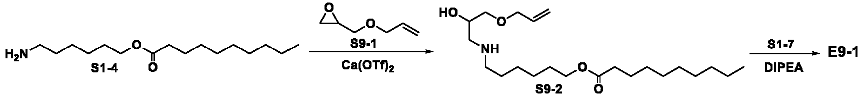

### Example-10: Cationic lipid (E10-1)

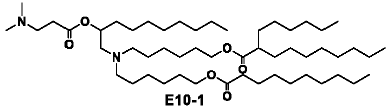

The preparation process is as follows:
Under nitrogen protection, **DCC** (0.91 g, 4.4 mmol) was added to a round-bottomed flask containing **E1-1** (1.84 g, 2.4 mmol), 3-(dimethylamino)propionic acid (**S10-1,** 0.23 g, 2.0 mmol) and **DMAP** (61.00 mg, 0.5 mmol) dissolved in dichloromethane (30 mL), and the reaction was conducted at room temperature for 16 h. After the end of the reaction, the precipitate was removed by filtration, the filtrate was concentrated, and the crude product was purified by column chromatography to obtain the cationic lipid **E10-1** (1.47 g). ¹H NMR (400 MHz, CDCl₃) δ: 4.94-4.88 (m, 1H), 4.05-4.01 (m, 4H), 2.62-2.24 (m, 13H), 2.24-2.22 (s, 6H), 1.59-1.21 (m, 68H), 0.87 (t, 12H). MS (ESI): m/z=865.79 ([M+H]⁺).

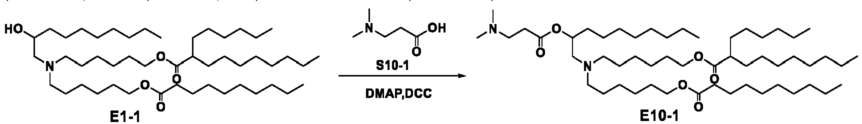

### Example-11: Cationic lipid (E11-1)

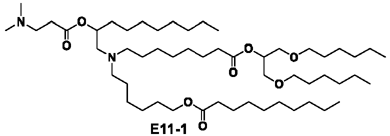

The starting material **E1-1** in Example **10** was replaced with **E2-1** (1.95 g, 2.4 mmol), and the same reaction steps were followed to obtain **E11-1** (1.52 g). ¹H NMR (400 MHz, CDCl₃) δ: 5.13-5.06 (m, 1H), 4.94-4.88 (m, 1H), 4.02 (t, 2H), 3.57-3.49 (m, 4H), 3.46-3.36 (m, 4H), 2.62-2.22 (m, 20H), 1.60-1.20 (m, 62H), 0.88 (t, 12H). MS (ESI): m/z=911.79 ([M+H]⁺).

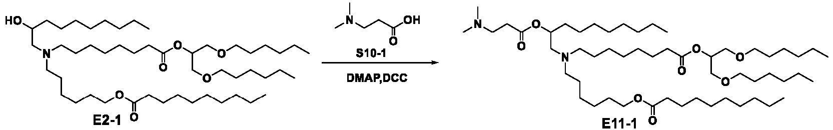

### Example-12: Cationic lipid (E12-1)

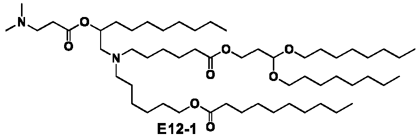

The starting material **E1-1** in Example **10** was replaced with **E3-1** (2.02 g, 2.4 mmol), and the same reaction steps were followed to obtain **E12-1** (1.58 g). ¹H NMR (400 MHz, CDCl₃) δ: 4.94-4.88 (m, 1H), 4.65 (t, 1H), 4.07 (t, 4H), 3.52-3.36 (m, 4H), 2.61-2.19 (m, 20H), 1.71-1.22 (m, 68H), 0.87 (t, 12H). MS (ESI): m/z=939.83 ([M+H]⁺).

### Example-13: Cationic lipid (E13-1)

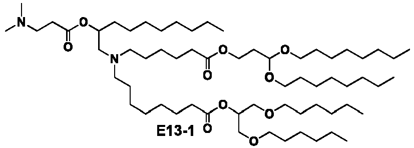

The starting material **E1-1** in Example **10** was replaced with **E4-1** (2.33 g, 2.4 mmol), and the same reaction steps were followed to obtain **E13-1** (1.79 g). ¹H NMR (400 MHz, CDCl₃) δ: 5.13-5.06 (m, 1H), 4.94-4.86 (m, 1H), 4.65 (t, 1H), 4.07 (t, 2H), 3.57-3.36 (m, 12H), 2.62-2.17 (m, 20H), 1.71-1.22 (m, 72H), 0.89 (t, 15H). MS (ESI): m/z=1069.93 ([M+H]⁺).

### Example-14: Cationic lipid (E14-1)

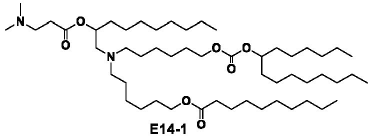

The starting material **E1-1** in Example **10** was replaced with **E5-1** (1.88 g, 2.4 mmol), and the same reaction steps were followed to obtain **E14-1** (1.46 g). ¹H NMR (400 MHz, CDCl₃) δ: 4.98-4.87 (m, 1H), 4.71-4.63 (m, 1H), 4.10 (t, 2H), 4.04 (t, 2H), 2.64-2.57 (m, 2H), 2.52-2.34 (m, 8H), 2.31-2.18 (m, 8H), 1.65-1.53 (m, 10H), 1.39-1.23 (m, 58H), 0.88 (t, 12H). MS (ESI): m/z=881.79 ([M+H]⁺).

### Example-15: Cationic lipid (E15-1)

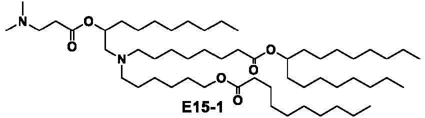

The starting material **E1-1** in Example **10** was replaced with **E6-1** (1.94 g, 2.4 mmol), and the same reaction steps were followed to obtain **E15-1** (1.52 g). ¹H NMR (400 MHz, CDCl₃) δ: 4.94-4.83 (m, 2H), 4.06 (t, 2H), 2.63-2.17 (m, 20H), 1.71-1.22 (m, 74H), 0.88 (t, 12H). MS (ESI): m/z=907.85 ([M+H]⁺).

### Example-16: Cationic lipid (E16-1)

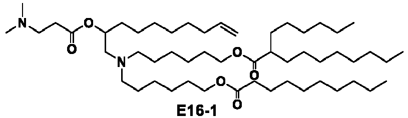

The starting material **E1-1** in Example **10** was replaced with **E7-1** (1.83 g, 2.4 mmol), and the same reaction steps were followed to obtain **E16-1** (1.44 g). ¹H NMR (400 MHz, CDCl₃) δ: 5.87-5.75 (m, 1H), 5.05-4.82 (m, 3H), 4.07 (t, 4H), 2.63-2.17 (m, 19H), 2.11-1.94 (m, 2H), 1.71-1.22 (m, 64H), 0.88 (t, 9H). MS (ESI): m/z=863.77 ([M+H]⁺).

### Example-17: Cationic lipid (E17-1)

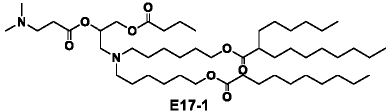

The starting material **E1-1** in Example **10** was replaced with **E8-1** (1.81 g, 2.4 mmol), and the same reaction steps were followed to obtain **E17-1** (1.44 g). ¹H NMR (400 MHz, CDCl₃) δ: 4.94-4.88 (m, 1H), 4.12-4.00 (m, 6H), 2.63-2.19 (m, 21H), 1.72-1.22 (m, 56H), 0.90 (t, 12H). MS (ESI): m/z=853.73 ([M+H]⁺).

### Example-18: Cationic lipid (E18-1)

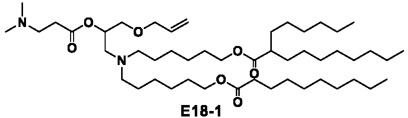

The starting material **E1-1** in Example **10** was replaced with **E9-1** (1.74 g, 2.4 mmol), and the same reaction steps were followed to obtain **E18-1** (1.39 g). ¹H NMR (400 MHz, CDCl₃) δ: 5.85-5.76 (m, 1H), 5.16-5.07 (d, 2H), 4.94-4.88 (m, 1H), 4.07 (t, 4H), 3.96-3.92 (d, 2H), 3.36-3.32 (m, 2H), 2.63-2.19 (m, 19H), 1.71-1.22 (m, 54H), 0.88 (t, 9H). MS (ESI): m/z=823.71 ([M+H]⁺).

### Example-19: Cationic lipid (E19-1)

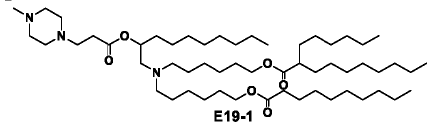

The starting material **S10-1** in Example **10** was replaced with 3-(4-phenyl-piperazin-1-yl)-propionic acid (**S19-1** represented by 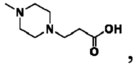 0.34 g, 2.0 mmol), and the same reaction steps were followed to obtain **E19-1** (1.56 g). ¹H NMR (400 MHz, CDCl₃) δ: 4.93-4.87 (m, 1H), 4.04-4.00 (m, 4H), 2.68-2.24 (m, 24H), 1.60-1.21 (m, 68H), 0.86 (t, 12H). MS (ESI): m/z=920.83 ([M+H]⁺).

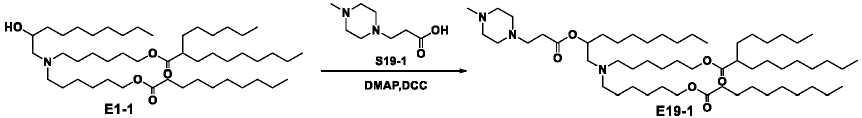

### Example-20: Cationic lipid (E20-1)

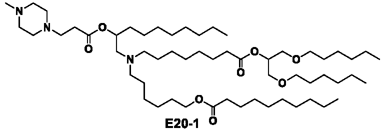

The starting material **E1-1** in Example **19** was replaced with **E2-1** (1.95 g, 2.4 mmol), and the same reaction steps were followed to obtain **E20-1** (1.62 g). ¹H NMR (400 MHz, CDCl₃) δ: 5.13-5.06 (m, 1H), 4.93-4.87 (m, 1H), 4.05-3.99 (t, 2H), 3.57-3.49 (m, 4H), 3.46-3.36 (m, 4H), 2.68-2.24 (m, 25H), 1.60-1.20 (m, 62H), 0.88 (t, 12H). MS (ESI): m/z=966.84 ([M+H]⁺).

### Example-21: Cationic lipid (E21-1)

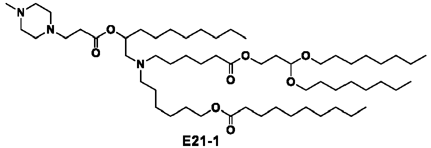

The starting material **E1-1** in Example **19** was replaced with **E3-1** (2.02 g, 2.4 mmol), and the same reaction steps were followed to obtain **E21-1** (1.67 g). ¹H NMR (400 MHz, CDCl₃) δ: 4.93-4.87 (m, 1H), 4.65 (t, 1H), 4.07 (t, 4H), 3.52-3.36 (m, 4H), 2.63-2.23 (m, 25H), 1.71-1.22 (m, 68H), 0.87 (t, 12H). MS (ESI): m/z=994.87 ([M+H]⁺).

### Example-22: Cationic lipid (E22-1)

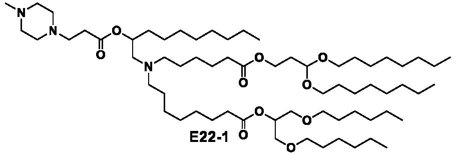

The starting material **E1-1** in Example **19** was replaced with **E4-1** (2.33 g, 2.4 mmol), and the same reaction steps were followed to obtain **E22-1** (1.87 g). ¹H NMR (400 MHz, CDCl₃) δ: 5.13-5.06 (m, 1H), 4.93-4.87 (m, 1H), 4.64 (t, 1H), 4.07 (t, 2H), 3.57-3.36 (m, 12H), 2.62-2.23 (m, 25H), 1.71-1.22 (m, 72H), 0.89 (t, 15H). MS (ESI): m/z=1124.97 ([M+H]⁺).

### Example-23: Cationic lipid (E23-1)

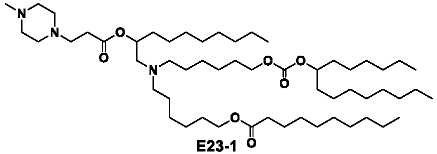

The starting material **E1-1** in Example **19** was replaced with **E5-1** (1.88 g, 2.4 mmol), and the same reaction steps were followed to obtain **E23-1** (1.56 g). ¹H NMR (400 MHz, CDCl₃) δ: 4.98-4.88 (m, 1H), 4.71-4.63 (m, 1H), 4.10 (t, 2H), 4.05 (t, 2H), 2.63-2.19 (m, 23H), 1.69-1.22 (m, 68H), 0.89 (t, 12H). MS (ESI): m/z=936.83 ([M+H]⁺).

### Example-24: Cationic lipid (E24-1)

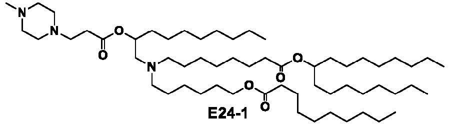

The starting material **E1-1** in Example **19** was replaced with **E6-1** (1.94 g, 2.4 mmol), and the same reaction steps were followed to obtain **E24-1** (1.61 g). ¹H NMR (400 MHz, CDCl₃) δ: 4.93-4.83 (m, 2H), 4.06 (t, 2H), 2.68-2.24 (m, 25H), 1.71-1.22 (m, 74H), 0.88 (t, 12H). MS (ESI): m/z=962.87 ([M+H]⁺).

### Example-25: Cationic lipid (E25-1)

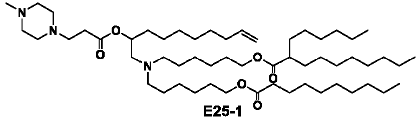

The starting material **E1-1** in Example **19** was replaced with **E7-1** (1.83 g, 2.4 mmol), and the same reaction steps were followed to obtain **E25-1** (1.55 g). ¹H NMR (400 MHz, CDCl₃) δ: 5.87-5.75 (m, 1H), 5.05-4.82 (m, 3H), 4.07 (t, 4H), 2.67-2.23 (m, 24H), 2.11-1.94 (m, 2H), 1.71-1.22 (m, 64H), 0.88 (t, 9H). MS (ESI): m/z=918.82 ([M+H]⁺).

### Example-26: Cationic lipid (E26-1)

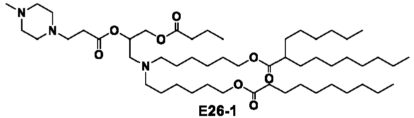

The starting material **E1-1** in Example **19** was replaced with **E8-1** (1.81 g, 2.4 mmol), and the same reaction steps were followed to obtain **E26-1** (1.53 g). ¹H NMR (400 MHz, CDCl₃) δ: 4.93-4.87 (m, 1H), 4.12-4.00 (m, 6H), 2.66-2.23 (m, 26H), 1.72-1.22 (m, 56H), 0.90 (t, 12H). MS (ESI): m/z=908.76 ([M+H]⁺).

### Example-27: Cationic lipid (E27-1)

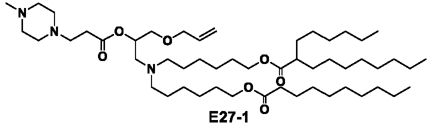

The starting material **E1-1** in Example **19** was replaced with **E9-1** (1.74 g, 2.4 mmol), and the same reaction steps were followed to obtain **E27-1** (1.48 g). ¹H NMR (400 MHz, CDCl₃) δ: 5.85-5.76 (m, 1H), 5.16-5.07 (d, 2H), 4.93-4.87 (m, 1H), 4.07 (t, 4H), 3.96-3.92 (d, 2H), 3.36-3.32 (m, 2H), 2.68-2.22 (m, 24H), 1.71-1.22 (m, 54H), 0.88 (t, 9H). MS (ESI): m/z=878.75 ([M+H]⁺).

### Example-28: Cationic lipid (E28-1)

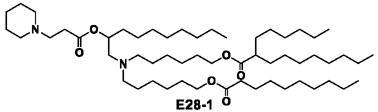

The starting material **S10-1** in Example **10** was replaced with 1-piperidinepropionic acid **(S28-1** represented by 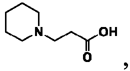 0.31 g, 2.0 mmol), and the same reaction steps were followed to obtain **E28-1** (1.54 g). ¹H NMR (400 MHz, CDCl₃) δ: 4.92-4.87 (m, 1H), 4.04-4.01 (m, 4H), 2.68-2.24 (m, 17H), 1.60-1.21 (m, 74H), 0.86 (t, 12H). MS (ESI): m/z=905.82 ([M+H]⁺).

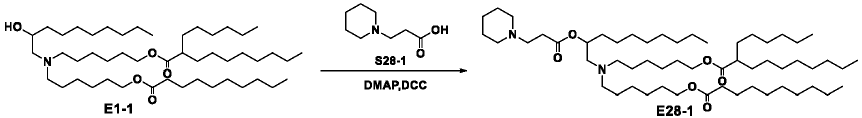

### Example-29: Cationic lipid (E29-1)

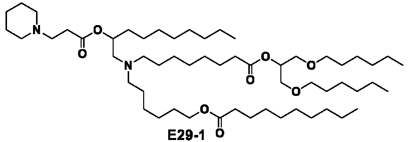

The starting material **E1-1** in Example **28** was replaced with **E2-1** (1.95 g, 2.4 mmol), and the same reaction steps were followed to obtain E29-1 (1.58 g). ¹H NMR (400 MHz, CDCl₃) δ: 5.13-5.06 (m, 1H), 4.93-4.87 (m, 1H), 4.03 (t, 2H), 3.57-3.49 (m, 4H), 3.46-3.36 (m, 4H), 2.68-2.24 (m, 18H), 1.60-1.20 (m, 68H), 0.88 (t, 12H). MS (ESI): m/z=951.83 ([M+H]⁺).

### Example-30: Cationic lipid (E30-1)

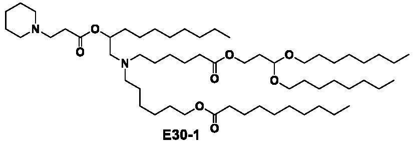

The starting material **E1-1** in Example **28** was replaced with **E3-1** (2.02 g, 2.4 mmol), and the same reaction steps were followed to obtain **E30-1** (1.64 g). ¹H NMR (400 MHz, CDCl₃) δ: 4.93-4.87 (m, 1H), 4.65 (t, 1H), 4.03 (t, 4H), 3.52-3.36 (m, 4H), 2.63-2.23 (m, 18H), 1.71-1.22 (m, 74H), 0.87 (t, 12H). MS (ESI): m/z=979.86 ([M+H]⁺).

### Example-31: Cationic lipid (E31-1)

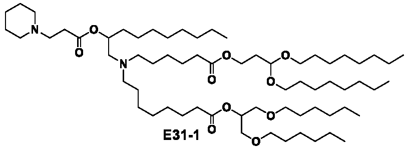

The starting material **E1-1** in Example **28** was replaced with **E4-1** (2.33 g, 2.4 mmol), and the same reaction steps were followed to obtain **E31-1** (1.85 g). ¹H NMR (400 MHz, CDCl₃) δ: 5.13-5.06 (m, 1H), 4.93-4.87 (m, 1H), 4.64 (t, 1H), 4.04 (t, 2H), 3.57-3.36 (m, 12H), 2.62-2.23 (m, 18H), 1.71-1.22 (m, 78H), 0.89 (t, 15H). MS (ESI): m/z=1109.96 ([M+H]⁺).

### Example-32: Cationic lipid (E32-1)

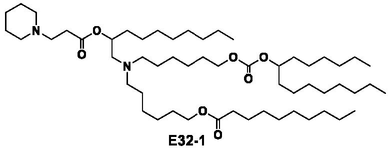

The starting material **E1-1** in Example **28** was replaced with **E5-1** (1.88 g, 2.4 mmol), and the same reaction steps were followed to obtain **E32-1** (1.56 g). ¹H NMR (400 MHz, CDCl₃) δ: 4.97-4.87 (m, 1H), 4.71-4.62 (m, 1H), 4.12 (t, 2H), 4.04 (t, 2H), 2.64-2.20 (m, 16H), 1.68-1.22 (m, 74H), 0.89 (t, 12H). MS (ESI): m/z=921.82 ([M+H]⁺).

### Example-33: Cationic lipid (E33-1)

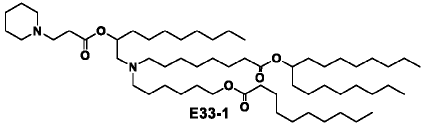

The starting material **E1-1** in Example **28** was replaced with **E6-1** (1.94 g, 2.4 mmol), and the same reaction steps were followed to obtain **E33-1** (1.58 g). ¹H NMR (400 MHz, CDCl₃) δ: 4.94-4.88 (m, 1H), 4.87-4.81 (m, 1H), 4.03 (t, 2H), 2.65-2.23 (m, 18H), 1.60-1.22 (m, 80H), 0.85 (t, 12H). MS (ESI): m/z=947.87 ([M+H]⁺).

### Example-34: Cationic lipid (E34-1)

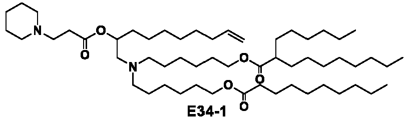

The starting material **E1-1** in Example **28** was replaced with **E7-1** (1.83 g, 2.4 mmol), and the same reaction steps were followed to obtain **E34-1** (1.52 g). ¹H NMR (400 MHz, CDCl₃) δ: 5.88-5.75 (m, 1H), 5.04-4.82 (m, 3H), 4.07 (t, 4H), 2.67-2.23 (m, 17H), 2.11-1.94 (m, 2H), 1.71-1.22 (m, 70H), 0.88 (t, 9H). MS (ESI): m/z=903.81 ([M+H]⁺).

### Example-35: Cationic lipid (E35-1)

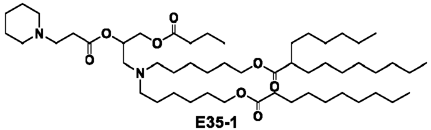

The starting material **E1-1** in Example **28** was replaced with **E8-1** (1.81 g, 2.4 mmol), and the same reaction steps were followed to obtain **E35-1** (1.51 g). ¹H NMR (400 MHz, CDCl₃) δ: 4.93-4.87 (m, 1H), 4.12-4.00 (m, 6H), 2.66-2.23 (m, 19H), 1.72-1.22 (m, 62H), 0.90 (t, 12H). MS (ESI): m/z=893.75 ([M+H]⁺).

### Example-36: Cationic lipid (E36-1)

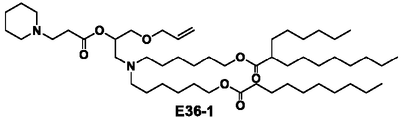

The starting material **E1-1** in Example **28** was replaced with **E9-1** (1.74 g, 2.4 mmol), and the same reaction steps were followed to obtain **E36-1** (1.46 g). ¹H NMR (400 MHz, CDCl₃) δ: 5.86-5.76 (m, 1H), 5.16-5.08 (d, 2H), 4.94-4.88 (m, 1H), 4.05 (t, 4H), 3.96-3.92 (d, 2H), 3.36-3.32 (m, 2H), 2.68-2.22 (m, 17H), 1.71-1.22 (m, 60H), 0.88 (t, 9H). MS (ESI): m/z=863.74 ([M+H]⁺).

### Example-37: Cationic lipid (E37-1)

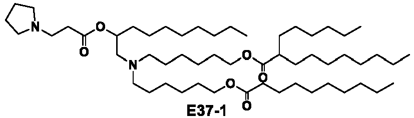

The starting material **S10-1** in Example **10** was replaced with3-(1-pyrrolidinyl)propionic acid (**S37-1** represented by 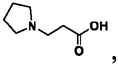 0.29 g, 2.0 mmol), and the same reaction steps were followed to obtain **E37-1** (1.51 g). ¹H NMR (400 MHz, CDCl₃) δ: 4.94-4.87 (m, 1H), 4.04-4.00 (m, 4H), 2.68-2.24 (m, 17H), 1.75-1.21 (m, 72H), 0.86 (t, 12H). MS (ESI): m/z=891.81 ([M+H]⁺).

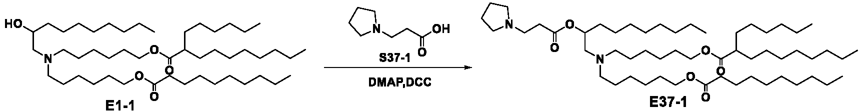

### Example-38: Cationic lipid (E38-1)

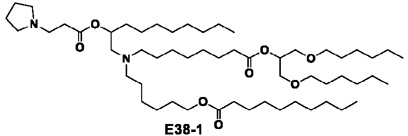

The starting material **E1-1** in Example **37** was replaced with E2-1 (1.95 g, 2.4 mmol), and the same reaction steps were followed to obtain **E38-1** (1.56 g). ¹H NMR (400 MHz, CDCl₃) δ: 5.14-5.06 (m, 1H), 4.93-4.87 (m, 1H), 4.05-3.99 (t, 2H), 3.57-3.49 (m, 4H), 3.46-3.36 (m, 4H), 2.68-2.24 (m, 18H), 1.75-1.20 (m, 66H), 0.88 (t, 12H). MS (ESI): m/z=937.81 ([M+H]⁺).

### Example-39: Cationic lipid (E39-1)

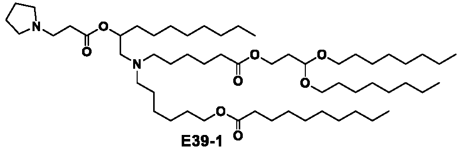

The starting material **E1-1** in Example 37 was replaced with **E3-1** (2.02 g, 2.4 mmol), and the same reaction steps were followed to obtain E39-1 (1.62 g). ¹H NMR (400 MHz, CDCl₃) δ: 4.93-4.87 (m, 1H), 4.65 (t, 1H), 4.07 (t, 4H), 3.52-3.36 (m, 4H), 2.63-2.23 (m, 18H), 1.77-1.22 (m, 72H), 0.87 (t, 12H). MS (ESI): m/z=965.84 ([M+H]⁺).

### Example-40: Cationic lipid (E40-1)

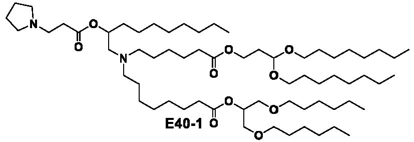

The starting material **E1-1** in Example **37** was replaced with **E4-1** (2.33 g, 2.4 mmol), and the same reaction steps were followed to obtain **E40-1** (1.86 g). ¹H NMR (400 MHz, CDCl₃) δ: 5.13-5.06 (m, 1H), 4.93-4.87 (m, 1H), 4.64 (t, 1H), 4.07 (t, 2H), 3.57-3.36 (m, 12H), 2.62-2.23 (m, 18H), 1.75-1.22 (m, 76H), 0.89 (t, 15H). MS (ESI): m/z=1095.94 ([M+H]⁺).

### Example-41: Cationic lipid (E41-1)

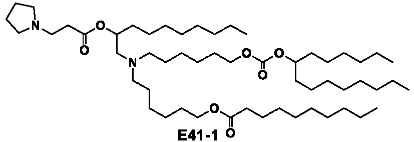

The starting material **E1-1** in Example **37** was replaced with **E5-1** (1.88 g, 2.4 mmol), and the same reaction steps were followed to obtain **E41-1** (1.52 g). ¹H NMR (400 MHz, CDCl₃) δ:4.98-4.87 (m, 1H), 4.71-4.63 (m, 1H), 4.10 (t, 2H), 4.04 (t, 2H), 2.63-2.19 (m, 16H) , 1.75-1.22 (m, 72H), 0.89 (t, 12H). MS (ESI): m/z=907.80 ([M+H]⁺).

### Example-42: Cationic lipid (E42-1)

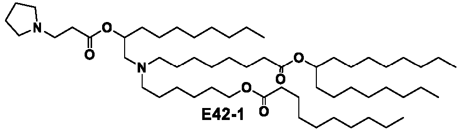

The starting material **E1-1** in Example **37** was replaced with **E6-1** (1.94 g, 2.4 mmol), and the same reaction steps were followed to obtain **E42-1** (1.56 g). ¹H NMR (400 MHz, CDCl₃) δ: 4.93-4.83 (m, 2H), 4.06 (t, 2H), 2.68-2.24 (m, 18H), 1.76-1.22 (m, 78H), 0.88 (t, 12H). MS (ESI): m/z=933.86 ([M+H]⁺).

### Example-43: Cationic lipid (E43-1)

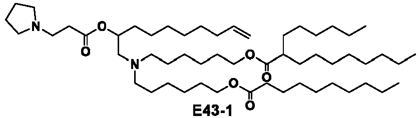

The starting material **E1-1** in Example **37** was replaced with **E7-1** (1.83 g, 2.4 mmol), and the same reaction steps were followed to obtain **E43-1** (1.50 g). ¹H NMR (400 MHz, CDCl₃) δ: 5.87-5.75 (m, 1H), 5.05-4.82 (m, 3H), 4.07 (t, 4H), 2.67-2.23 (m, 17H), 2.11-1.94 (m, 2H), 1.75-1.22 (m, 68H), 0.88 (t, 9H). MS (ESI): m/z=889.79 ([M+H]⁺).

### Example-44: Cationic lipid (E44-1)

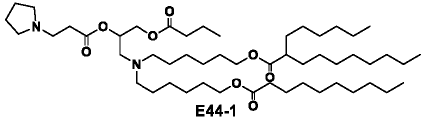

The starting material **E1-1** in Example **37** was replaced with **E8-1** (1.81 g, 2.4 mmol), and the same reaction steps were followed to obtain **E44-1** (1.48 g). ¹H NMR (400 MHz, CDCl₃) δ: 4.93-4.87 (m, 1H), 4.12-4.00 (m, 6H), 2.66-2.23 (m, 19H), 1.75-1.22 (m, 60H), 0.90 (t, 12H). MS (ESI): m/z=879.73 ([M+H]⁺).

### Example-45: Cationic lipid (E45-1)

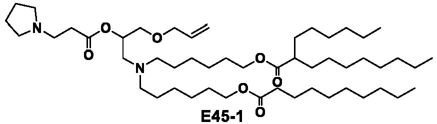

The starting material **E1-1** in Example **37** was replaced with **E9-1** (1.74 g, 2.4 mmol), and the same reaction steps were followed to obtain **E45-1** (1.44 g). ¹H NMR (400 MHz, CDCl₃) δ: 5.85-5.76 (m, 1H), 5.16-5.07 (d, 2H), 4.93-4.87 (m, 1H), 4.07 (t, 4H), 3.96-3.92 (d, 2H), 3.36-3.32 (m, 2H), 2.68-2.22 (m, 17H), 1.76-1.22 (m, 58H), 0.88 (t, 9H). MS (ESI): m/z=849.72 ([M+H]⁺).

### Example 46. Preparation of PEGylated lipids E46-1 and E46-2

### Example 46.1 Preparation of PEGylated lipid E46-1

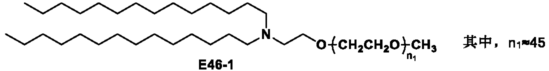

The preparation process is as follows:
Step a: compound **S46-1** (20.00 g, 10.0 mmol, mPEG-OH, Mw≈2000, n₁≈45, PDI=1.03) and toluene (200 mL) underwent the azeotropic water removal at 140°C. After 60 mL of solvent was evaporated, the reaction was cooled to room temperature. **TEA** (2.02 g, 20.0 mmol) and **MsCl** (2.05 g, 18.0 mmol) were added, and the reaction was stirred overnight at room temperature. After the reaction, the reaction solution was added into water (200 mL), and then extracted twice with EtOAc (100 mL*2). The aqueous phase was retained, and the aqueous phase was extracted twice with dichloromethane (100 mL*2). The organic phases were then combined, dried, filtered, concentrated, dissolved at 50 °C with isopropanol, and then recrystallized in an ice bath, and filtered to obtain compound **S46-2** (18.00 g, 90%).
Step b: the above compound **S46-2** (18.00 g, 9.0 mmol) was added to 80 mL of water and dissolved at room temperature with stirring. **Potassium carbonate** (12.42 g, 90.0 mmol), compound **S46-3** (9.58 g, 45.0 mmol) and **tetra-n-butylammonium bromide** (0.29 g, 0.9 mmol) were added, and the reaction solution was stirred at room temperature for 72 h. At the end of the reaction, the reaction solution was extracted twice with dichloromethane (100 mL*2), and the organic phases were combined and then backwashed once with saturated aqueous sodium chloride (100 mL). The organic phase was retained, dried over anhydrous sodium sulfate, filtered, and the filtrate was concentrated to obtain the crude compound **S46-4.** The crude product was purified by column chromatography, concentrated, and dried with an oil pump to obtain the target compound **S46-4** (12.00 g).
Step c: the above compound **S46-4** (12.00 g, 6.0 mmol) was dissolved in dry THF (120 mL), and **NaH** (60%, 2.40 g, 60.0 mmol) was slowly added to the solution in an ice bath and reacted in an ice bath for 1 h. Then, compound **S46-5** (8.28 g, 30.0 mmol) was added to the solution, and the mixture was stirred and reacted in an ice bath for 1 h. Afterward, the reaction solution was slowly cooled to room temperature and the reaction continued overnight. After the reaction was over, the reaction solution was under an ice bath, and 2 mL of methanol was slowly added to quench the reaction. After stirring for 30 min, water (300 mL) was added and stirred to mix the solution. The reaction solution was extracted twice with EtOAc (150 mL*2). The aqueous phase was retained and then extracted twice with dichloromethane (100 mL*2). The organic phases were collected and combined, and then backwashed once with saturated sodium chloride (100 mL). The organic phase was dried with anhydrous sodium sulfate, then filtered, and the filtrate was concentrated to obtain the crude product of PEGylated lipid **E46-1.** The crude product was purified by column chromatography, then concentrated and dried with an oil pump to obtain the PEGylated lipid **E46-1** (9.00 g). ¹H NMR (400 MHz, CDCl₃) δ: 3.85-3.45 (m, 182H), 3.37 (s, 3H), 3.15 (t, 2H), 2.94 (t, 2H), 2.62 (t, 2H), 1.58-1.48 (m, 4H), 1.36-1.19 (m, 44H), 0.86 (t, 6H). The molecular weight of **E46-1** was determined to be 2447 Da by MALDI-TOF-MS, with PDI=1.03.

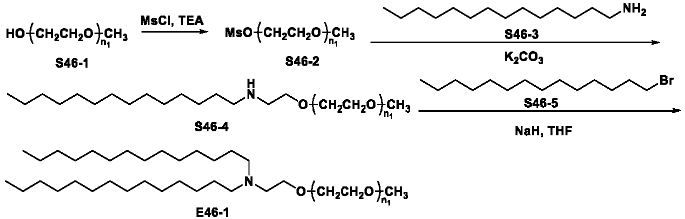

### Example 46.2 Preparation of PEGylated lipid E46-2

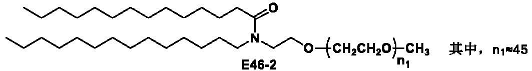

The preparation process is as follows:

The above compounds **S46-4** (11.26 g, 5.0 mmol), **S46-6** (1.95 g, 6.0 mmol), and triethylamine **(TEA,** 0.76 g, 7.5 mmol) were dissolved in dichloromethane (100 mL) and reacted overnight at room temperature with stirring. Then, the reaction solution was concentrated, dissolved with 100 mL of water, and then extracted twice with EtOAc (100 mL*2). The aqueous phase was retained and added with sodium chloride. The mixture was then extracted twice with dichloromethane (100 mL*2). The organic phases were combined and then backwashed once with saturated NaCl (100 mL). The organic phase was dried with anhydrous magnesium sulfate, then filtered and the filtrate was concentrated. The crude product was purified by column chromatography, then concentrated, and dried with an oil pump to obtain the PEGylated lipid **E46-2** (10.1 g, 84.8%). ¹H NMR (400 MHz, CDCl₃) δ: 3.84-3.45 (m, 182H), 3.37 (s, 3H), 3.35 (t, 2H), 3.18 (t, 2H), 2.27 (t, 2H), 1.56-1.40 (m, 4H), 1.36-1.18 (m, 46H), 0.87 (t, 6H). The molecular weight of **E46-2** was determined to be 2461 Da by MALDI-TOF-MS, with PDI=1.03.

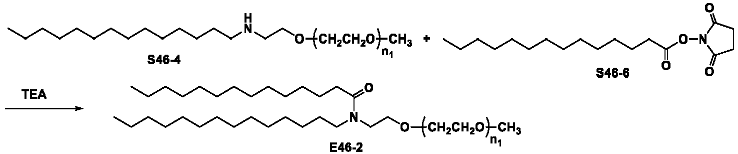

### Example 47: Preparation of LNP-mRNA drug composition and assays of its physicochemical properties

### Example 47.1: Preparation of LNP-mRNA drug composition

In the present embodiment, multiple groups of LNP-mRNA drug compositions containing Fluc-mRNA were prepared for comparison. The phospholipids contained in each group were all DSPC, and the steroid lipids contained were all cholesterol. The differences in each group were the cationic lipid and PEGylated lipid. Wherein, the control group L-0 contained the cationic lipid ALC-0315 and the PEGylated lipid PEG2k-DMG (abbreviated as DMG); the experimental group series (L-1~L-45) each contained a cationic lipid prepared in the Examples of the present application, and also contained the PEGylated lipid PEG2k-DMG; the experimental groups (L-46~L-47) each contained a cationic lipid prepared in the Examples of the present application, and also contained the PEGylated lipid **E46-1** or **E46-2** prepared in this application, specific as shown in Table 1.

The preparation method for LNP-mRNA drug composition is as follows:
Step a: cationic lipid, DSPC, cholesterol, and PEG-lipid were dissolved in ethanol at a molar ratio of 48:9:42:1.5 to obtain an ethanol phase solution;
Step b: Fluc-mRNA was added to 10~50 mM citrate buffer (pH=4) to obtain an aqueous solution;
Step c: the ethanol phase solution and the aqueous phase solution were mixed (1:3 v/v) to prepare LNP-mRNA, and washed by multiple DPBS ultrafiltration to remove ethanol and free molecules, and finally, filtered through a sterile filter of 0.2 µm to obtain the LNP-mRNA drug composition.

### Example 47.2: Assays of physicochemical properties of LNP-mRNA drug compositions

Determination of encapsulation rate: the encapsulation rate of LNP-mRNA compositions was determined using the Quant-it Ribogreen RNA Quantification Kit. The results showed that the lipid compositions (L-1~L-47) of the present application had high encapsulation rates for nucleic acid drugs (mRNA), all in the range of 80%-98%, and most of the encapsulation rates were in the range of 90%-98%. The results showed that the lipid compositions prepared by the cationic lipids in most experimental groups could encapsulate mRNA well, showing an encapsulation rate that is close to or even better than that of the existing cationic lipid ALC-0315. There were also differences in the encapsulation rate among different cationic lipids.

Determination of particle size: in the present embodiment, the particle size of LNP-mRNA was determined by dynamic light scattering (DLS), and the results are shown in Table 1 below. The measured LNP-mRNA had high dimensional uniformity, and the PDI values were all less than 0.3. The particle sizes of the LNP-mRNA prepared with the lipid compositions of the present application were in the range of 90-120 nm.

**Table 1: Summary of the formulation of each lipid composition, and particle size and encapsulation rate of the prepared LNP-mRNA**

| **LNP-mRNA** | **L-0** | **L-1** | **L-2** | **L-3** | **L-4** | **L-5** |
|---|---|---|---|---|---|---|
| **Cationic Lipid, CL** | ALC-0315 | E1-1 | E2-1 | E3-1 | E4-1 | E5-1 |
| **PEGylated Lipid, PL** | DMG | | | | | |
| **Encapsulation Rate /%** | 93.22 | 97.86 | 97.39 | 93.23 | 94.86 | 93.74 |
| **Particle Size /nm** | 91.05 | 103.47 | 95.61 | 93.10 | 93.17 | 94.34 |

| **LNP-mRNA** | **L-6** | **L-7** | **L-8** | **L-9** | **L-10** | **L-11** |
|---|---|---|---|---|---|---|
| **Cationic Lipid, CL** | E6-1 | E7-1 | E8-1 | E9-1 | E10-1 | E11-1 |
| **PEGylated Lipid, PL** | DMG | | | | | |
| **Encapsulation Rate /%** | 93.30 | 92.37 | 94.06 | 93.82 | 97.55 | 94.23 |
| **Particle Size /nm** | 97.33 | 95.88 | 108.20 | 92.53 | 95.10 | 93.10 |

| **LNP-mRNA** | **L-12** | **L-13** | **L-14** | **L-15** | **L-16** | **L-17** |
|---|---|---|---|---|---|---|
| **Cationic Lipid, CL** | E12-1 | E13-1 | E14-1 | E15-1 | E16-1 | E17-1 |
| **PEGylated Lipid, PL** | DMG | | | | | |
| **Encapsulation Rate /%** | 93.67 | 91.85 | 97.96 | 94.53 | 92.16 | 93.50 |
| **Particle Size /nm** | 96.12 | 93.33 | 97.30 | 106.25 | 93.26 | 93.10 |

| **LNP-mRNA** | **L-18** | **L-19** | **L-20** | **L-21** | **L-22** | **L-23** |
|---|---|---|---|---|---|---|
| **Cationic Lipid, CL** | E18-1 | E19-1 | E20-1 | E21-1 | E22-1 | E23-1 |
| **PEGylated Lipid, PL** | DMG | | | | | |
| **Encapsulation Rate /%** | 94.80 | 97.73 | 93.88 | 94.84 | 93.05 | 93.36 |
| **Particle Size /nm** | 106.25 | 93.33 | 92.43 | 94.10 | 93.23 | 95.61 |

| **LNP-mRNA** | **L-24** | **L-25** | **L-26** | **L-27** | **L-28** | **L-29** |
|---|---|---|---|---|---|---|
| **Cationic Lipid, CL** | E24-1 | E25-1 | E26-1 | E27-1 | E28-1 | E29-1 |
| **PEGylated Lipid, PL** | DMG | | | | | |
| **Encapsulation Rate /%** | 93.56 | 93.42 | 94.69 | 93.06 | 94.66 | 95.39 |
| **Particle Size /nm** | 92.53 | 98.29 | 94.34 | 95.68 | 93.19 | 97.37 |

| **LNP-mRNA** | **L-30** | **L-31** | **L-32** | **L-33** | **L-34** | **L-35** |
|---|---|---|---|---|---|---|
| **Cationic Lipid, CL** | E30-1 | E31-1 | E32-1 | E33-1 | E34-1 | E35-1 |
| **PEGylated Lipid, PL** | DMG | | | | | |
| **Encapsulation Rate** /% | 92.94 | 95.36 | 92.84 | 97.35 | 94.83 | 93.38 |
| **Particle Size /nm** | 101.33 | 92.43 | 95.43 | 96.12 | 97.40 | 93.36 |

| **LNP-mRNA** | **L-36** | **L-37** | **L-38** | **L-39** | **L-40** | **L-41** |
|---|---|---|---|---|---|---|
| **Cationic Lipid, CL** | E36-1 | E37-1 | E38-1 | E39-1 | E40-1 | E41-1 |
| **PEGylated Lipid, PL** | DMG | | | | | |
| **Encapsulation Rate /%** | 94.16 | 95.44 | 96.78 | 95.82 | 95.31 | 94.20 |
| **Particle Size /nm** | 106.22 | 93.24 | 95.10 | 93.10 | 97.31 | 93.27 |

| **LNP-mRNA** | **L-42** | **L-43** | **L-44** | **L-45** | **L-46** | **L-47** |
|---|---|---|---|---|---|---|
| **Cationic Lipid, CL** | E42-1 | E43-1 | E44-1 | E45-1 | E10-1 | E10-1 |
| **PEGylated Lipid, PL** | DMG | | | | E46-1 | E46-2 |
| **Encapsulation Rate /%** | 94.48 | 93.79 | 95.81 | 96.32 | 95.67 | 95.87 |
| **Particle Size /nm** | 98.16 | 95.61 | 92.43 | 95.10 | 96.12 | 95.43 |

### Example 48: Biological activity assays of LNP-mRNA drug composition formulations (1) Assays for cytotoxicity (biocompatibility)

The cytotoxicity of the formulation of LNP-mRNA drug composition of the present application was tested by MTT assay. The formulation of LNP-mRNA drug composition was dissolved in the culture medium to prepare the required concentration; if necessary, an appropriate amount of co-solvent could be added. 293T cells were used as a cell model, and 100 µL/well of cell suspension was seeded into 96-well plates at a density of 1×10⁴ cells/well. After inoculation, the cells were incubated in a cell culture incubator at 37 °C, 4% CO₂ for 24 h, and then the culture medium was discarded. Then, 100 µL culture medium containing the LNP-mRNA drug composition (prepared in Example 47) was added into each well of the experimental group; and 100 µL of fresh culture medium was added to the blank control group. Each group had six replicate wells. After 24 h co-incubation of the formulation of LNP-mRNA drug composition with 293T cells, 20 µL of PBS buffer containing 5 mg/mL MTT was added to each well. After 4 h incubation of MTT with cancer cells, the mixture of medium and MTT buffer was discarded, followed by adding 150 µL DMSO per well to dissolve the purple formazan crystals formed in living cells. After sufficient shaking, the absorbance was tested with a microplate reader and calculated from the measured absorbance value. The results showed that, compared with the blank control group, the cell viability rate of the formulation of LNP-mRNA drug compositions prepared by the present application was all greater than 96%. The results are shown in Table 2, indicating that the formulation of LNP-mRNA drug composition of the present application had good biocompatibility.

**Table 2: Cytotoxicity Results**

| **Group** | L-0 | L-1 | L-2 | L-3 | L-4 | L-5 | L-6 | L-7 |
|---|---|---|---|---|---|---|---|---|
| **Cell Survival Rate** | 98.05% | 99.04% | 99.13% | 97.64% | 96.41% | 97.18% | 96.73% | 98.62% |

| **Group** | L-8 | L-9 | L-10 | L-11 | L-12 | L-13 | L-14 | L-15 |
|---|---|---|---|---|---|---|---|---|
| **Cell** | 97.75% | 98.77% | 99.26% | 97.42% | 98.29% | 98.16% | 99.06% | 99.01% |
| **Survival Rate** | | | | | | | | |

| **Group** | L-16 | L-17 | L-18 | L-19 | L-20 | L-21 | L-22 | L-23 |
|---|---|---|---|---|---|---|---|---|
| **Cell Survival Rate** | 96.49% | 98.25% | 98.62% | 99.28% | 97.63% | 97.42% | 98.73% | 98.83% |

| **Group** | L-24 | L-25 | L-26 | L-27 | L-28 | L-29 | L-30 | L-31 |
|---|---|---|---|---|---|---|---|---|
| **Cell Survival Rate** | 98.56% | 97.33% | 96.58% | 98.81% | 98.50% | 97.46% | 97.51% | 97.27% |

| **Group** | L-32 | L-33 | L-34 | L-35 | L-36 | L-37 | L-38 | L-39 |
|---|---|---|---|---|---|---|---|---|
| **Cell Survival Rate** | 97.52% | 99.44% | 96.57% | 97.53% | 99.19% | 98.58% | 97.82% | 98.61% |

| **Group** | L-40 | L-41 | L-42 | L-43 | L-44 | L-45 | L-46 | L-47 |
|---|---|---|---|---|---|---|---|---|
| **Cell Survival Rate** | 97.63% | 98.86% | 97.82% | 96.69% | 97.58% | 98.82% | 98.84% | 97.97% |

### (2) Serum stability evaluation

LNP-mRNA drug compositions were added to the medium containing 10% fetal bovine serum (FBS) with agitation at 37 °C. Samples were taken at regular intervals to determine the particle size change of LNP-mRNA, and the serum stability of the formulation of LNP-mRNA drug composition was analyzed by testing its particle size change. The experimental results showed that, within 7 days, the particle size changes of the control group and the experimental groups were all less than 10%, and especially, the particle size changes of experimental groups L-1, L-2, L-10, L-14, L-19, L-24, L-28, and L-33 were less than 5%, indicating that the formulation of LNP-mRNA drug composition prepared by cationic lipids of the present application had good serum stability.

### (3) Study on the mRNA transfection efficiency in cells

To investigate the mRNA transfection efficiency at the cellular level of each group of LNP-mRNA drug compositions prepared in Example 47 of the present application, luciferase bioluminescence was used for testing. The LNP-mRNA drug composition was dissolved in medium to prepare the required dose. 293T cells were used as the cell model, and cell suspensions of 100 µL/well were seeded in black-edged, clear-bottomed 96-well plates at a density of 6000 cells/well. After inoculation, the cells were cultured in a cell culture incubator for 24 h. The cells were then dosed with 0.2 ug mRNA per well, and the blank control group was added with the corresponding dose of free Fluc-mRNA. Each concentration of each group corresponded to 6 duplicate wells. After 24 h of transfection, the old culture medium was replaced with a fresh one containing the D-fluorescein sodium substrate (1.5 mg/mL). After 5 min of incubation, bioluminescence was detected using a microplate reader. The stronger the fluorescence, the more Fluc-mRNA was transported into the cytoplasm and translated into the corresponding fluorescent protein, as shown in Table 3. Wherein, the relative value of fluorescence intensity was the ratio of the fluorescence intensity value of each group to the fluorescence intensity of the blank control group. The results showed that the LNP-mRNA drug compositions prepared by the present application had excellent in vitro transfection effects, that was, the LNPs in the experimental groups were effective nucleic acid delivery carriers, most of which were superior to the L-0 group prepared with a cationic lipid in the prior art (except L-5, L-13, L-23 and L-32 groups). Wherein, the relative fluorescence values of experimental groups L-1, L-2, L-10, L-14, L-16, L-17, L-19, and L-26 were higher, which might be due to the presence of two ionizable tertiary amine structures and/or multiple degradable ester bonds and/or unsaturated bonds. The ionization of the ionizable tertiary amine could generate a partial positive charge to be combined with negatively charged nucleic acids. The degradable ester bonds could promote the endosomal escape of LNP-mRNA, and therefore promote the release of mRNA into the cytoplasm to exert the pharmaceutical effects. The unsaturated hydrocarbon tail chains were more loosely arranged, which can enhance the membrane fusion ability while maintaining the stability and fluidity of the membrane, therefore increasing the cellular uptake of the LNP-mRNA drug composition. The relative fluorescence values of experimental groups L-4, L-13, L-22 and L-40 were relatively low; the cationic lipids used in these groups had more hydrophobic tail chains and contained ether bonds; the encapsulation efficiency of these groups was not very low, but the cell transfection activity was poorer, probably due to the lower cellular uptake of LNP-mRNA or hindered endosomal escape.

**Table 3: Results of Cell Transfection Assays**

| **No.** | **Fluorescence relative value** | **No.** | **Fluorescence relative value** | **No.** | **Fluorescence relative value** |
|---|---|---|---|---|---|
| **Control** | 1 | **L-16** | 11.33 | **L-33** | 15.16 |
| **L-0** | 10.08 | **L-17** | 12.19 | **L-34** | 14.71 |
| **L-1** | 14.45 | **L-18** | 10.72 | **L-35** | 11.40 |
| **L-2** | 13.93 | **L-19** | 14.94 | **L-36** | 12.23 |
| **L-3** | 12.63 | **L-20** | 12.45 | **L-37** | 13.54 |
| **L-4** | 13.62 | **L-21** | 10.92 | **L-38** | 12.52 |
| **L-5** | 13.91 | **L-22** | 13.20 | **L-39** | 14.88 |
| **L-6** | 12.38 | **L-23** | 9.33 | **L-40** | 9.55 |
| **L-7** | 9.64 | **L-24** | 10.28 | **L-41** | 13.22 |
| **L-8** | 13.90 | **L-25** | 12.63 | **L-42** | 10.59 |
| **L-9** | 10.78 | **L-26** | 11.37 | **L-43** | 12.92 |
| **L-10** | 15.03 | **L-27** | 8.99 | **L-44** | 9.63 |
| **L-11** | 14.52 | **L-28** | 8.73 | **L-45** | 13.26 |
| **L-12** | 14.68 | **L-29** | 13.25 | **L-46** | 13.91 |
| **L-13** | 12.88 | **L-30** | 7.89 | **L-47** | 14.51 |
| **L-14** | 15.21 | **L-31** | 12.43 | | |
| **L-15** | 9.80 | **L-32** | 13.72 | | |

### (4) Study on the mRNA transfection efficiency in animals

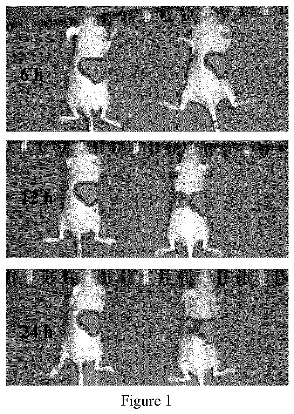

The LNP-mRNA drug compositions (L-1, L-10, L-19) were administered to 6-8-week-old female Babl/c mice through tail vein injection at a dose of 10 µg mRNA/mouse, and in vivo fluorescence imaging of small animals was performed 6 h, 12 h and 24 h after administration. After imaging at the last time point, mice were euthanized and their major organs (heart, liver, spleen, lungs, kidneys) and muscles at the injection site were imaged. Mice were intraperitoneally injected with 0.2 mL of D-luciferin sodium (15 mg/mL) 10-15 min before imaging. The results showed that the LNP-mRNA drug composition prepared from the cationic lipid of the present application also had excellent transfection efficiency in vivo. The transfection activity of group L-10 in animals is shown in Figure 1.

Those described above are only embodiments of the present application and are not for limitation. Any modification of equivalent structures or equivalent routes according to the present application, which may be applied in other related art directly or indirectly, should be included in the scope of the present application.

For those skilled in the art, without departing from the spirit and scope of the present application, and without unnecessary experimentation, the present application can be implemented in a wider range under equivalent parameters, concentrations, and conditions. While the present application has given particular examples, it should be understood that the present application can be further modified. In conclusion, under the principles of the present application, the present application is intended to cover any alterations, uses, or improvements of the present application, including changes departing from the scope disclosed in this application but made using conventional techniques known in the art.

## Claims

1. A cationic lipid, wherein the structure is represented by the general formula (1): 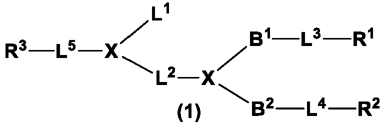
wherein, X is independently N or CR^{a} at each occurrence, and the R^{a} is H or a C₁₋₁₂ alkyl group;
L¹ is -L^{c}R, and L^{c} is a linking bond or a divalent linking group, and R is a C₁₋₃₀ aliphatic hydrocarbon group; wherein, the aliphatic hydrocarbon group is an alkyl group, an alkenyl group or an alkynyl group;
L² is a linking bond or a divalent linking group;
L⁵ is a linking bond or a divalent linking group;
L³ and L⁴ are each independently a linking bond or a divalent linking group;
B¹ and B² are each independently a linking bond or a C₁₋₃₀ alkylene group;
R¹ and R² are each independently a C₁₋₃₀ aliphatic hydrocarbon group or 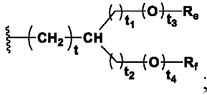 wherein, t is an integer from 0 to 12; t₁ and t₂ are each independently an integer from 0 to 5; t₃ and t₄ are each independently 0 or 1; t₁, t₂, t₃ and t₄ are not 0 simultaneously; Rₑ and R_{f} are each independently selected from the group consisting of a C₁₋₁₅ alkyl group, a C₂₋₁₅ alkenyl group, and a C₂₋₁₅ alkynyl group;
R³ is, at each occurrence, independently a hydrogen atom, -R^{d}, -OR^{d}, a C₃₋₆ carbocyclic group, a nitrogen-containing heterocyclyic group, -NR^{d}R^{d}, -SR^{d}, -C(=O)R^{d}, -C(=O)OR^{d}, - OC(=O)R^{d}, -OC(=O)OR^{d} or a functional group R⁰¹ that can interact with bio-related substances; wherein, R^{d} is a C₁₋₁₂ alkyl group;
or a salt, tautomer, stereoisomer, or solvate thereof.

2. The cationic lipid according to claim 1, wherein the R is selected from the group consisting of a linear alkyl group, a branched alkyl group, a linear alkenyl group, a branched alkenyl group, a linear alkynyl group, and a branched alkynyl group; preferably, R is a linear alkyl group or a linear alkenyl group; more preferably, R is a C₁₋₂₅ linear alkyl group; more preferably, R is a C₁₋₁₇ linear alkyl group, and specifically selected from the group consisting of a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group and a heptadecyl group.

3. The cationic lipid according to claim 1, wherein L^{c} is selected from the group consisting of a linking bond, -(CH₂)tₘ-, -(CH₂)tₘZ-, -Z(CH₂)ₜₘ-, -(CH₂)ₜₘZ(CH₂)ₜₘ- and - (CH₂)ₜₘZ(CH₂)ₜₘZ-; wherein, tm is independently an integer from 1 to 12 at each occurrence; the Z is independently selected from the group consisting of -C(=O)-, -OC(=O)-, -C(=O)O-, - OC(=O)O-, -O-, -S-, -C(=O)S-, -SC(=O)-, -NR^{c}C(=O)-, -C(=O)NR^{c}-, -NR^{c}C(=O)NR^{c-}, - OC(=O)NR^{c}-, -NR^{c}C(=O)O-, -SC(=O)NR^{c}- and -NR^{c}C(=O)S- at each occurrence; wherein, R^{c} is independently H or a C₁₋₁₂ alkyl group at each occurrence; the L^{c} is preferably selected from the group consisting of a linking bond, -(CH₂)ₜₘ-, -(CH₂)ₜₘO-, -(CH₂)ₜₘC(=O)-, - (CH₂)ₜₘC(=O)O-, -(CH₂)ₜₘOC(=O)-, -(CH₂)ₜₘC(=O)NH-, -(CH₂)ₜₘNHC(=O)-, - (CH₂)ₜₘOC(=O)O-, -(CH₂)ₜₘNHC(=O)O-, -(CH₂)ₜₘOC(=O)NH-, -(CH₂)ₜₘNHC(=O)NH-, - (CH₂)ₜₘO(CH₂)ₜₘ-, -(CH₂)ₜₘC(=O)(CH₂)ₜₘ-, -(CH₂)ₜₘC(=O)O(CH₂)ₜₘ-, - (CH₂)ₜₘOC(=O)(CH₂)ₜₘ-, -(CH₂)ₜₘC(=O)NH(CH₂)ₜₘ-, -(CH₂)ₜₘNHC(=O)(CH₂)ₜₘ-, - (CH₂)ₜₘOC(=O)O(CH₂)ₜₘ-, -(CH₂)ₜₘNHC(=O)O(CH₂)ₜₘ-, -(CH₂)ₜₘOC(=O)NH(CH₂)ₜₘ- and - (CH₂)ₜₘNHC(=O)NH(CH₂)ₜₘ-; more preferably, L^{c} is selected from the group consisting of a linking bond, -(CH₂)ₜₘO(CH₂)ₜₘ-, -(CH₂)ₜₘC(=O)O(CH₂)ₜₘ-, and -(CH₂)ₜₘOC(=O)(CH₂)ₜₘ-.

4. The cationic lipid according to claim 1, wherein B¹ and B² are each independently a linking bond or a C₁₋₂₀ alkylene group; specifically, B¹ and B² are selected from any of the following cases:
Case (1): B¹ and B² are each independently a C₁₋₂₀ alkylene group, specifically, B¹ and B² are each independently selected from the group consisting of a methylene group, an ethylene group, a propylene group, a butylene group, a pentylene group, a hexylene group, a heptylene group, an octylene group, a nonylene group, a decylene group, a undecylene group, a dodecylene group, a tridecylene group, a tetradecylene group, a pentadecylene group, a hexadecylene group, a heptadecylene group, an octadecylene group, a nonadecylene group, and an eicosylene group; more preferably, B¹ and B² are each independently a C₂₋₁₀ alkylene group;
Case (2): one of B¹ and B² is a linking bond, and the other is a C₁₋₂₀ alkylene group;
Case (3): both B¹ and B² are linking bonds.

5. The cationic lipid according to claim 1, wherein L³ and L⁴ are each independently selected from the group consisting of a linking bond, -OC(=O)-, -C(=O)O-, -OC(=O)O- - C(=O)-, -O-, -NH-, -O(CR^{c}R^{c})ₛO-, -S-, -C(=O)S-, -SC(=O)-, -NR^{c}C(=O)-, -C(=O)NR^{c}-, - NR^{c}C(=O)NR^{c}-, -OC(=O)NR^{c}-, -NR^{c}C(=O)O-, -SC(=O)NR^{c}-, and -NR^{c}C(=O)S-; wherein, R^{c} is independently a hydrogen atom or a C₁₋₁₂ alkyl group at each occurrence, and s is 2, 3, or 4; L³ and L⁴ are more preferably selected from any one of the following cases:
Case (1): L³ and L⁴ are each independently selected from the group consisting of - OC(=O)-, -C(=O)O-, -OC(=O)O-, -C(=O)-, -O-, -O(CH₂)ₛO-, -S-, -C(=O)S-, -SC(=O)-, - NHC(=O)-, -C(=O)NH-, -NHC(=O)NH-, -OC(=O)NH-, -NHC(=O)O-, -SC(=O)NH-, and - NHC(=O)S-;
Case (2): one of L³ and L⁴ is a linking bond, and the other is selected from the group consisting of -OC(=O)-, -C(=O)O-, -OC(=O)O-, -C(=O)-, -O-, -O(CR^{c}R^{c})ₛO-, -S-, -C(=O)S-, -SC(=O)-, -NR^{c}C(=O)-, -C(=O)NR^{c}-, -NR^{c}C(=O)NR^{c}-, -OC(=O)NR^{c}-, -NR^{c}C(=O)O-, - SC(=O)NR^{c}-, and -NR°C(=O)S-;
Case (3): both L³ and L⁴ are linking bonds;
more preferably, L³ and L⁴ are each independently selected from the group consisting of -OC(=O)-, -C(=O)O-, and -OC(=O)O-; more preferably, both L³ and L⁴ are -OC(=O)-, - C(=O)O- or -OC(=O)O-, simultaneously.

6. The cationic lipid according to claim **1,** wherein the L² is selected from the group consisting of a linking bond, -(CH₂)ₘ-, -(CH₂)ₘC(=O)-, -C(=O)(CH₂)ₘ-, and - C(=O)(CH₂)ₘC(=O)-; wherein, m is an integer from 1 to 4.

7. The cationic lipid according to claim **1,** wherein R¹ and R² are each independently a C₁₋₃₀ linear aliphatic hydrocarbon group, a C₁₋₃₀ branched aliphatic hydrocarbon group or 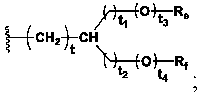
the linear aliphatic hydrocarbon group is a linear alkyl group, a linear alkenyl group or a linear alkynyl group; the linear aliphatic hydrocarbon group is preferably a C₁₋₂₅ linear aliphatic hydrocarbon group, and more preferably a C₅₋₁₇ linear aliphatic hydrocarbon group;
the branched aliphatic hydrocarbon group is a branched alkyl group, a branched alkenyl group, or a branched alkynyl group, and the branched aliphatic hydrocarbon group is independently represented by 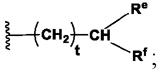 wherein, t is an integer from 0 to 12, t₁ and t₂ are each independently an integer from 0 to 5, t₃ and t₄ are each independently 0 or 1, and t₁, t₂, t₃, and t₄ are not 0 simultaneously; R^{e} and R^{f} are each independently selected from the group consisting of a C₁₋₁₅ alkyl group, a C₂₋₁₅ alkenyl group, and a C₂₋₁₅ alkynyl group; more preferably, R^{e} and R^{f} are each independently selected from the group consisting of a methyl group, an ethyl group, a propyl group, a butyl group, a pentyl group, a hexyl group, a heptyl group, an octyl group, a nonyl group, a decyl group, a undecyl group, a dodecyl group, a tridecyl group, a tetradecyl group, a vinyl group, a propenyl group, an allyl group, a butenyl group, an allyl carbinyl group, a pentenyl group, a neopentyl group, a hexenyl group, a neohexenyl group, a heptenyl group, a neoheptenyl group, an octenyl group, a neooctenyl group, a nonenyl group, a neononenyl group, a decenoyl group, a neodecenoyl group, an ethynyl group, a propynyl group, a propargyl group, a butynyl group, a butynediyl group, a pentynyl group, a neopentyl group, a hexynyl group, a neohexyl group, a heptynyl group, a neoheptyl group, an octynyl group, a neooctyl group, a nonynyl group, a neononyl group, a decynylgroup, and a neodecyl group; more preferably, R^{e} and R^{f} are each independently selected from the group consisting of a methyl group, an ethyl group, a propyl group, a butyl group, a pentyl group, a hexyl group, a heptyl group, an octyl group, a nonyl group, and a decyl group.

8. The cationic lipid according to claim **7,** wherein R¹ and R² are selected from any of the following cases:
Case (1): one of R¹ and R² is a C₁₋₃₀ linear aliphatic hydrocarbon group, and the other is a C₁₋₃₀ branched aliphatic hydrocarbon group;
Case (2): R¹ and R² are each independently a C₁₋₃₀ branched aliphatic hydrocarbon group represented by 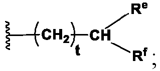
Case (3): R¹ and R² are each independently represented by 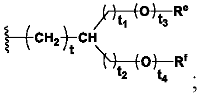
Case (4): one of R¹ and R² is a C₁₋₃₀ linear aliphatic hydrocarbon group or a C₁₋₃₀ branched aliphatic hydrocarbon group represented by 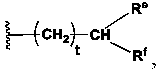
and the other is represented by 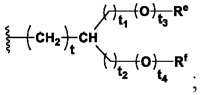 further preferably, the aforementioned R¹ and R² are each independently selected from the group consisting of the following structures:

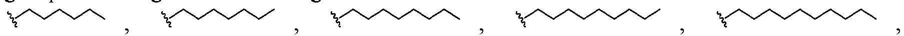

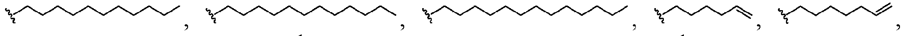

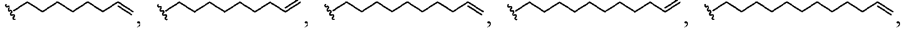

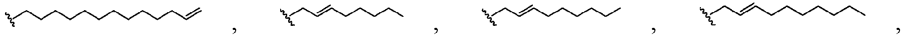

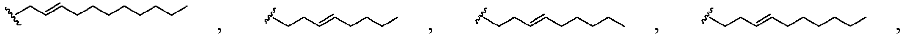

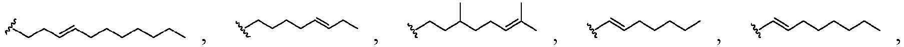

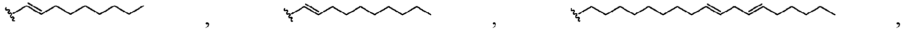

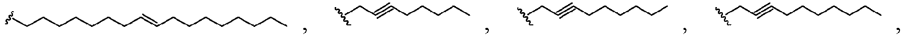

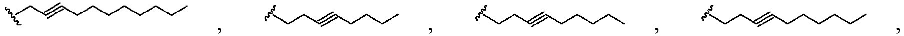

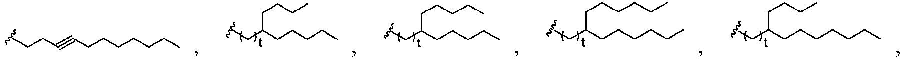

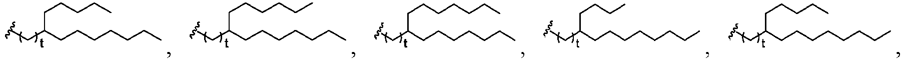

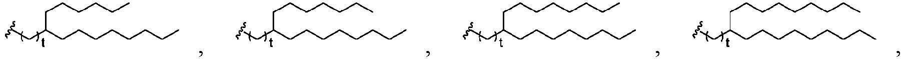

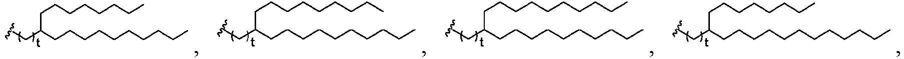

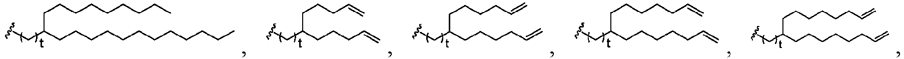

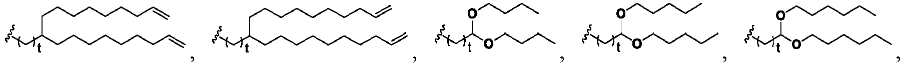

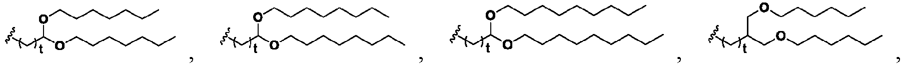

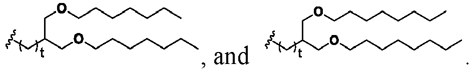

9. The cationic lipid according to claim 1, wherein the L⁵ is a divalent linking group selected from the group consisting of L⁶, L⁷, Z, and combinations of any two or more thereof; more preferably, L⁵ is selected from the group consisting of -L⁶-, -L⁶-Z-, -Z-L⁶- -Z-L⁶-Z-, -L⁶-Z-L⁷-, -Z-L⁶-Z-L⁷- , -L⁶-Z-L⁷-Z-, -Z-L⁶-Z-L⁷-Z-, and -L⁶-Z-L⁷-Z-L⁶-Z-; wherein L⁶ and L⁷ are carbon-chain linking groups, and L⁶ and L⁷ are each independently represented by -(CR^{a}R^{b})ₜ-(CR^{a}R_{b})ₒ-(CR^{a}R^{b})ₚ-; wherein, t, o, p are each independently an integer from 0 to 12, and t, o, p are not 0 simultaneously; R^{a} and R^{b} are each independently a hydrogen atom or a C₁₋₁₂ alkyl group at each occurrence; the Z is independently selected from the group consisting of -C(=O)-, -OC(=O)-, -C(=O)O-, -OC(=O)O-, -O-, -S-, -C(=O)S-, -SC(=O)-, -NR^{c}C(=O)-, -C(=O)NR^{c}-, - NR^{c}C(=O)NR^{c}-, -OC(=O)NR^{c}-, -NR^{c}C(=O)O-, -SC(=O)NR^{c}- and -NR^{c}C(=O)S-; wherein, R^{c} is independently H or a C₁₋₁₂ alkyl group at each occurrence.

10. The cationic lipid according to claim **9,** wherein the L⁵ is selected from the group consisting of a linking bond, -(CH₂)ₜₙZ-, -Z(CH₂)ₜₙ-, -Z(CH₂)ₜₙZ-, -(CH₂)ₜₙZ(CH₂)ₜₙZ- and - Z(CH₂)ₜₙZ(CH₂)ₜₙZ-; wherein, tn is independently an integer from 1 to 12 at each occurrence; L⁵ is preferably selected from the group consisting of a linking bond, -(CH₂)ₜₙO-, - (CH₂)ₜₙC(=O)O-, -(CH₂)ₜₙOC(=O)-, -(CH₂)ₜₙNHC(=O)-, -(CH₂)ₜₙOC(=O)O-, - (CH₂)ₜₙNHC(=O)O-, -O(CH₂)ₜₙO-, -C(=O)O(CH₂)ₜₙC(=O)O-, -OC(=O)(CH₂)ₜₙOC(=O)-, - C(=O)O(CH₂)ₜₙOC(=O)-, -OC(=O)(CH₂)ₜₙC(=O)O-, -OC(=O)O(CH₂)ₜₙOC(=O)O-, - NHC(=O)O(CH₂)ₜₙNHC(=O)O-, -OC(=O)NH(CH₂)ₜₙNHC(=O)O-, -C(=O)(CH₂)ₜₙO-, - C(=O)(CH₂)ₜₙC(=O)O-, -C(=O)(CH₂)ₜₙOC(=O)-, -C(=O)(CH₂)ₜₙOC(=O)O-, - C(=O)(CH₂)ₜₙNHC(=O)O- and -C(=O)(CH₂)ₜₙC(=O)(CH₂)ₜₙNHC(=O)O-; more preferably, L⁵ is selected from the group consisting of a linking bond, -C(=O)O-, -(CH₂)ₜₙO-, - (CH₂)ₜₙC(=O)O-, -(CH₂)ₜₙO-, -C(=O)(CH₂)ₜₙO-, -C(=O)(CH₂)ₜₙC(=O)O-, - C(=O)(CH₂)ₜₙNHC(=O)O-, and -C(=O)(CH₂)ₜₙC(=O)(CH₂)ₜₙNHC(=O)O-.

11. The cationic lipid according to claim **1,** wherein the R³ is independently selected from the group consisting of a hydrogen atom, -R^{d}, -OR^{d}, -NR^{d}R^{d}, -C(=O)R^{d}, -C(=O)OR^{d}, -OC(=O)R^{d}, -OC(=O)OR^{d} and R⁰¹; more preferably, the R³ is independently selected from the group consisting of a hydrogen atom, an alkyl group, an alkoxy group, -C(=O)OR^{d}, -OC(=O)R^{d}, - OC(=O)OR^{d}, an epoxy group, an alcoholic hydroxyl group, a protected alcoholic hydroxyl group, a sulfhydryl group, a protected sulfhydryl group, a carboxyl group, a protected carboxyl group, an amino group, a protected amino group, an aldehyde group, a protected aldehyde group, an active ester group, a carbonate group, a urethane group, an isocyanate group, an isothiocyanate group, a succinimidyl group, a maleimide group, a protected maleimide group, a dimethylamino group, an alkenyl group, an enoate group, an azido group, a cyano group, a dithiopyridinyl group, an α-haloacetyl alkynyl group, an alkynyl group, a folate group, a rhodamine group, a biotin group, a monosaccharide group, a polysaccharide group,

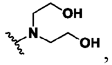

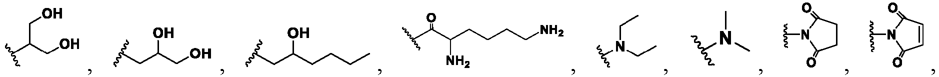

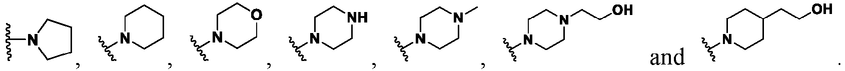

12. The cationic lipid according to claim **11,** wherein the -L⁵-R³ is independently selected from the group consisting of the following structures:

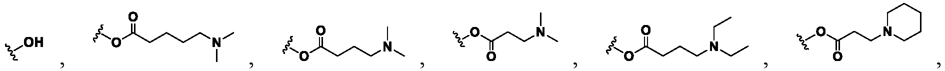

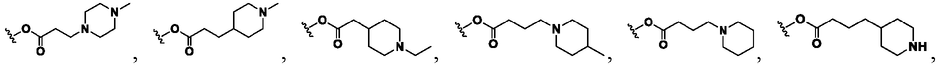

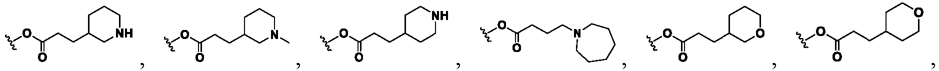

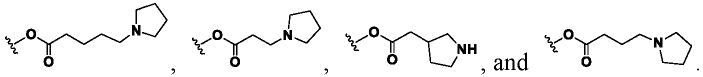

13. The cationic lipid according to claim **1,** wherein the R⁰¹ is a functional group with therapeutic targeting property; preferably, R⁰¹ is a residue of folic acid, N-acetylgalactosamine or any functional derivative thereof; further preferably, R⁰¹ is selected from the group consisting of the following structures:

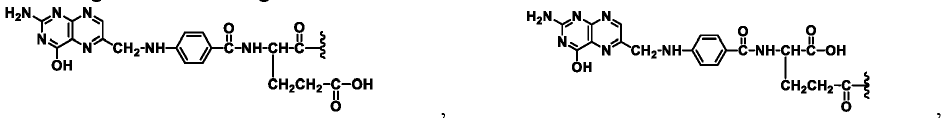

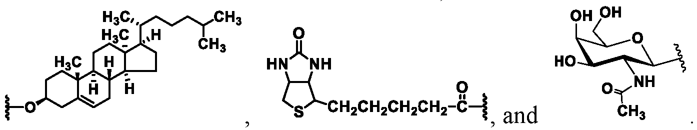

14. The cationic lipid according to claim **1,** wherein the structure of the cationic lipid is represented by the following general formula (2): 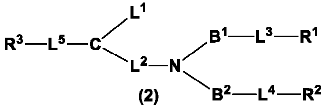 more preferably, the structure of the cationic lipid is selected from the group consisting of the following general formulas: 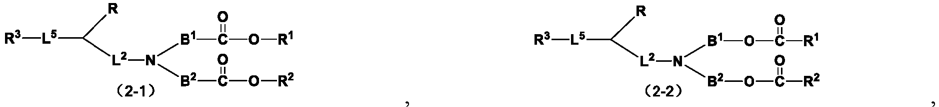 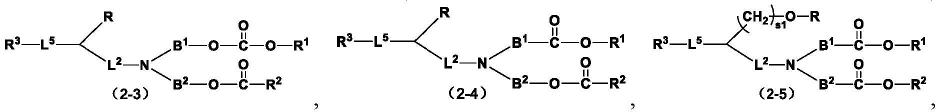 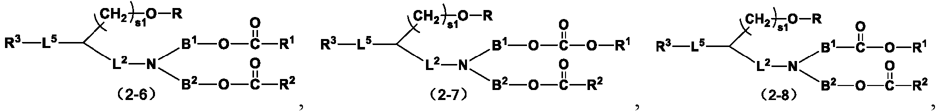 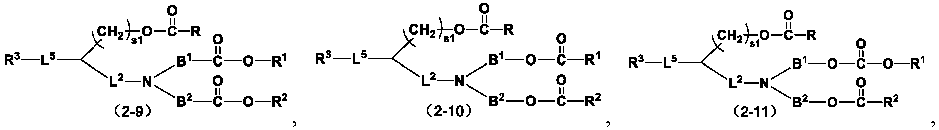 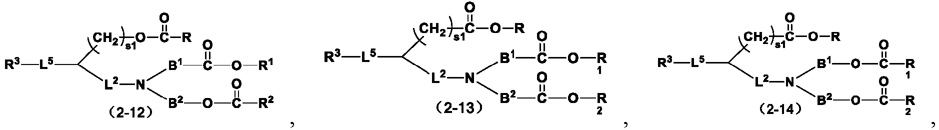 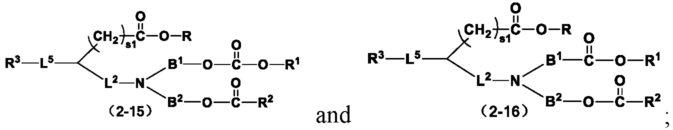 wherein, s1 is 0, 1, 2, 3, or 4.

15. The cationic lipid according to claim **1,** wherein the structure is selected from the group consisting of the following structures: 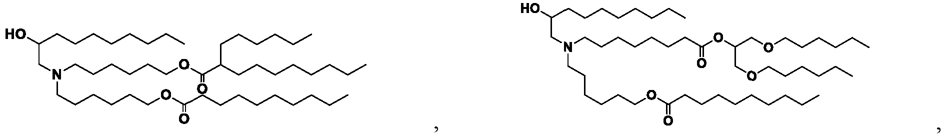 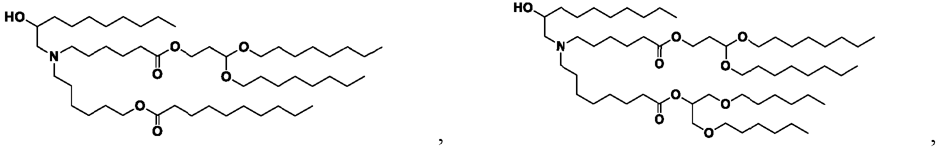 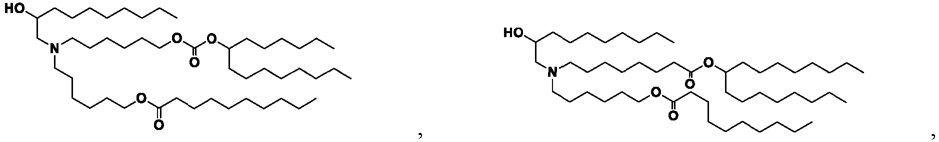 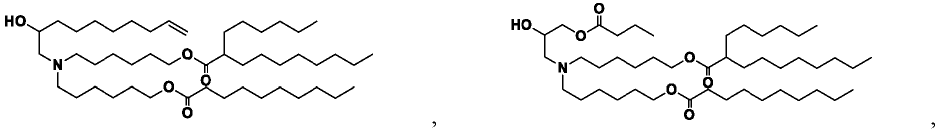 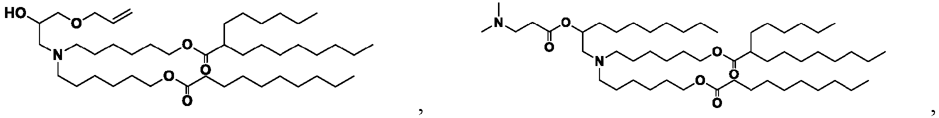 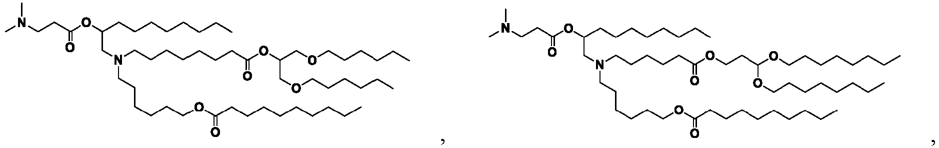 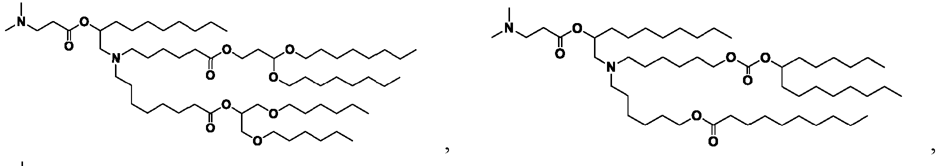 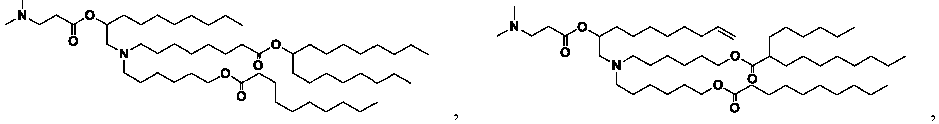 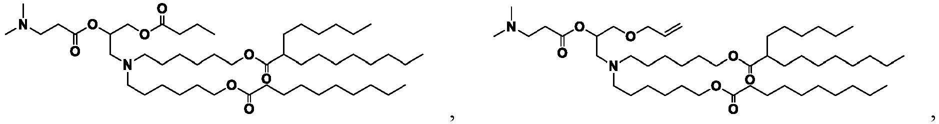 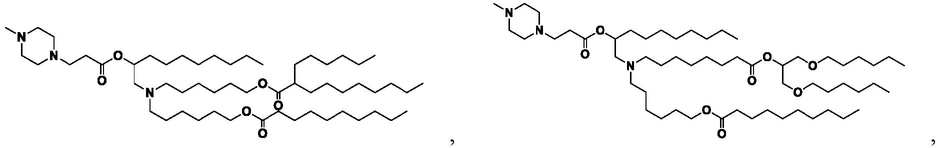 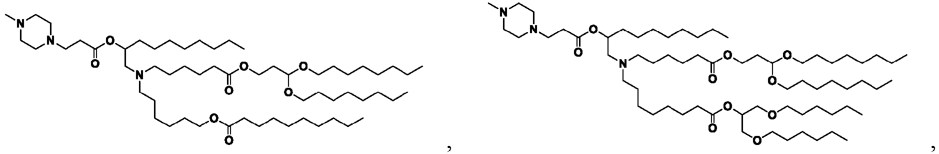 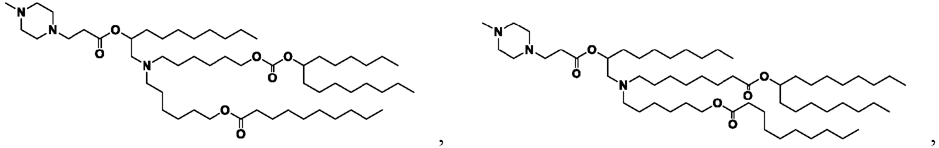 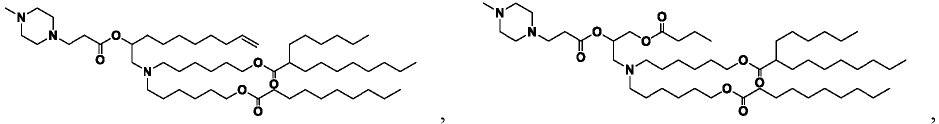 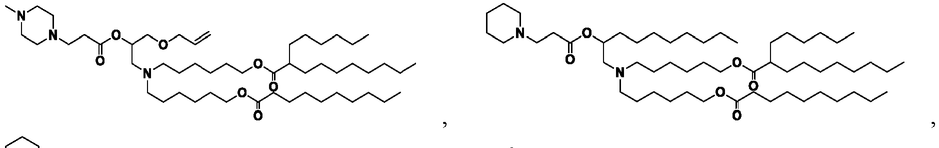 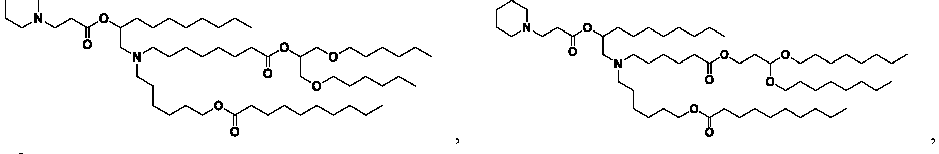 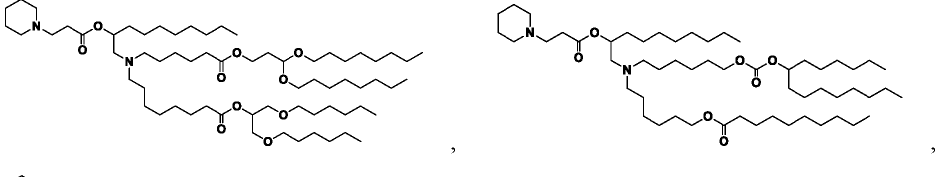 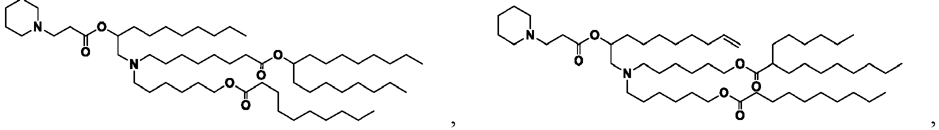 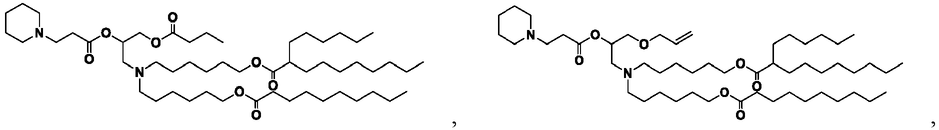 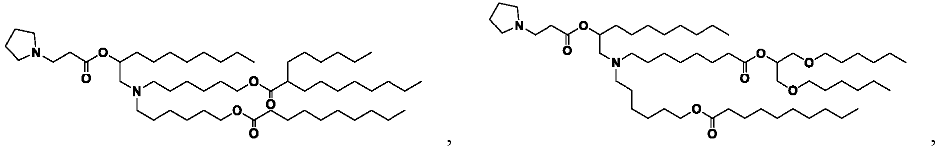 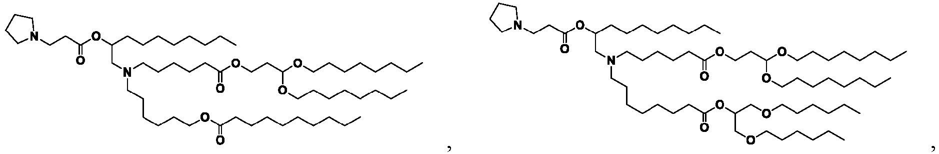 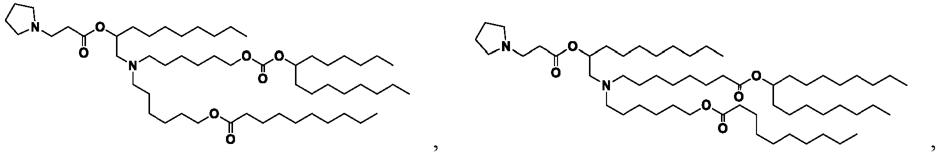 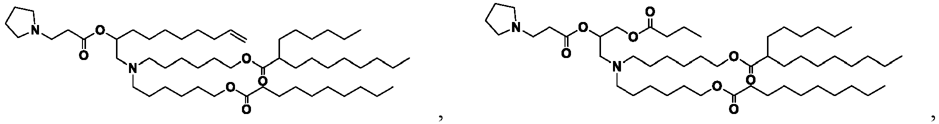 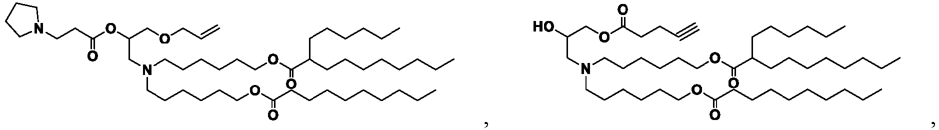 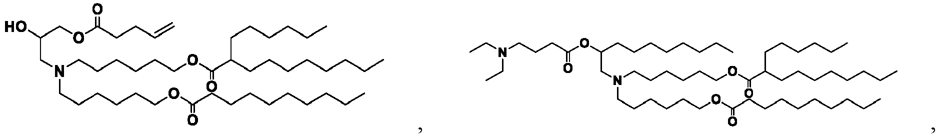 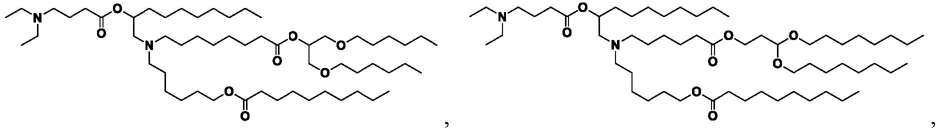 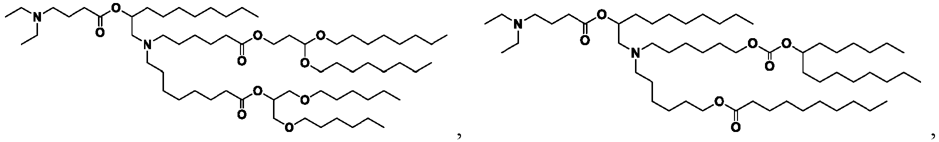 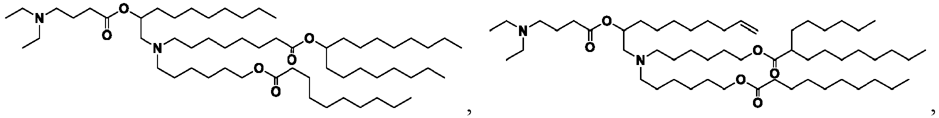 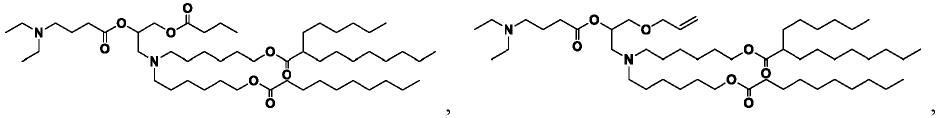 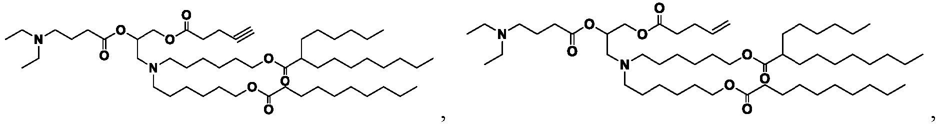 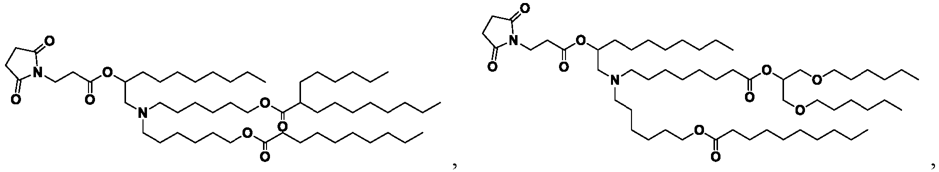 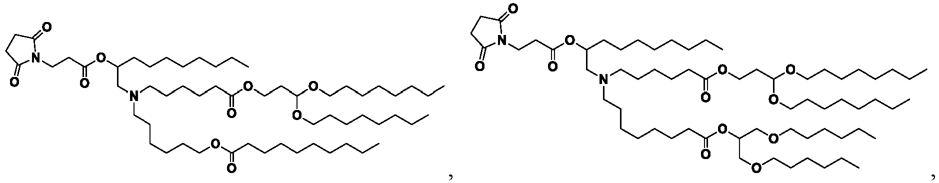 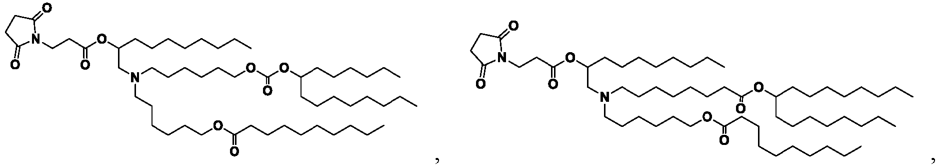 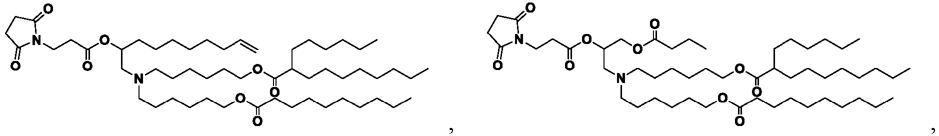 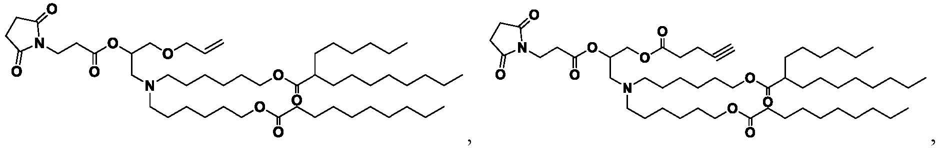 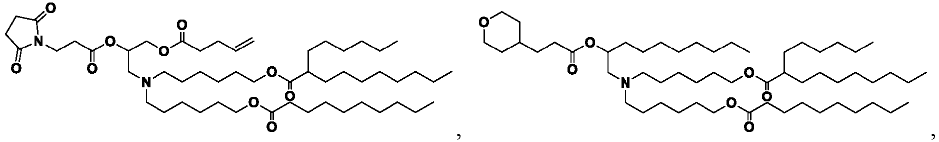 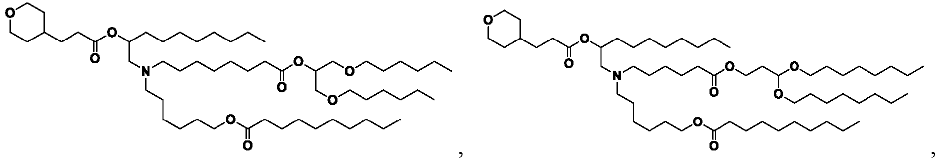 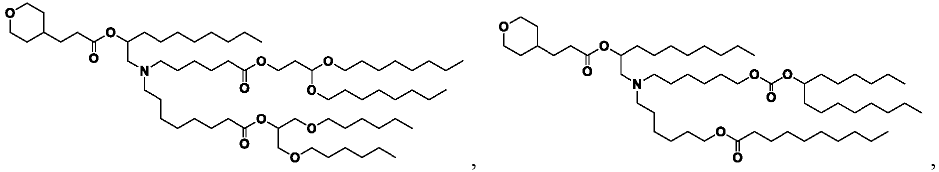 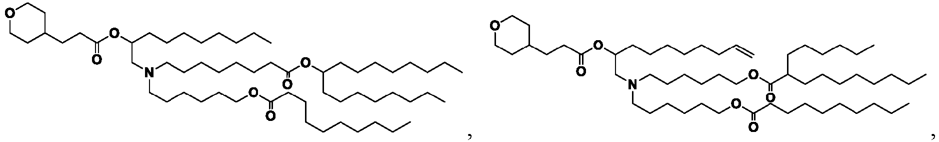 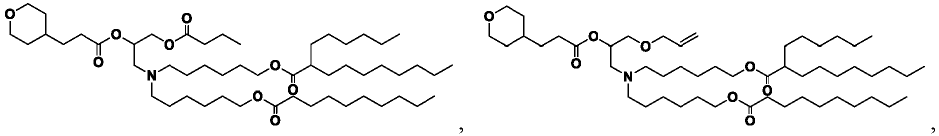 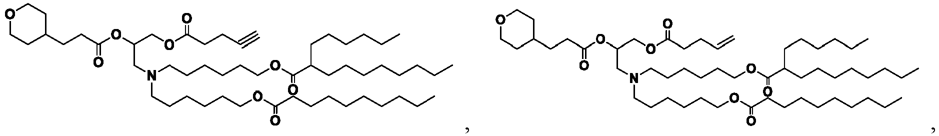 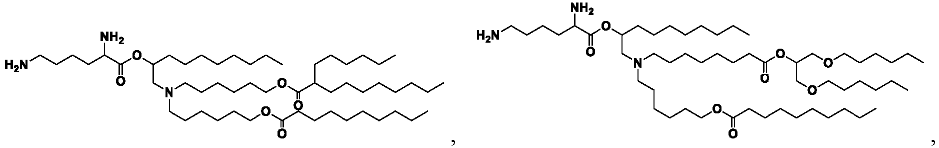 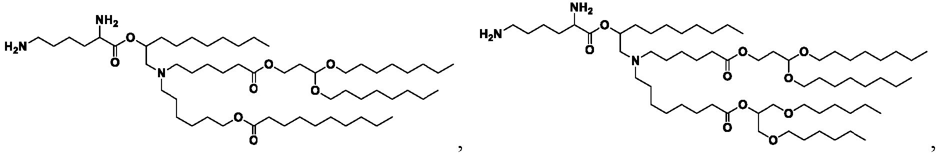 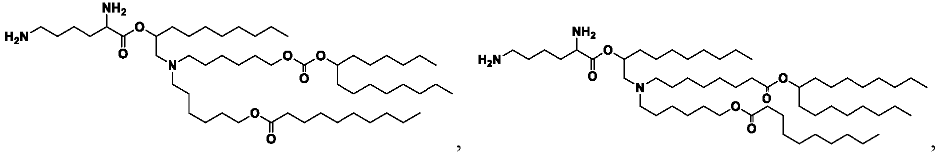 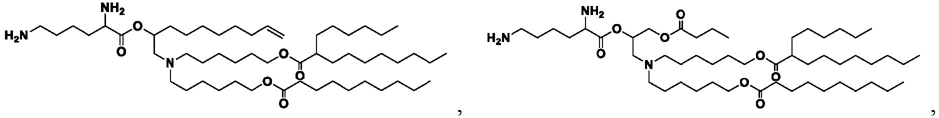 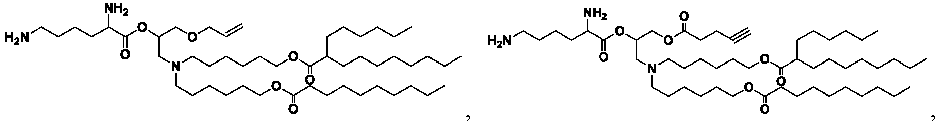 and

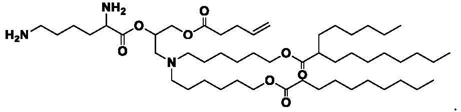

16. A lipid composition containing a cationic lipid of any one of claims **1** to **15.**

17. The lipid composition according to claim **16,** wherein the lipid composition contains one or more types of lipids selected from the group consisting of phospholipid, steroid lipid, and PEGylated lipid; the lipid composition is selected from any of the following cases:
Case (1): also contains a phospholipid;
Case (2): also contains a steroid lipid;
Case (3): also contains a PEGylated lipid;
Case (4): also contains a phospholipid and a steroid lipid;
Case (5): also contains a phospholipid and a PEGylated lipid;
Case (6): also contains a steroid lipid and a PEGylated lipid;
Case (7): also contains a phospholipid, a steroid lipid, and a PEGylated lipid;
more preferably, the lipid composition also contains a phospholipid, a steroid lipid, and a PEGylated lipid, simultaneously.

18. The lipid composition according to claim **17,** wherein the phospholipid is selected from the group consisting of 1,2-dilinoleoyl-sn-glycero-3-phosphocholine, 1,2-dimyristoleoyl-sn-glycero-3-phosphocholine, 1,2-dioleoyl-sn-glycero-3-phosphocholine, 1,2-dipalmitoyl-sn-glycero-3-phosphocholine, 1,2-distearoyl-sn-glycero-3-phosphatidylcholine, 1,2-diundecanoyl-sn-glycero-3-phosphatidylcholine, 1-plamitoyl-2-oleoyl-sn-glycero-3-phosphocholine, 1,2-di-O-octadecenyl-sn-glycero-3-phosphatidylcholine, 1-oleoyl-2-cholesterylhemisuccinyl-sn-glycero-3-phosphocholine, 1-O-hexadecyl-sn-glycero-3-phosphatidylcholine, 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholine, 1,2-dioleoyl-sn-glycero-3-phosphoethanolamine, 1,2-diphytanyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt, dioleoyl phosphatidylserine, dipalmitoyl phosphatidylglycerol, palmitoyloleoyl phosphatidylethanolamine, distearoyl phosphatidylethanolamine, dipalmitoyl phosphatidylethanolamine, dimyristoleoyl phosphoethanolamine, 1-stearoyl-2-oleoyl-stearoylethanolamine, 1-stearoyl-2-oleoyl-phosphatidylcholine, sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine, and combinations thereof;
the steroid lipid is selected from the group consisting of cholesterol, fecal sterol, sitosterol, ergosterol, campesterol, stigmasterol, rapeseed sterol, lycopene, ursolic acid, α-tocopherol, and combinations thereof;
the PEGylated lipid is selected from the group consisting of 1,2-dimyristoyl-sn-glycerol methoxypolyethylene glycol, 1,2-distearoyl-sn-glycero-3-phosphoethanolamine-N-[amino(polyethylene glycol)], PEG-cholesterol, PEG-diacylglycamide, PEG-dialkyloxypropyl, PEG500-dipalmitoylphosphatidylcholine, PEG2000-dipalmitoylphosphatidylcholine, PEG500-stearylphosphatidylethanolamine PEG2000-stearylphosphatidylethanolamine, PEG500-1,2-oleoylphosphatidylethanolamine, PEG2000-1,2-oleoylphosphatidylethanolamine, PEG2000-2,3-distearoylglycerol, and combinations thereof.

19. The lipid composition according to any one of claims **17** to **18,** wherein the lipid composition comprises 20-80% cationic lipid, 5-15% phospholipid, 25-55% steroid lipid, and 0.5-10% PEGylated lipid, and the percentage is the molar percentages of each lipid in the total lipids in a solution containing solvent.

20. The lipid composition according to claim **19,** wherein the molar percentage of cationic lipid in the total lipids in a solution containing a solvent is 30-65%, more preferably 35%, 40%, 45%, 46%, 47%, 48%, 49%, 50%, or 55%;
the molar percentage of phospholipid in the total lipids in a solution containing solvent is 7.5-13%, more preferably 8%, 9%, 10%, 11%, or 12%;
the molar percentage of steroid lipid in the total lipids in a solution containing solvent is 35-50%, more preferably 40%, 41%, 42%, 43%, 44%, 45%, 46%, 47%, 48%, 49%, or 50%;
the molar percentage of PEGylated lipid in the total lipid in a solution containing solvent is 0.5-5%, preferably 1-3%, more preferably 1.5%, 1.6%, 1.7%, 1.8%, or 1.9%.

21. A lipid pharmaceutical composition containing a lipid composition of any one of claims **16** to **20** and a drug, wherein the drug is selected from the group consisting of a nucleic acid drug, a gene vaccine, an antitumor drug, a small molecule drug, a peptide drug and a protein drug.

22. The lipid pharmaceutical composition according to claim **21,** wherein the nucleic acid drug is selected from the group consisting of RNA, DNA, antisense nucleic acid, plasmid, interfering nucleic acid, aptamer, antagomir and ribozyme, and the RNA is selected from the group consisting of mRNA, saRNA, circRNA, miRNA and siRNA; preferably, the nucleic acid drug is selected from the group consisting of DNA, mRNA, miRNA and siRNA.

23. The lipid pharmaceutical composition according to any one of claims **21** to **22,** the pharmaceutical composition is used as a drug, selected from the group consisting of the following drugs: an antineoplastic agent, an antiviral agent, an antifungal agent and a vaccine.

24. A formulation of lipid pharmaceutical composition containing any lipid pharmaceutical composition of claim **23** and a pharmaceutically acceptable diluent or excipient; the diluent or excipient is preferably selected from the group consisting of deionized water, ultrapure water, phosphate buffer, and physiological saline, more preferably phosphate buffer or physiological saline, most preferably physiological saline.

25. A liposome or lipid nanoparticle containing a lipid composition of any one of claims **16** to **20.**

26. The liposome or lipid nanoparticle according to claim **25,** wherein the lipid nanoparticle is an LNP pharmaceutical composition, an LPP pharmaceutical composition or a PNP pharmaceutical composition, preferably an LNP pharmaceutical composition, more preferably an LNP-nucleic acid pharmaceutical composition, more preferably an LNP-mRNA pharmaceutical composition.
